# EUROPEAN PATENT APPLICATION

(11) **EP 3 392 246 A1**
(43) Date of publication of application: **24.10.2018**
(21) Application number: 16873355.8
(22) Date of filing: 08.12.2016
(51) Int. Cl.: C07D 403/10, C07D 409/14, C07D 405/14, C07D 209/82, C07D 333/76, C07D 307/91, C09K 11/06, H01L 51/50, H01L 51/00

(54) **HETEROCYCLIC COMPOUND AND ORGANIC LIGHT EMITTING ELEMENT USING SAME**

(30) Priority: 08.12.2015 KR 20150174500
(71) Applicant: Heesung Material Ltd., Yongin-City, Gyeonggi-do 17118 (KR)
(72) Inventor: PARK, Geon-yu, Osan-si Gyeonggi-do 18120 (KR); OH, Han-Kook, Osan-si Gyeonggi-do 18134 (KR); LEE, Yun-Ji, Osan-si Gyeonggi-do 18138 (KR); KIM, Dong-Jun, Yongin-si Gyeonggi-do 17118 (KR); CHOI, Jin-Seok, Suwon-si Gyeonggi-do 16438 (KR); CHOI, Dae-Hyuk, Yongin-si Gyeonggi-do 16953 (KR); LEE, Joo-Dong, Seongnam-si Gyeonggi-do 13586 (KR)
(74) Representative: Goddar, Heinz J.
(86) International application number: PCT/KR2016/014367
(87) International publication number: WO 2017/099490

(57) **Abstract**

The present application provides a hetero-cyclic compound which may significantly improve the service life, efficiency, electrochemical stability, and thermal stability of an organic light emitting device, and an organic light emitting device in which the hetero-cyclic compound is contained in an organic compound layer.

## Description

### [Technical Field]

This application claims priority to and the benefit of Korean Patent Application No. 10-2015-0174500 filed in the Korean Intellectual Property Office on December 8, 2015, the entire contents of which are incorporated herein by reference.

The present application relates to a hetero-cyclic compound and an organic light emitting device using the same.

### [Background Art]

An electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

### [Disclosure]

### [Technical Problem]

It is necessary to perform studies on an organic light emitting device comprising a compound having a chemical structure, which may satisfy conditions required for a material which is available for the organic light emitting device, for example, appropriate energy levels, electrochemical stability, thermal stability, and the like, and may perform various functions required for the organic light emitting device according to the substituent.

### [Technical Solution]

An exemplary embodiment of the present application provides a hetero-cyclic compound represented by the following Chemical Formula 1:

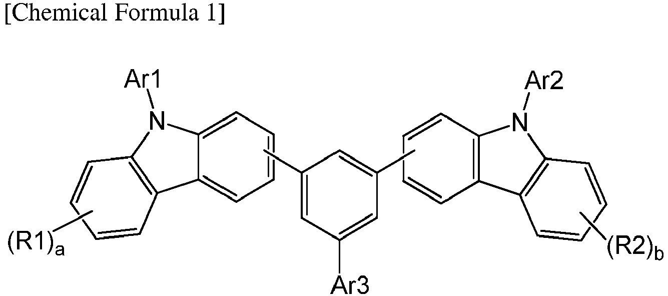

In Chemical Formula 1,
Ar1 to Ar3 are the same as or different from each other, and are each independently a substituted or unsubstituted C₆ to C₆₀ aryl group; or a substituted or unsubstituted C₂ to C₆₀ heteroaryl group,
R1 and R2 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group,
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
a and b are each independently an integer from 0 to 4.

Further, another exemplary embodiment of the present application provides an organic light emitting device comprising a positive electrode, a negative electrode, and an organic material layer having one or more layers disposed between the positive electrode and the negative electrode, in which one or more layers of the organic material layer comprise the hetero-cyclic compound represented by Chemical Formula 1.

In addition, still another exemplary embodiment of the present application provides an organic light emitting device in which the organic material layer comprising the hetero-cyclic compound additionally comprises a compound represented by the following Chemical Formula 2 or 3.

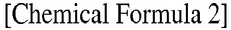

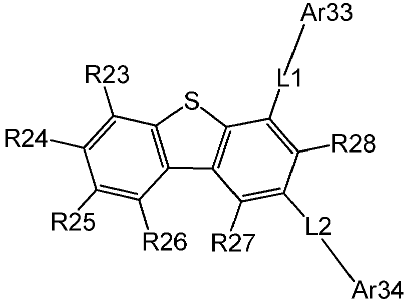

In Chemical Formula 4,
L1 and L2 are the same as or different from each other, and are each independently a direct bond or a substituted or unsubstituted C₆ to C₆₀ arylene group,
Ar33 is a substituted or unsubstituted C₂ to C₆₀ heteroaryl group comprising at least one N,
Ar34 is represented by the following Chemical Formula 4 or 5, 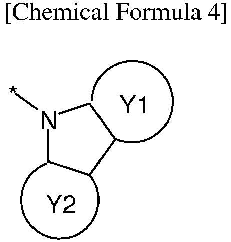 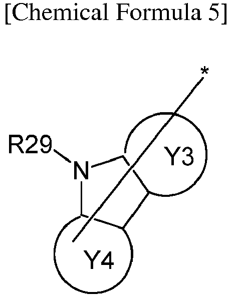 Y1 to Y4 are the same as or different from each other, and are each independently a substituted or unsubstituted C₆ to C₆₀ aromatic hydrocarbon ring; or a substituted or unsubstituted C₂ to C₆₀ aromatic hetero ring,
R23 to R29 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and

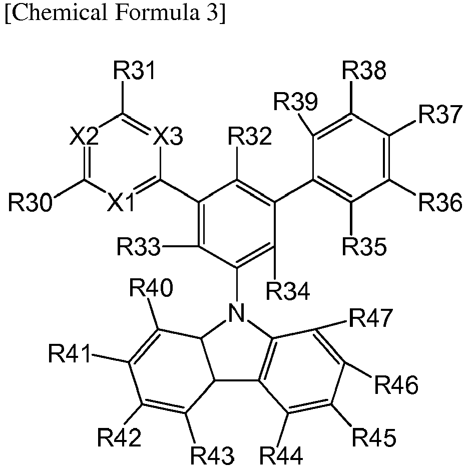

In Chemical Formula 3,
at least one of X1 to X3 is N, and the others are each independently N or CR48,
R31, R32, and R48 are the same as or different from each other, and are each independently selected from the group consisting of hydrogen; deuterium; a halogen group; - CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R";-P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,
R32 to R34 and R40 to R43 are the same as or different from each other, and are each independently selected from the group consisting of hydrogen; deuterium; a halogen group; - CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; - P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group,
R44 to R47 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted hydrocarbon ring or hetero ring,
at least one of R35 to R39 is -CN, and the others are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring, and
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group.

### [Advantageous Effects]

A hetero-cyclic compound according to an exemplary embodiment of the present application may be used as a material for an organic material layer of an organic light emitting device. The hetero-cyclic compound may be used as a material for a hole injection layer, a hole transporting layer, a light emitting layer, an electron transporting layer, an electron injection layer, and the like in an organic light emitting device. In particular, the hetero-cyclic compound represented by Chemical Formula 1 may be used as a material for an electron transporting layer, a hole transporting layer, or a light emitting layer of an organic light emitting device. In addition, when the hetero-cyclic compound represented by Chemical Formula 1 is used for an organic light emitting device, the driving voltage of the device may be lowered, the light efficiency of the device may be improved, and the service life characteristics of the device may be improved due to the thermal stability of the compound.

In particular, the organic light emitting device according to an exemplary embodiment of the present application comprises as a host material for a light emitting layer: both the hetero-cyclic compound represented by Chemical Formula 1; and the compound represented by Chemical Formula 2 or 3, and thus may exhibit significantly improved characteristics in terms of all of the driving, efficiency, and service life as compared to an organic light emitting device to which a single compound is applied as a host material.

### [Description of Drawings]

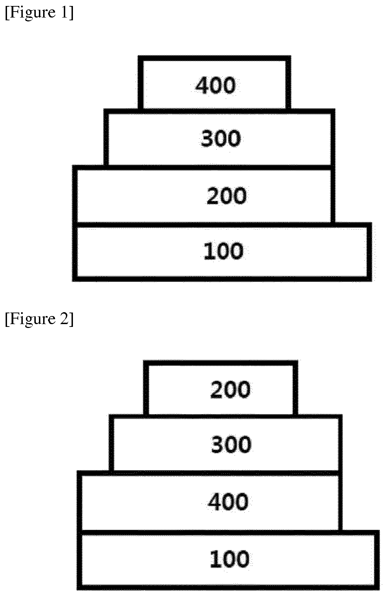

FIGS. 1 to 3 each are a view schematically illustrating a stacking structure of an organic light emitting device according to an exemplary embodiment of the present application.

### <Explanation of Reference Numerals and Symbols>

100: Substrate
200: Positive electrode
300: Organic material layer
301: Hole injection layer
302: Hole transporting layer
303: Light emitting layer
304: Hole blocking layer
305: Electron transporting layer
306: Electron injection layer
400: Negative electrode

### [Best Mode]

Hereinafter, the present application will be described in detail.

A hetero-cyclic compound according to an exemplary embodiment of the present application is represented by any one of Chemical Formulae 1 to 3. More specifically, the hetero-cyclic compound represented by any one of Chemical Formulae 1 to 3 may be used as a material for an organic material layer of an organic light emitting device by the structural characteristics of the core structure and the substituent as described above.

In an exemplary embodiment of the present application, Chemical Formula 1 may be represented by any one of the following Chemical Formulae 1a to 1j.

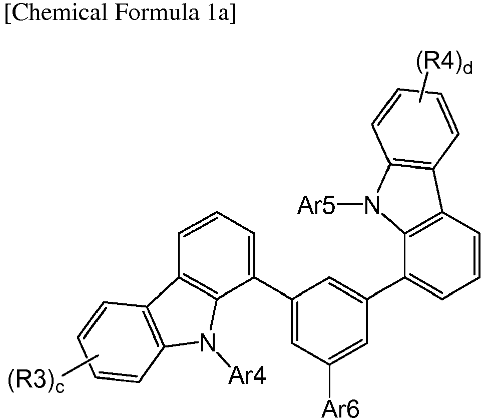

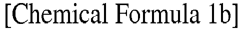

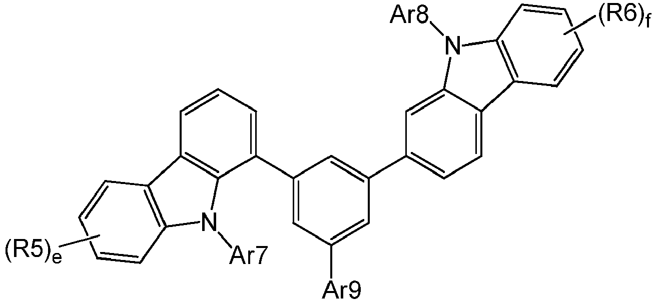

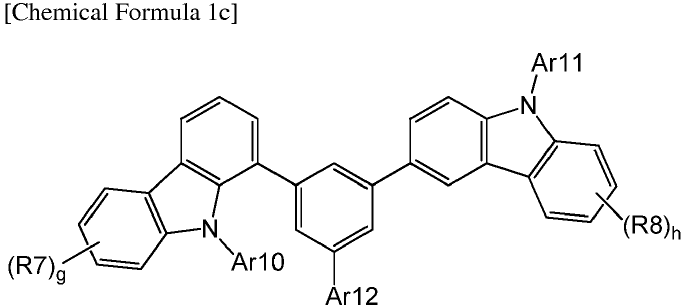

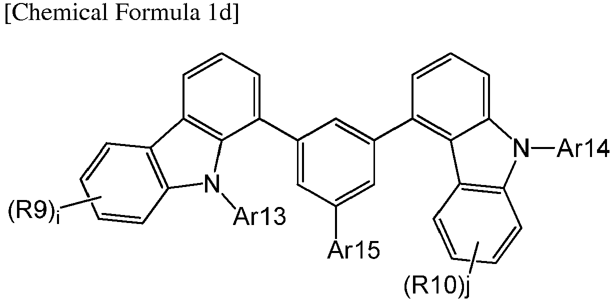

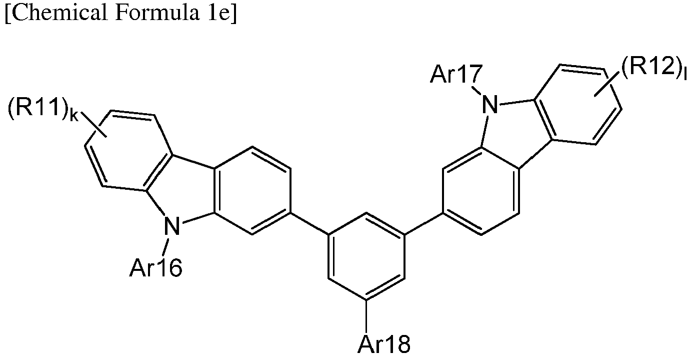

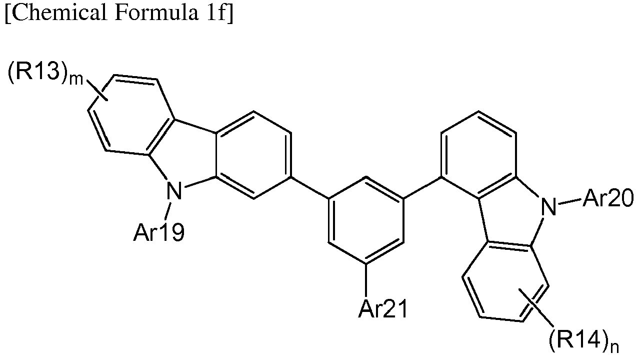

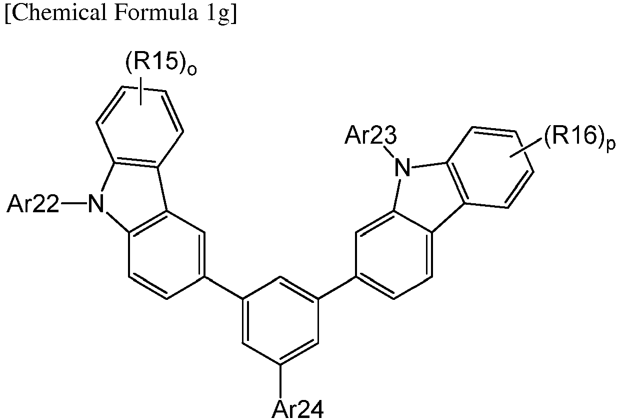

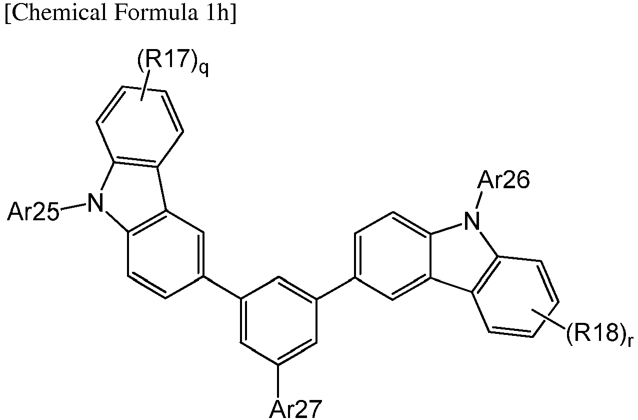

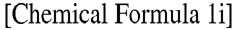

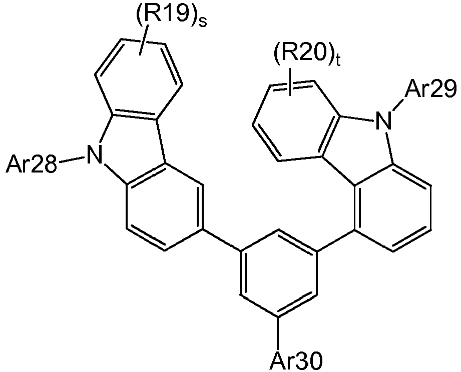

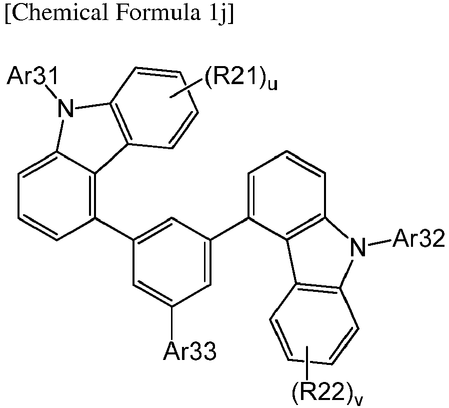

In Chemical Formulae 1a to 1j,
the definitions of Ar4 to Ar32 are the same as those of Ar1 to Ar4 in Chemical Formula 1,
the definitions of R3 to R22 are the same as those of R1 and R2 in Chemical Formula 1, and
the definitions of c, d, e, f, g, h, i, j, k, 1, m, n, o, p, q, r, s, t, u, and v are the same as those of a and b in Chemical Formula 1.

In an exemplary embodiment of the present application, R1 and R2 of Chemical Formula 1 may be each independently hydrogen or deuterium.

In the present application, the substituents of the chemical formulae will be more specifically described as follows.

In the present specification, "substituted or unsubstituted" means being unsubstituted or substituted with one or more substituents selected from the group consisting of deuterium; a halogen group; -CN; a C₁ to C₆₀ alkyl group; a C₂ to C₆₀ alkenyl group; a C₂ to C₆₀ alkynyl group; a C₃ to C₆₀ cycloalkyl group; a C₂ to C₆₀ heterocycloalkyl group; a C₆ to C₆₀ aryl group; a C₂ to C₆₀ heteroaryl group; -SiRR'R"; -P(=O)RR'; a C₁ to C₂₀ alkylamine group; a C₆ to C₆₀ arylamine group; and a C₂ to C₆₀ heteroarylamine group, being unsubstituted or substituted with a substituent to which two or more substituents among the substituents are bonded, or being unsubstituted or substituted with a substituent to which two or more substituents selected among the substituents are linked. For example, "the substituent to which two or more substituents are linked" may be a biphenyl group. That is, the biphenyl group may also be an aryl group, and may be interpreted as a substituent to which two phenyl groups are linked. The additional substituents may also be additionally substituted. R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group.

According to an exemplary embodiment of the present application, the "substituted or unsubstituted" means being unsubstituted or substituted with one or more substituents selected from the group consisting of deuterium, a halogen group, -CN, SiRR'R", P(=O)RR', a C₁ to C₂₀ straight or branched alkyl group, a C₆ to C₆₀ aryl group, and a C₂ to C₆₀ heteroaryl group, and
R, R', and R" are the same as or different from each other, and are each independently hydrogen; deuterium; -CN; a C₁ to C₆₀ alkyl group which is unsubstituted or substituted with deuterium, a halogen group, -CN, a C₁ to C₂₀ alkyl group, a C₆ to C₆₀ aryl group, and a C₂ to C₆₀ heteroaryl group; a C₃ to C₆₀ cycloalkyl group which is unsubstituted or substituted with deuterium, halogen, -CN, a C₁ to C₂₀ alkyl group, a C₆ to C₆₀ aryl group, and a C₂ to C₆₀ heteroaryl group; a C₆ to C₆₀ aryl group which is unsubstituted or substituted with deuterium, halogen, -CN, a C₁ to C₂₀ alkyl group, a C₆ to C₆₀ aryl group, and a C₂ to C₆₀ heteroaryl group; or a C₂ to C₆₀ heteroaryl group which is unsubstituted or substituted with deuterium, halogen,-CN, a C₁ to C₂₀ alkyl group, a C₆ to C₆₀ aryl group, and a C₂ to C₆₀ heteroaryl group.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the halogen may be fluorine, chlorine, bromine or iodine.

In the present specification, the alkyl group comprises a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group comprises a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group comprises a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, the cycloalkyl group comprises a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group comprises O, S, Se, N, or Si as a heteroatom, comprises a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group comprises a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group comprises a spiro group. The number of carbon atoms of the aryl group may be 6 to 60, specifically 6 to 40, and more specifically 6 to 25. Specific examples of the aryl group comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the spiro group is a group comprising a spiro structure, and may have 15 to 60 carbon atoms. For example, the spiro group may comprise a structure in which a 2,3-dihydro-1H-indene group or a cyclohexane group is spiro-bonded to a fluorenyl group. Specifically, the spiro group may comprise any one of the groups of the following structural formulae.

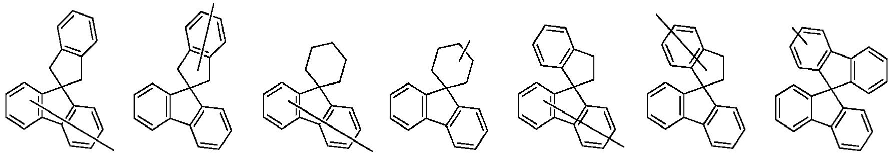

In the present specification, the heteroaryl group comprises S, O, Se, N, or Si as a heteroatom, comprises a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, the arylene group means that there are two bonding positions in an aryl group, that is, a divalent group. The above-described description on the aryl group may be applied to the arylene group, except for a divalent arylene group. Further, the heteroarylene group means that there are two bonding positions in a heteroaryl group, that is, a divalent group. The above-described description on the heteroaryl group may be applied to the heteroarylene group, except for a divalent heteroarylene group.

According to an exemplary embodiment of the present application, the compound represented by Chemical Formula 1 may be represented by any one of the following compounds, but is not limited thereto.

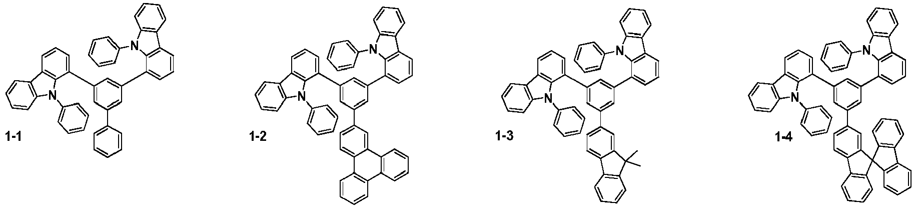

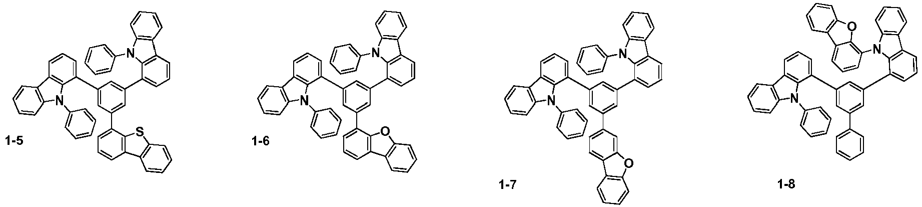

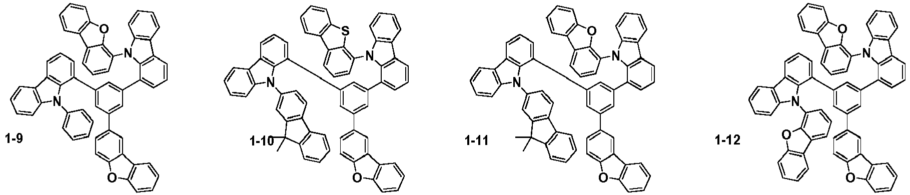

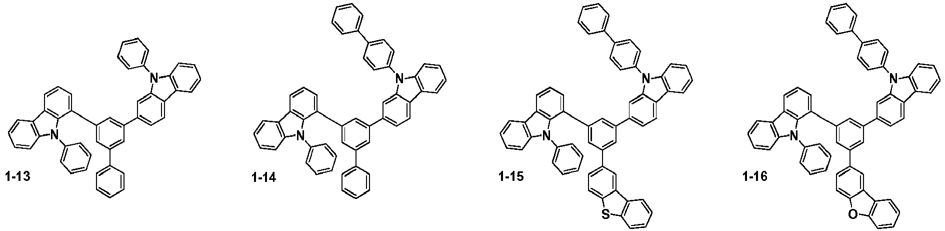

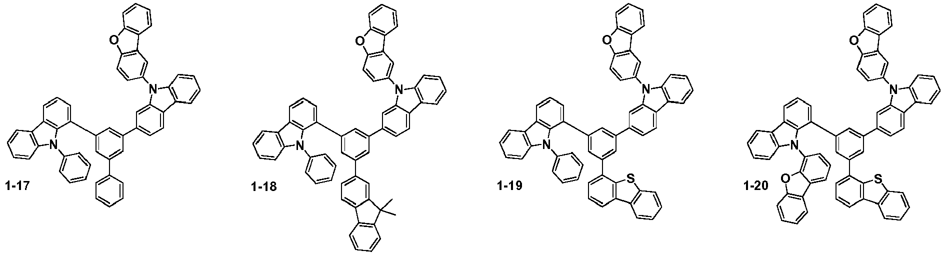

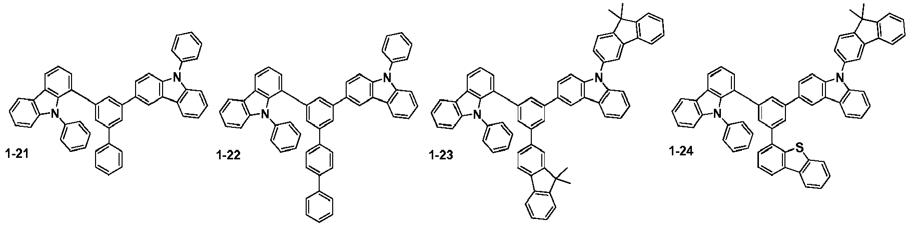

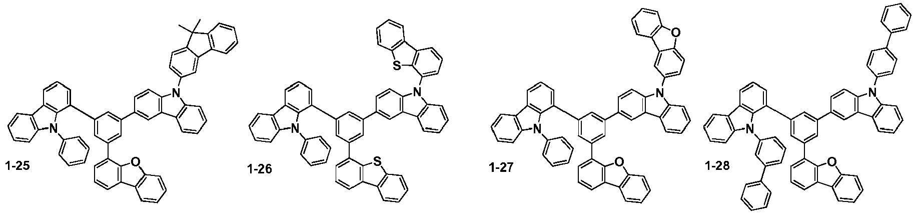

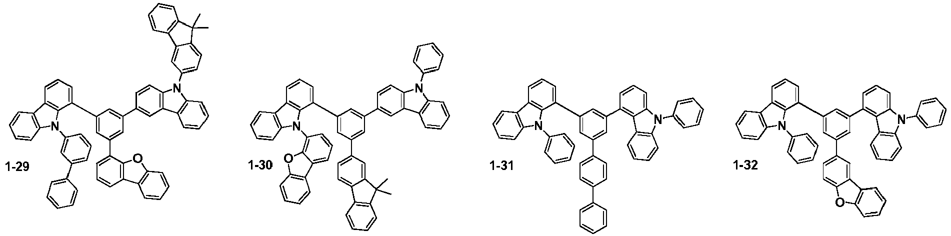

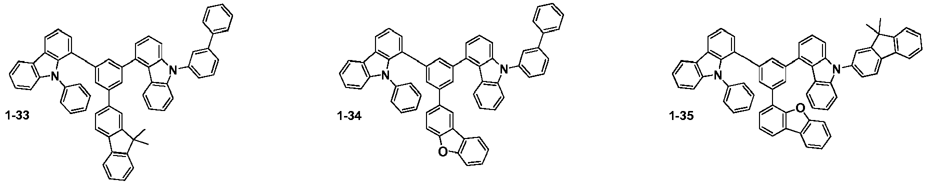

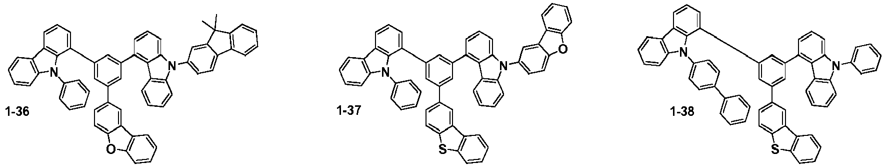

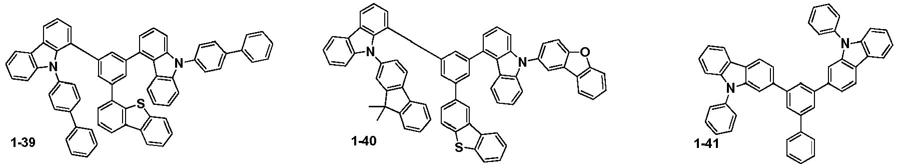

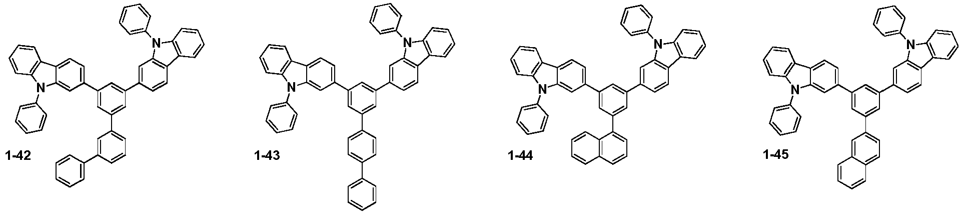

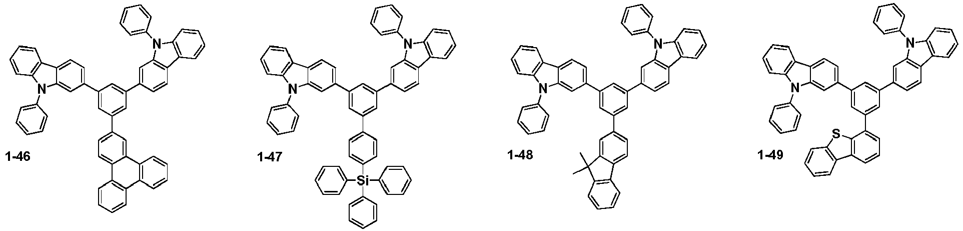

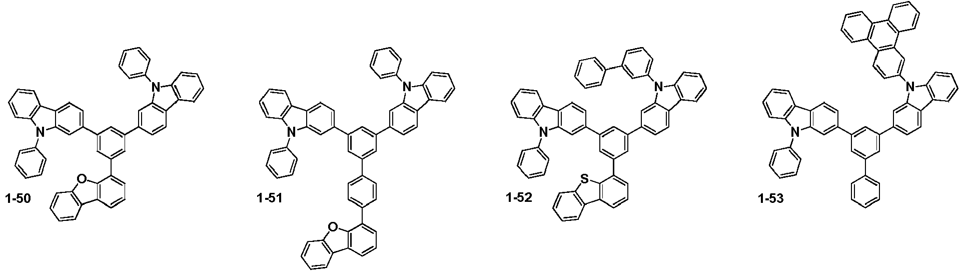

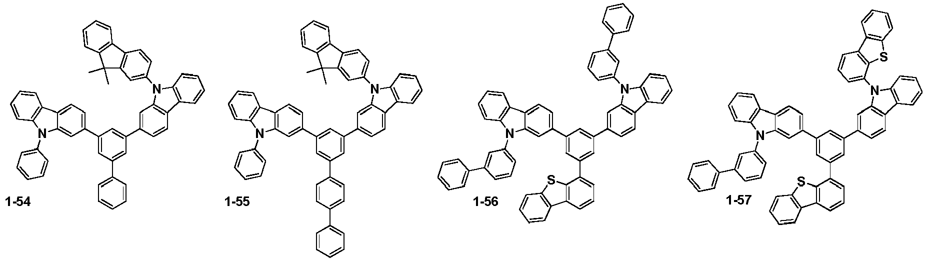

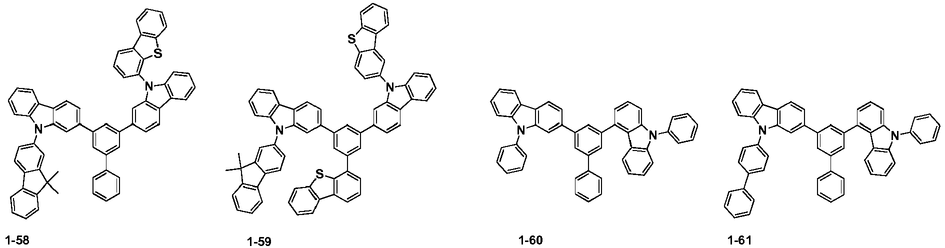

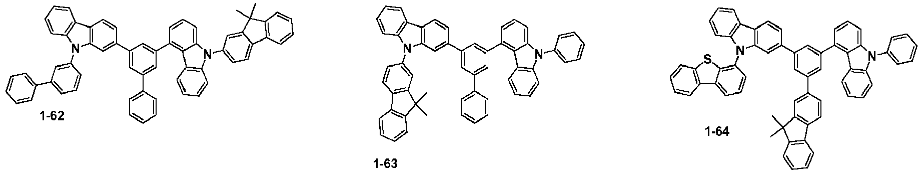

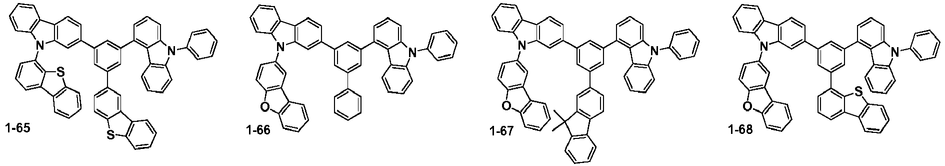

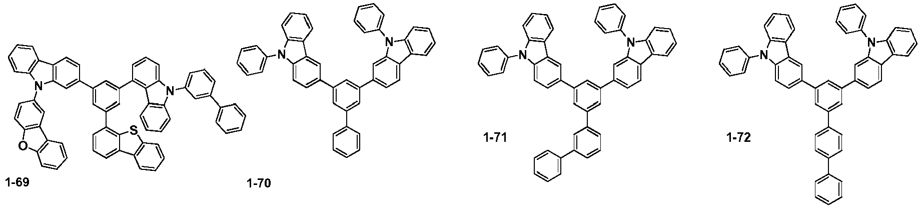

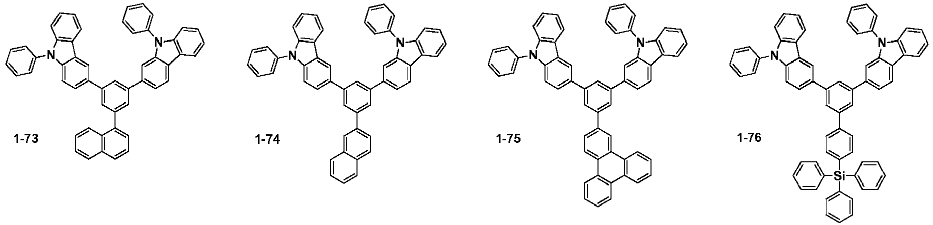

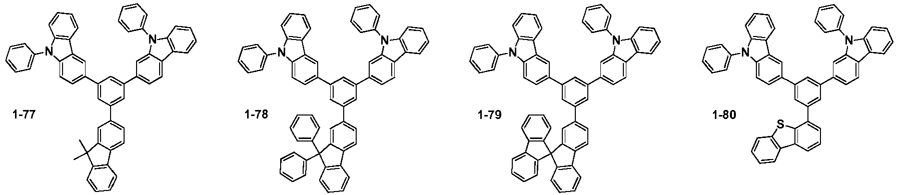

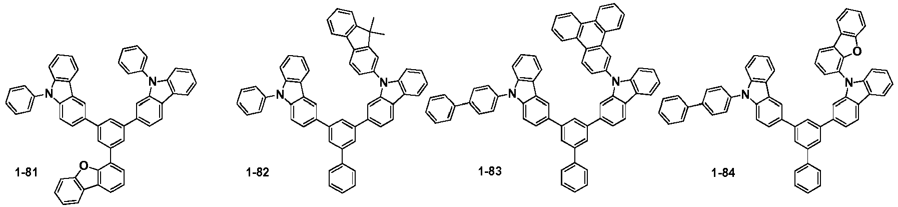

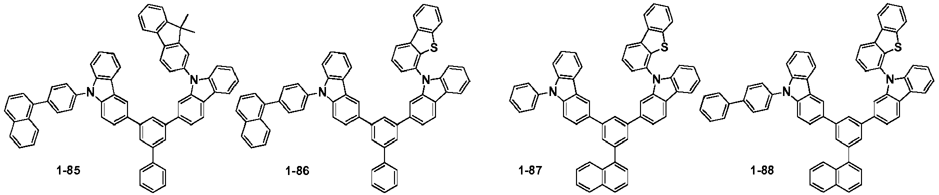

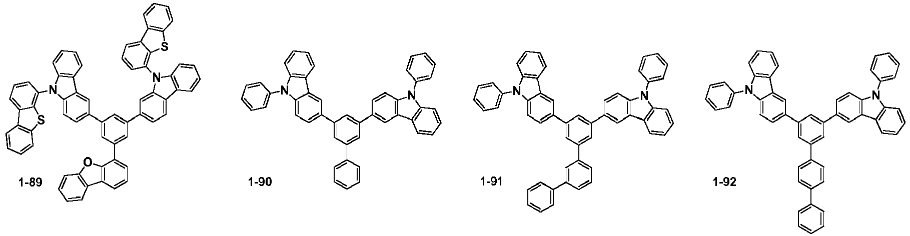

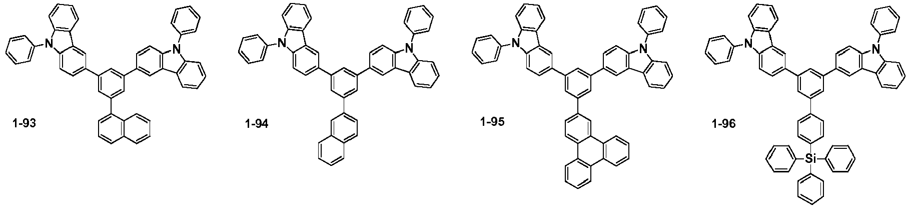

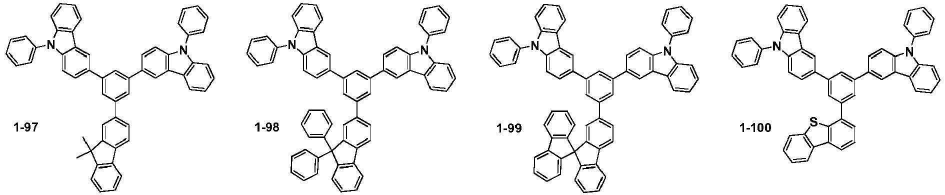

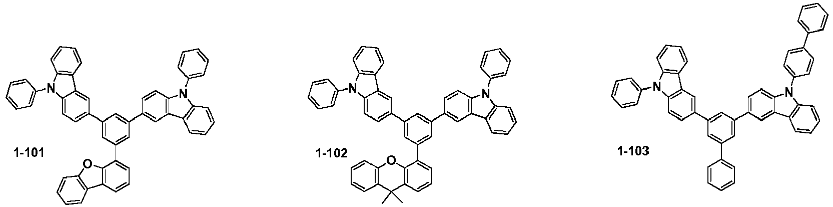

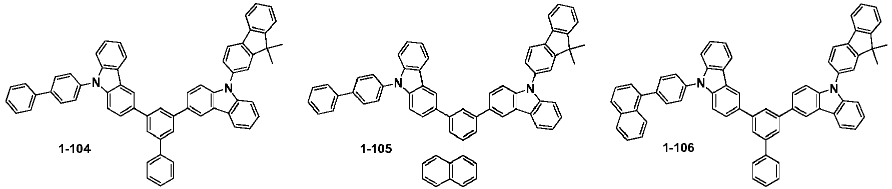

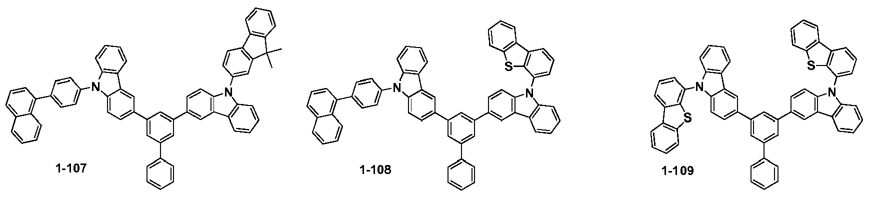

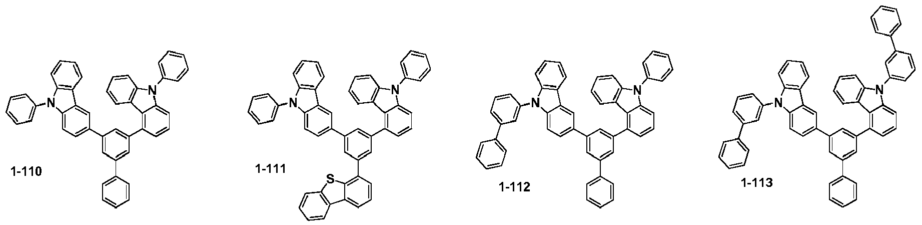

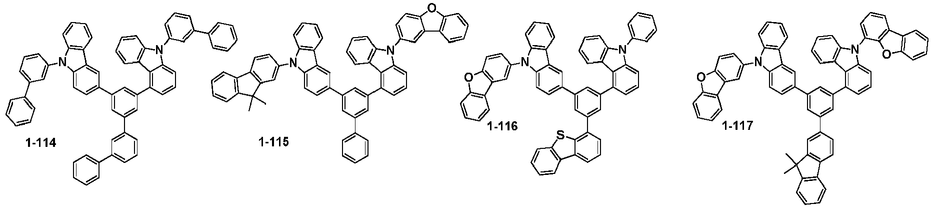

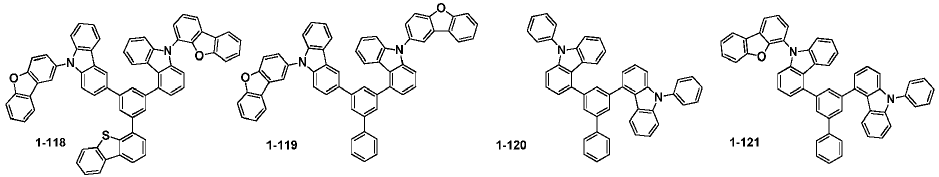

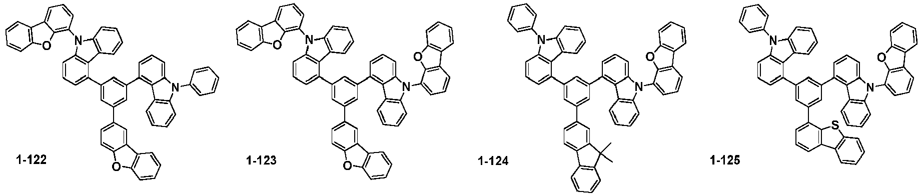

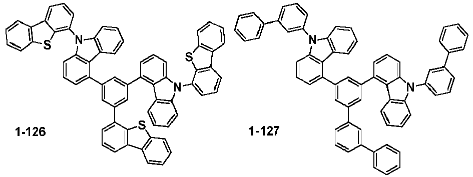

Further, it is possible to synthesize a compound having inherent characteristics of a substituent introduced by introducing various substituents into the structure of Chemical Formula 1. For example, a substituent usually used for a hole injection layer material, a material for transporting holes, a light emitting layer material, and an electron transporting layer material, which are used for manufacturing an organic light emitting device, may be introduced into the core structure to synthesize a material which satisfies conditions required for each organic material layer.

**In** addition, it is possible to finely adjust an energy band gap by introducing various substituents into the structure of Chemical Formula 1, and meanwhile, it is possible to improve characteristics at the interface between organic materials and diversify the use of material.

Meanwhile, the hetero-cyclic compound has a high glass transition temperature (Tg) and thus has excellent thermal stability. The increase in thermal stability becomes an important factor which provides driving stability to a device.

The hetero-cyclic compound according to an exemplary embodiment of the present application may be prepared by a multi-step chemical reaction. Some intermediate compounds are first prepared, and the compound of Chemical Formula 1 may be prepared from the intermediate compounds. More specifically, the hetero-cyclic compound according to an exemplary embodiment of the present application may be prepared based on the Preparation Examples to be described below.

An exemplary embodiment of the present application provides an organic light emitting device comprising a positive electrode, a negative electrode, and an organic material layer having one or more layers disposed between the positive electrode and the negative electrode, in which one or more layers of the organic material layer comprise the hetero-cyclic compound represented by Chemical Formula 1.

Another exemplary embodiment of the present application provides an organic light emitting device comprising a positive electrode, a negative electrode, and an organic material layer having one or more layers disposed between the positive electrode and the negative electrode, in which the organic material layer comprises a light emitting layer, and the light emitting layer comprises: the hetero-cyclic compound represented by Chemical Formula 1; and the compound represented by Chemical Formula 2 or 3.

In Chemical Formulae 4 and 5, * denotes a position to be linked to L2 of Chemical Formula 2.

According to an exemplary embodiment of the present application, Chemical Formula 4 may be represented by any one of the following Chemical Formulae.

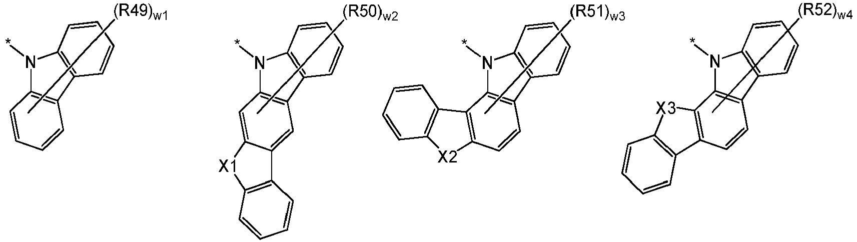

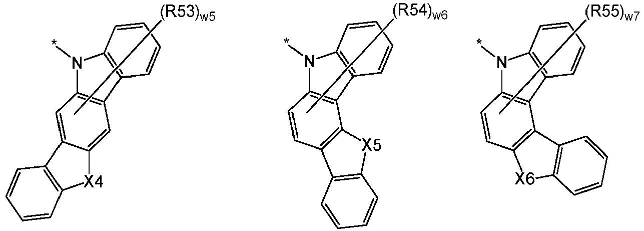

In the structural formulae, X1 to X6 are the same as or different from each other, and are each independently NR, S, O, or CR'R",

R49 to R55 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,

R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
w1 is an integer from 0 to 8, and w2 to w7 are each independently an integer from 0 to 6.

According to an exemplary embodiment of the present application, Chemical Formula 5 may be represented by any one of the following structural formulae.

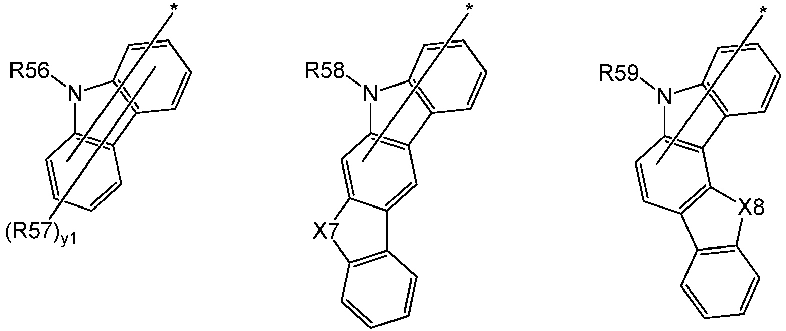

In the structural formulae, X7 and X8 are the same as or different from each other, and are each independently NR, S, O, or CR'R",
R56 to R59 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
y1 is an integer from 0 to 7.

In an exemplary embodiment of the present application, R24 to R28 of Chemical Formula 4 may be each independently hydrogen or deuterium.

According to an exemplary embodiment of the present application, the compound represented by Chemical Formula 2 may be represented by any one of the following compounds, but is not limited thereto.

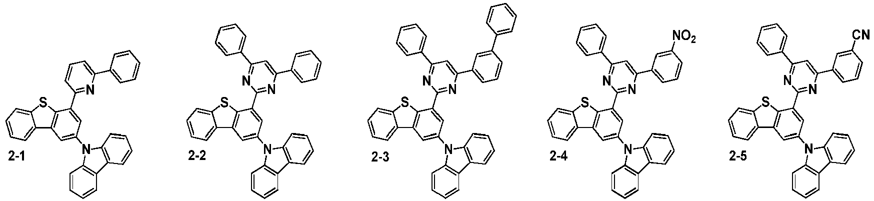

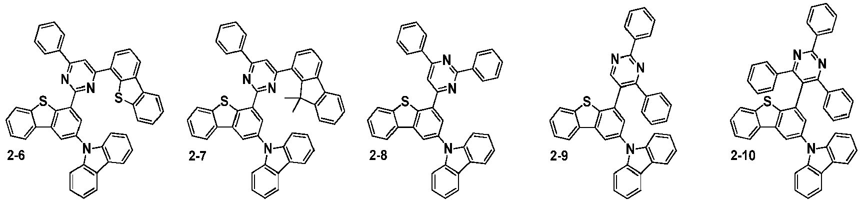

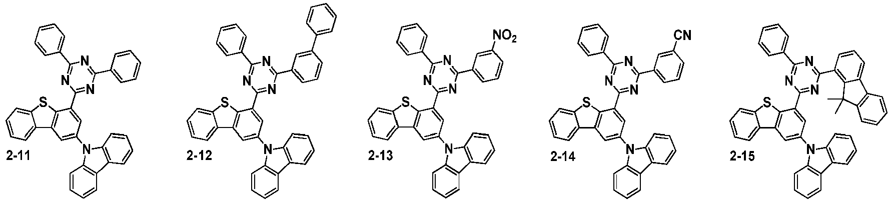

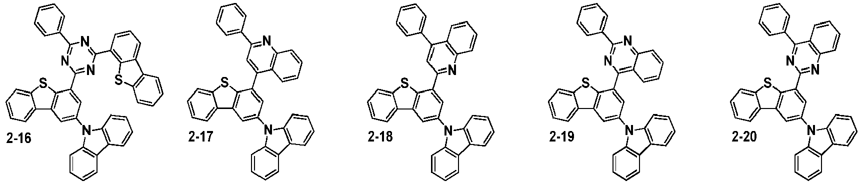

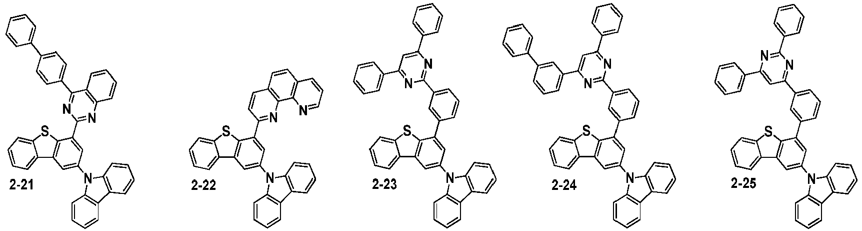

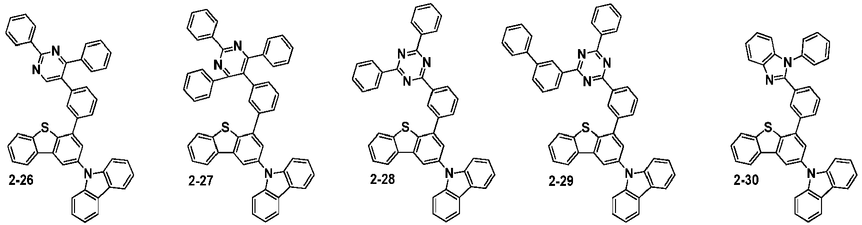

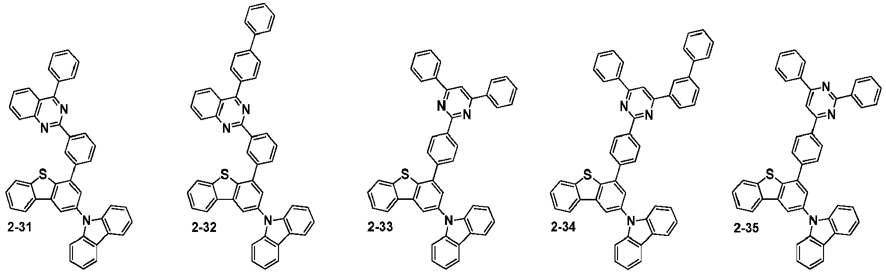

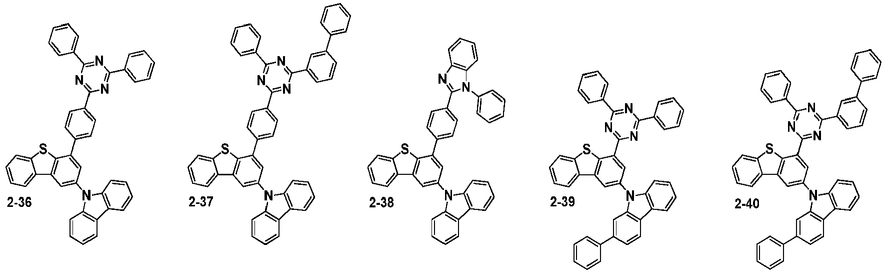

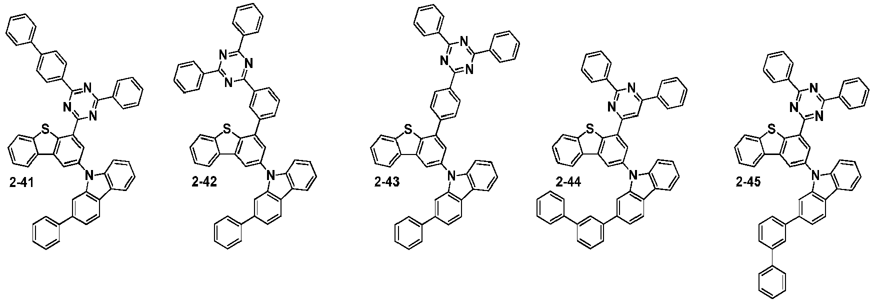

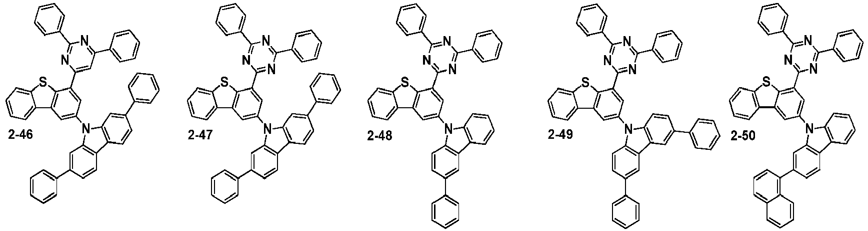

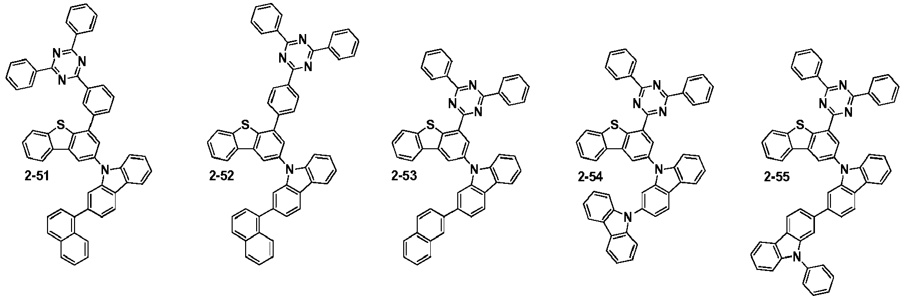

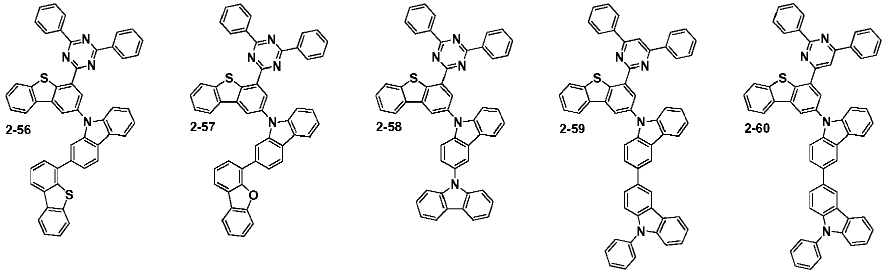

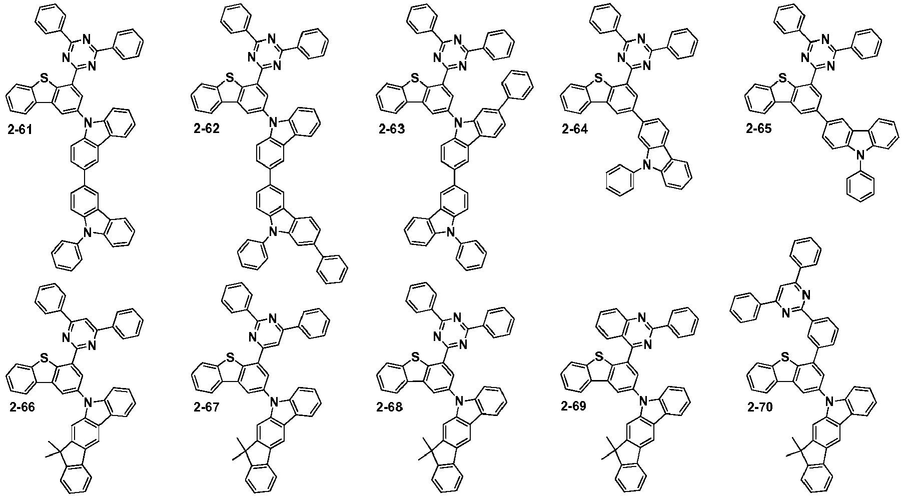

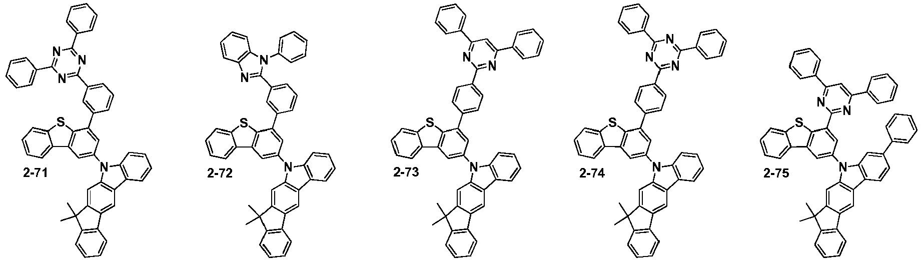

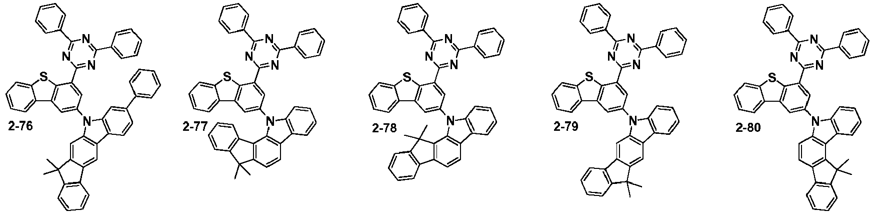

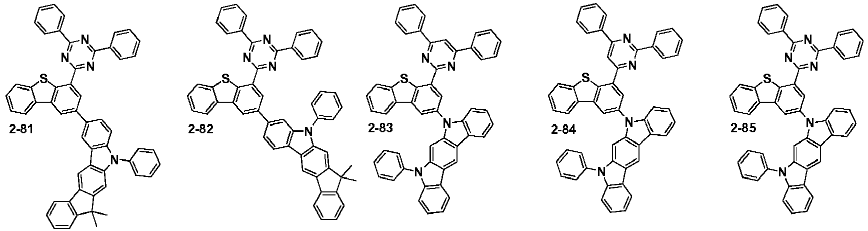

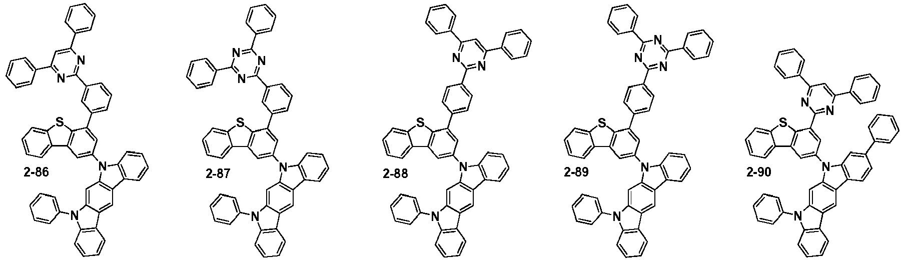

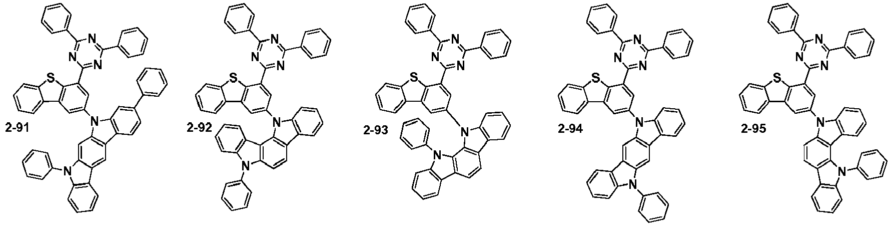

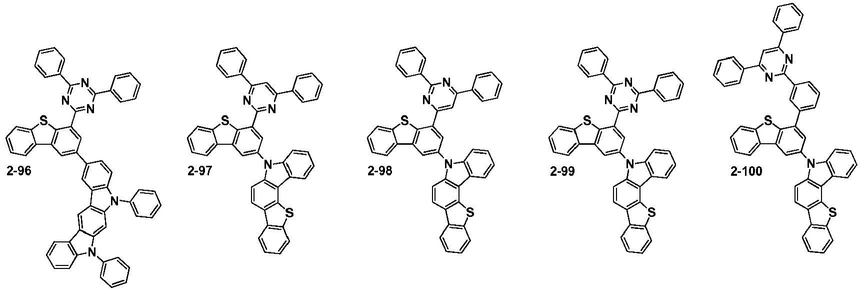

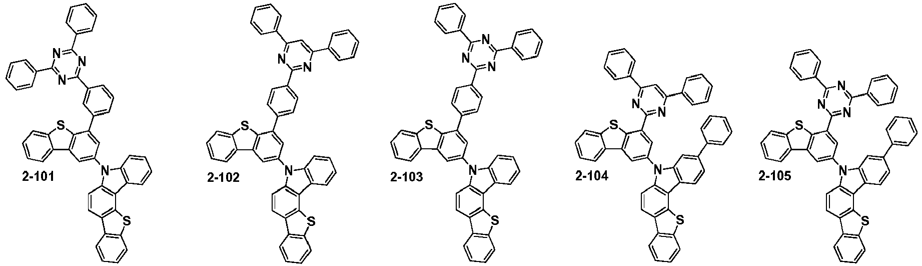

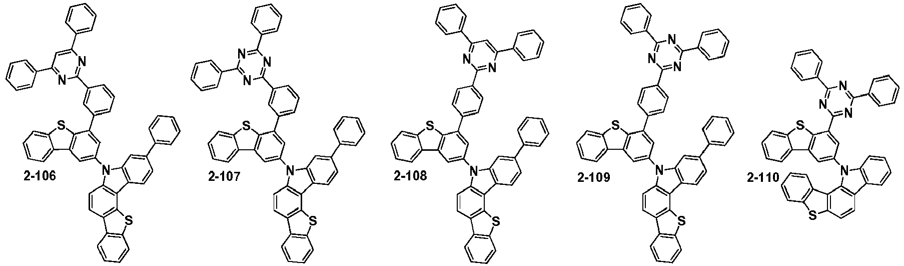

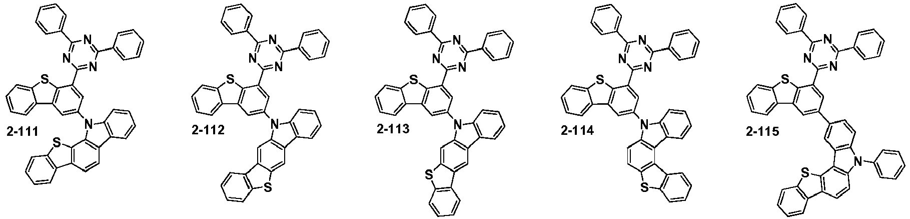

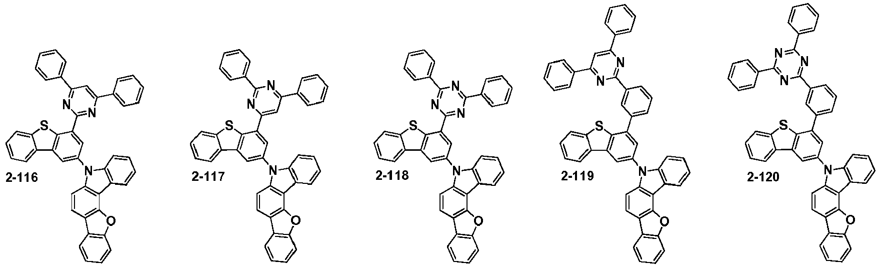

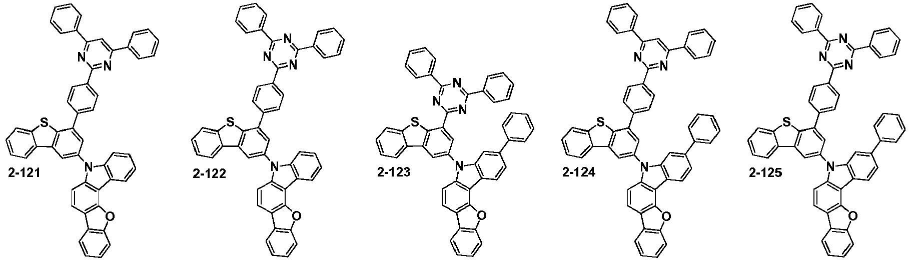

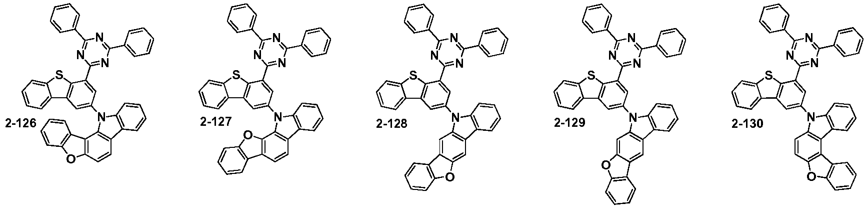

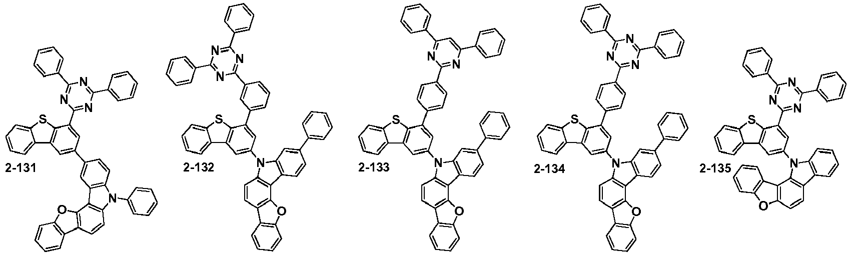

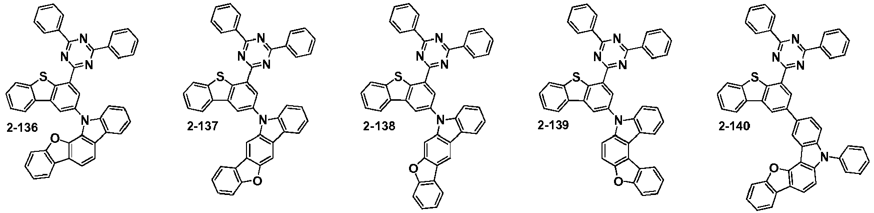

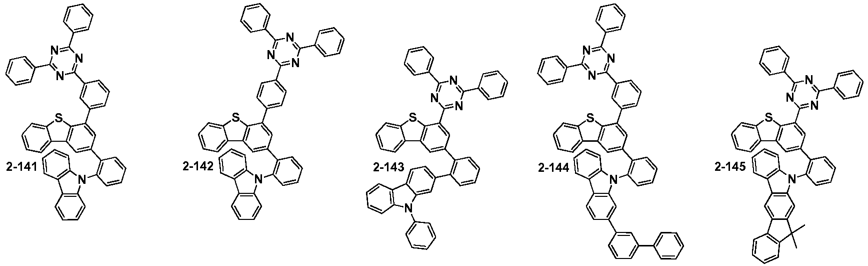

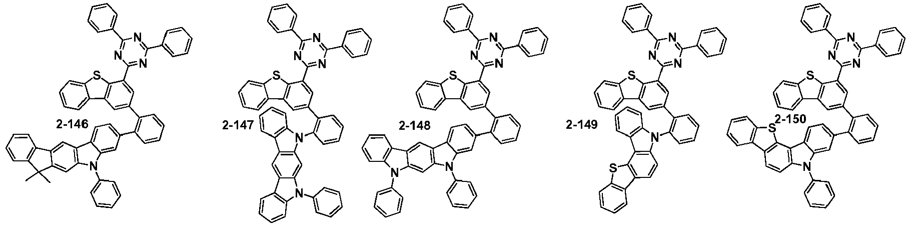

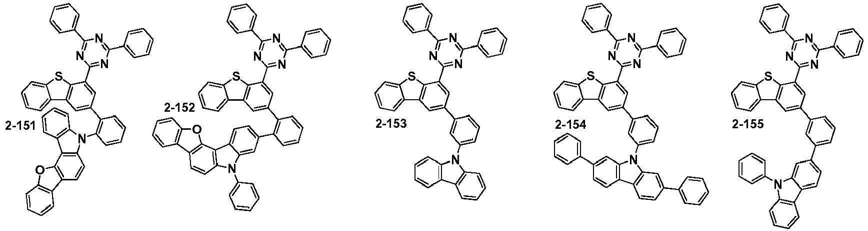

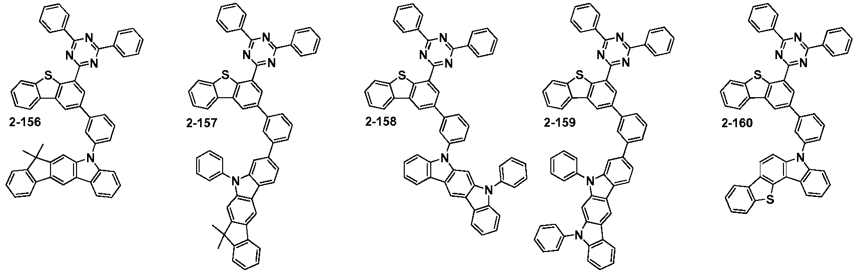

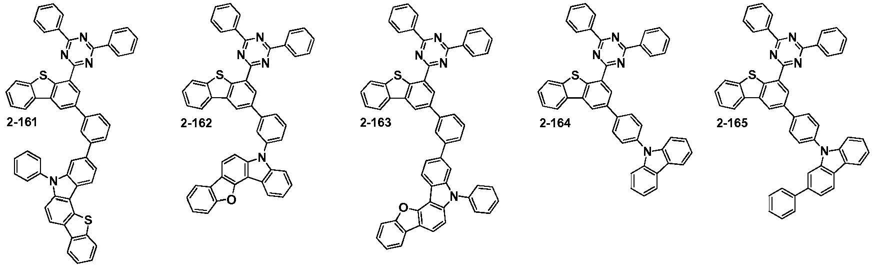

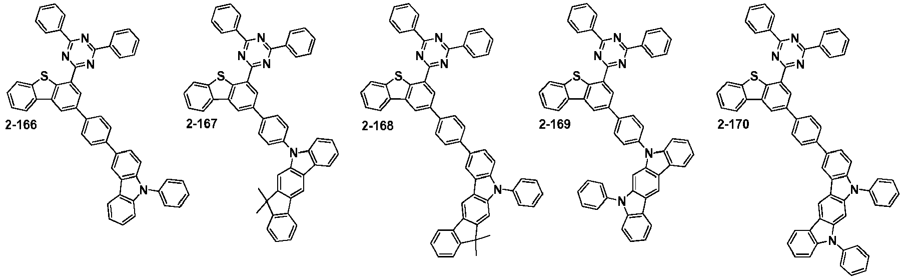

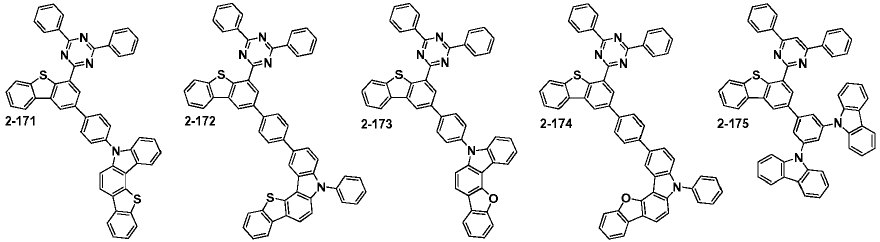

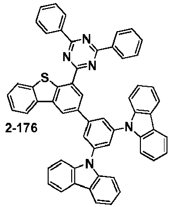

In an exemplary embodiment of the present application, in Chemical Formula 3, one of X1 to X3 may be N, two of X1 to X3 may be N, and all of X1 to X3 may be N.

In an exemplary embodiment of the present application, R30 and R31 of Chemical Formula 3 may be each independently a substituted or unsubstituted C₆ to C₆₀ aryl group; or a substituted or unsubstituted C₂ to C₆₀ heteroaryl group.

In an exemplary embodiment of the present application, R32 to R34 of Chemical Formula 3 may be each independently hydrogen or deuterium.

In an exemplary embodiment of the present application, any one of R35 to R39 of Chemical Formula 3 may be -CN, and the others may be each independently hydrogen or deuterium.

According to an exemplary embodiment of the present application, the compound represented by Chemical Formula 3 may be represented by any one of the following compounds, but is not limited thereto.

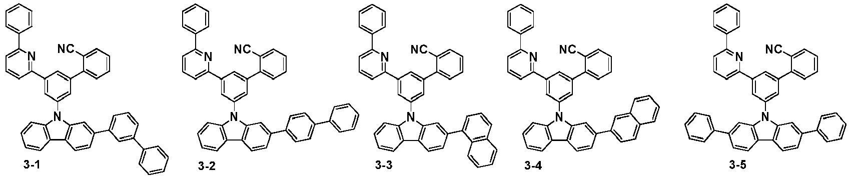

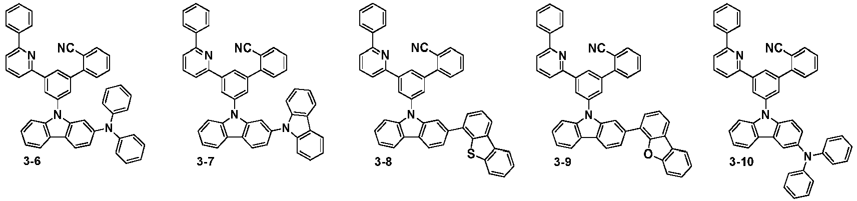

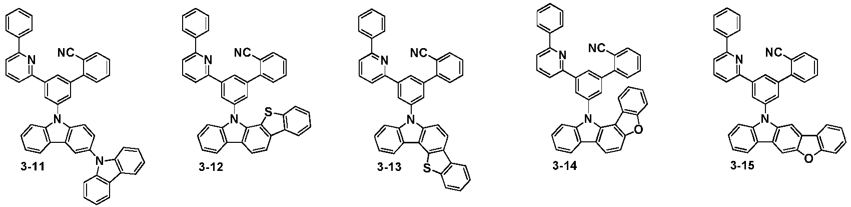

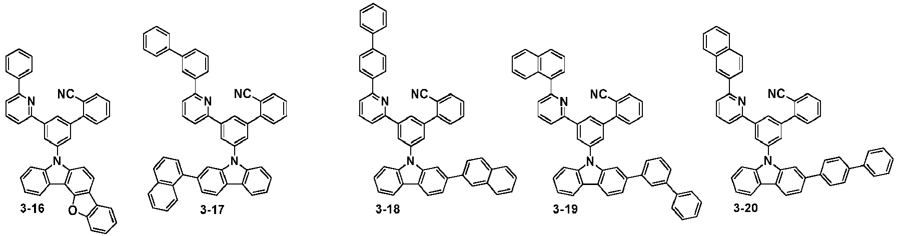

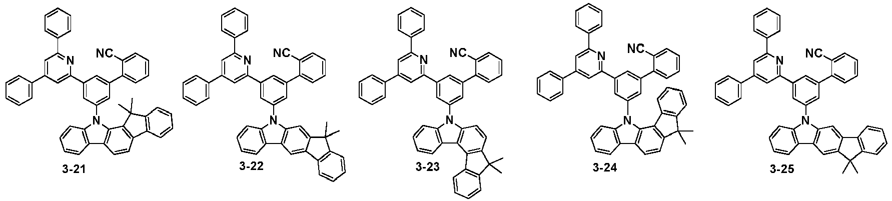

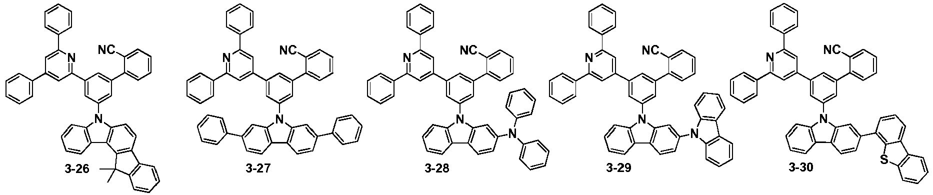

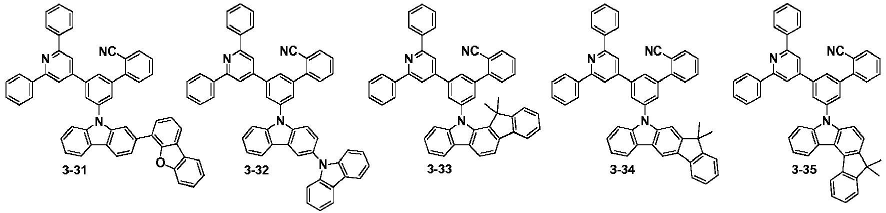

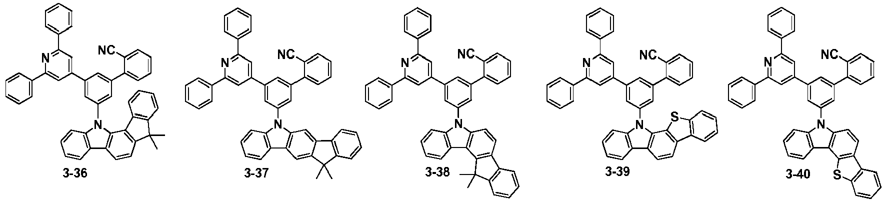

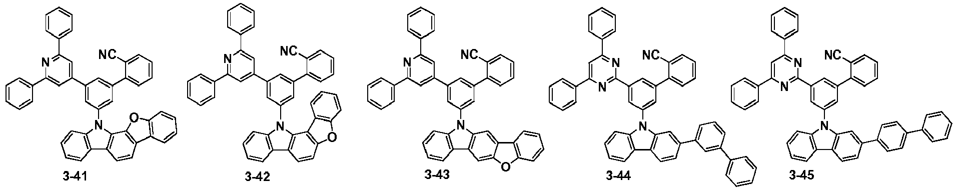

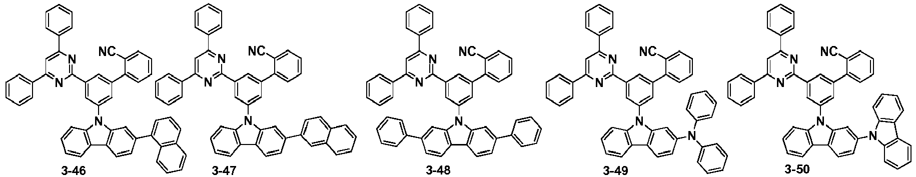

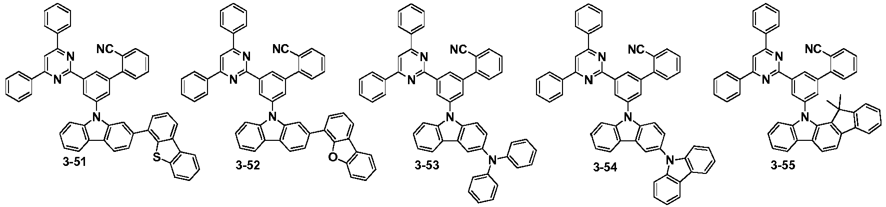

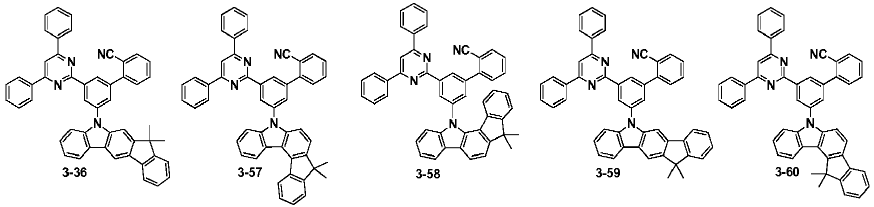

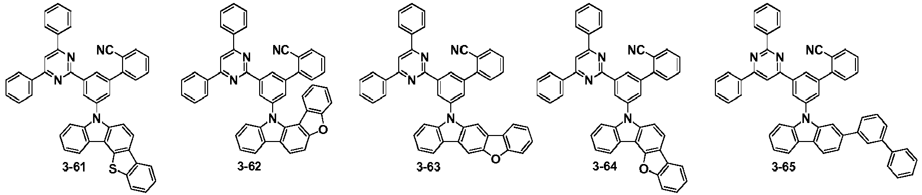

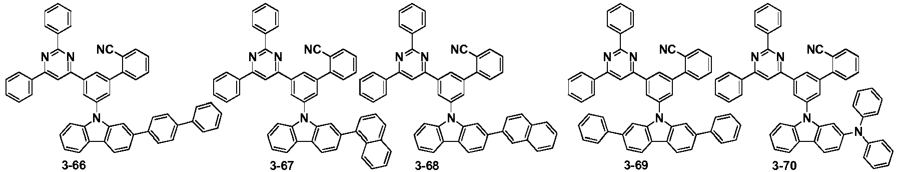

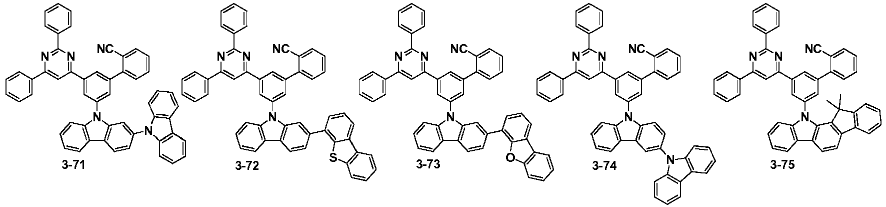

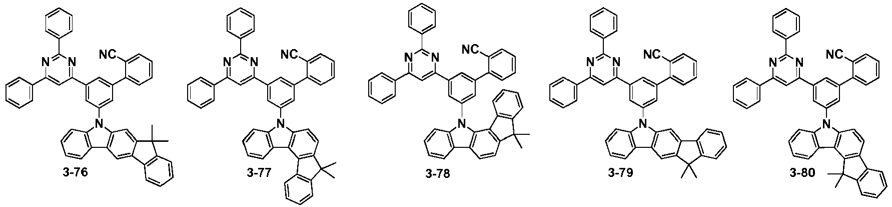

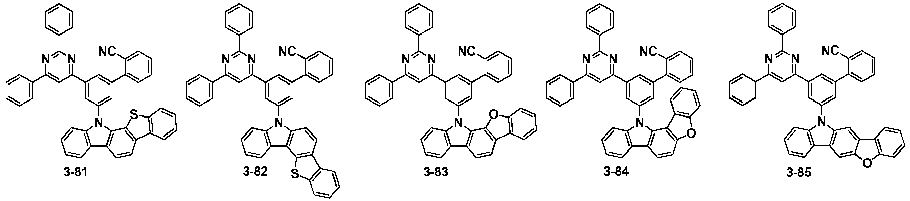

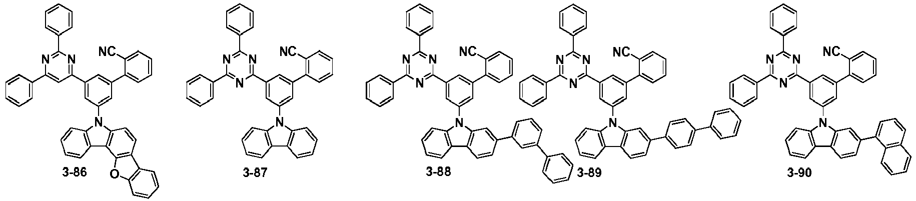

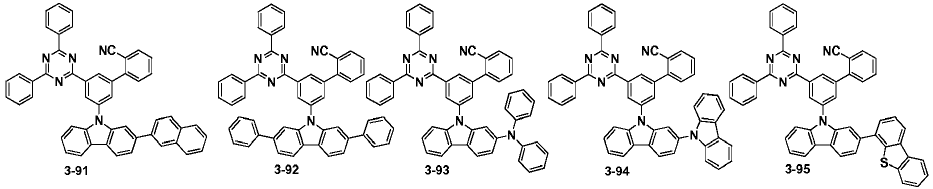

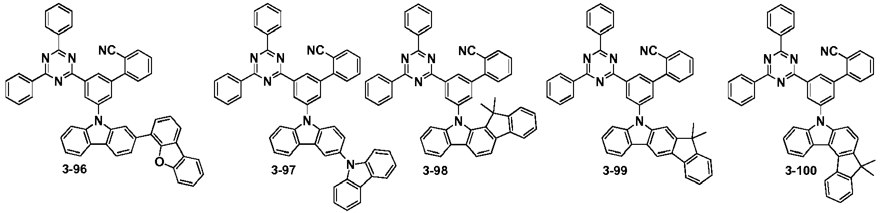

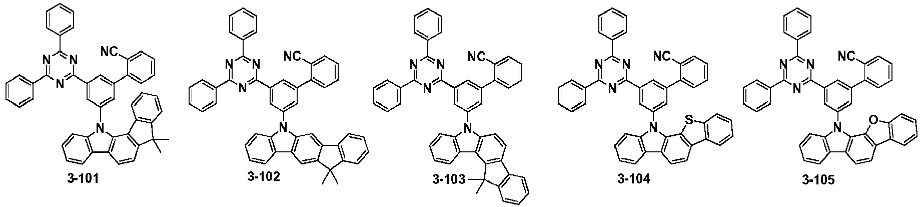

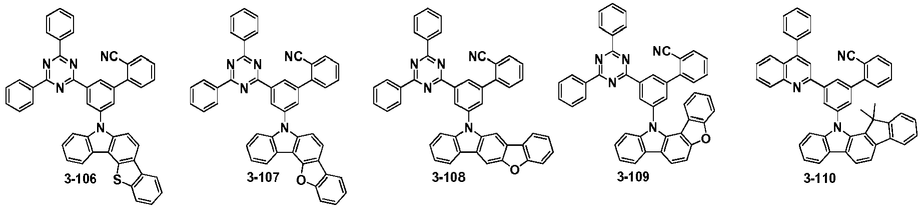

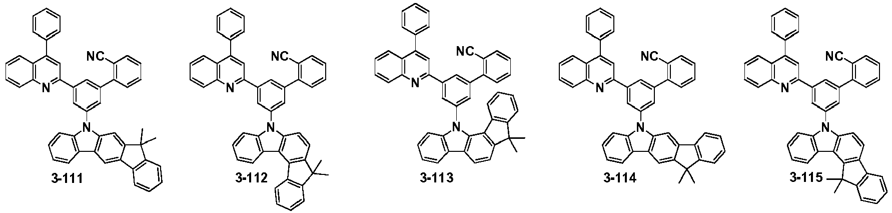

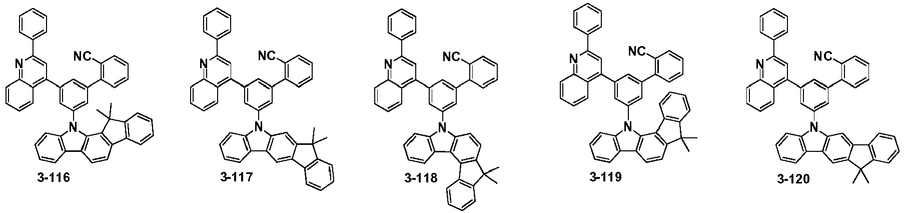

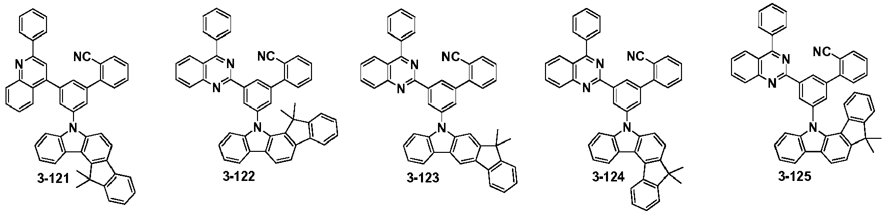

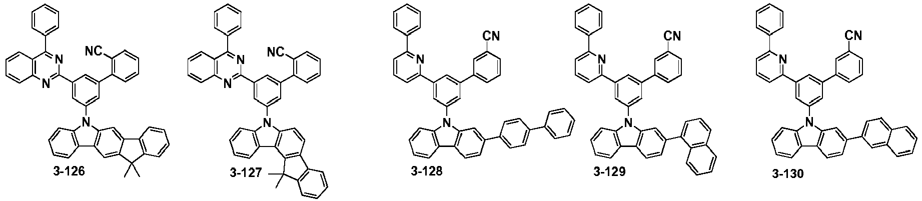

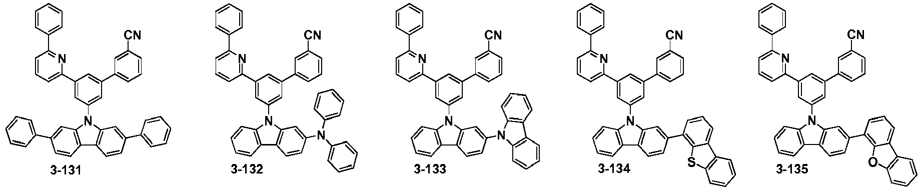

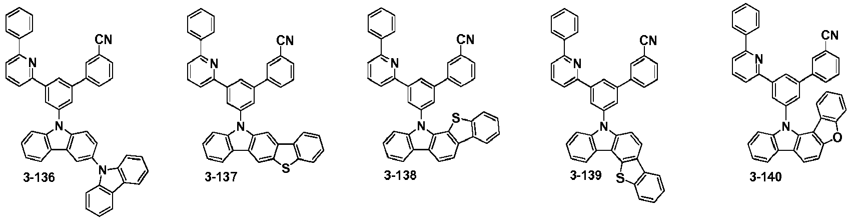

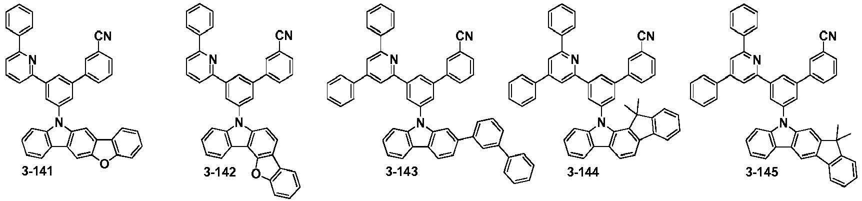

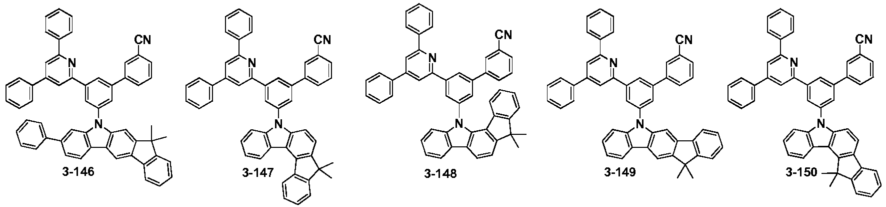

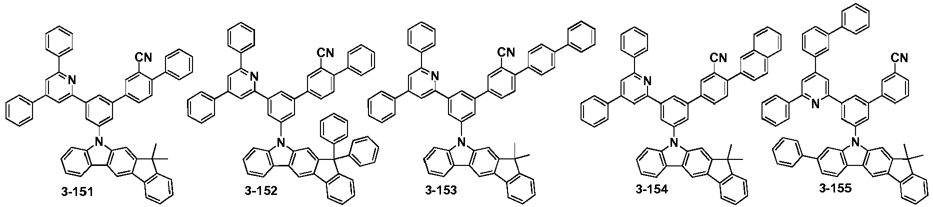

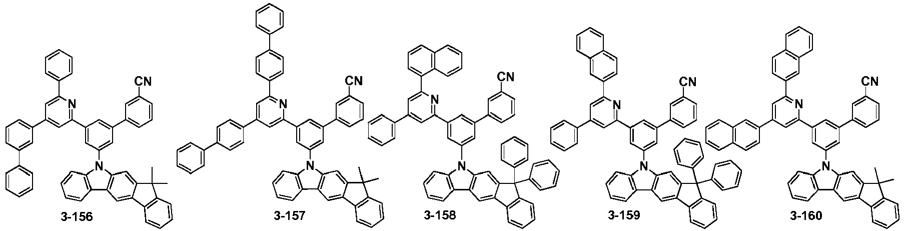

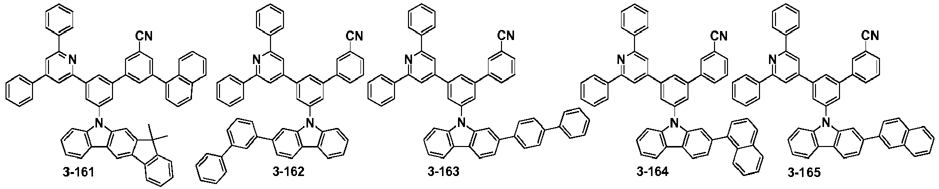

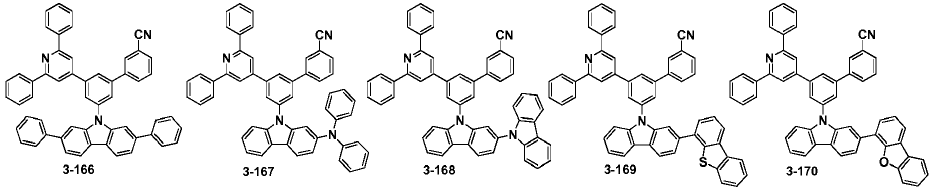

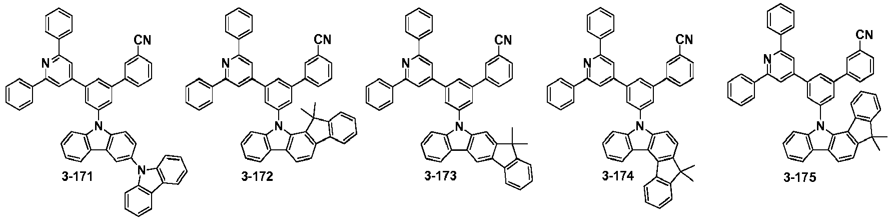

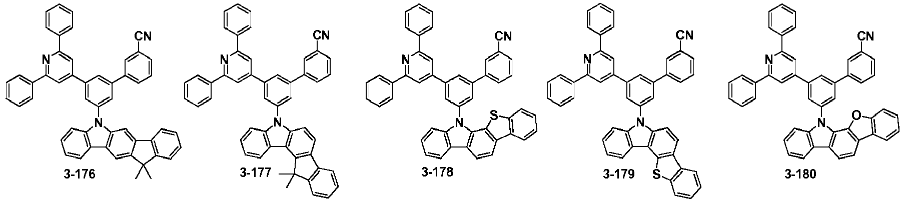

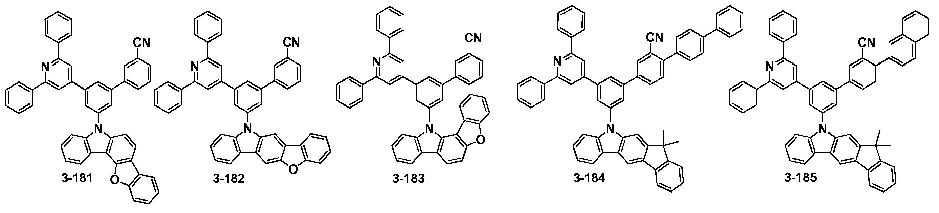

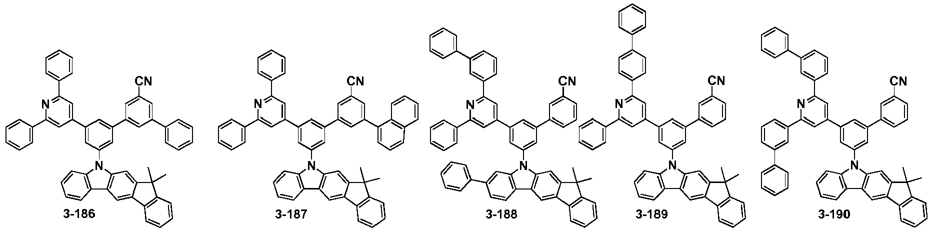

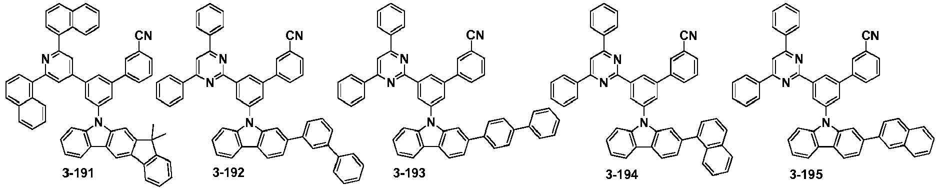

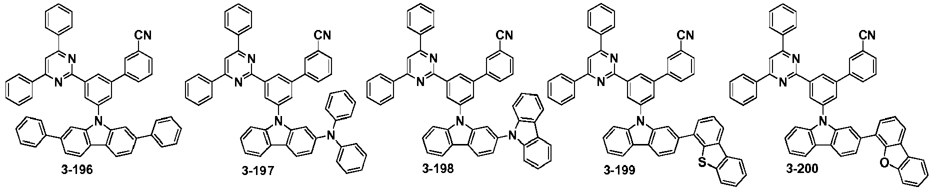

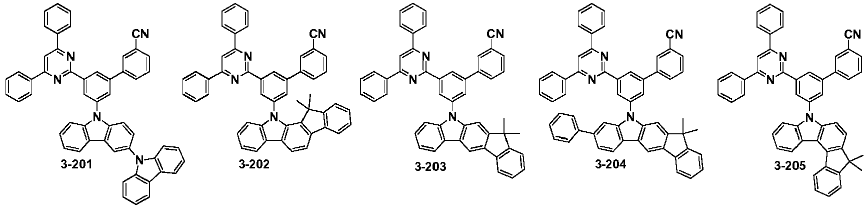

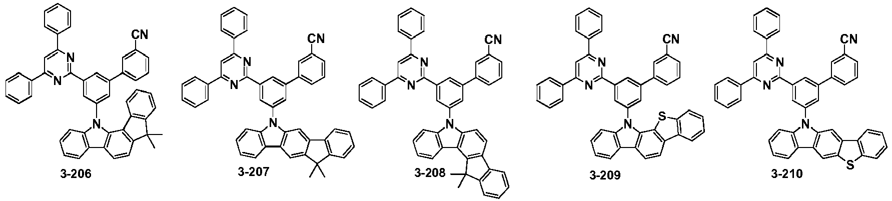

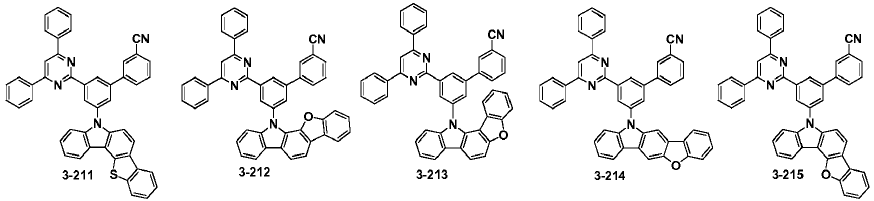

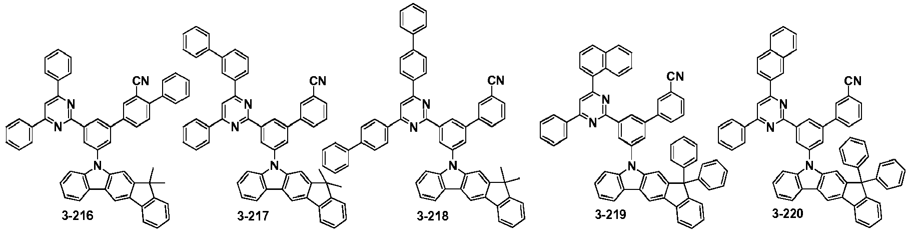

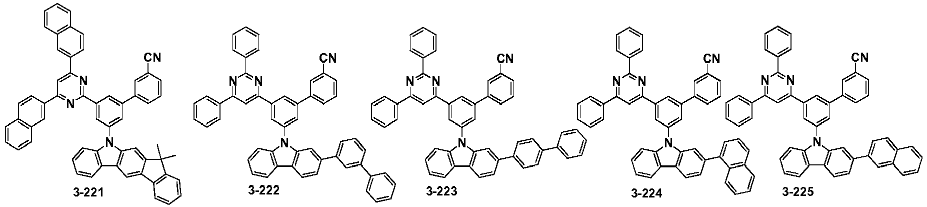

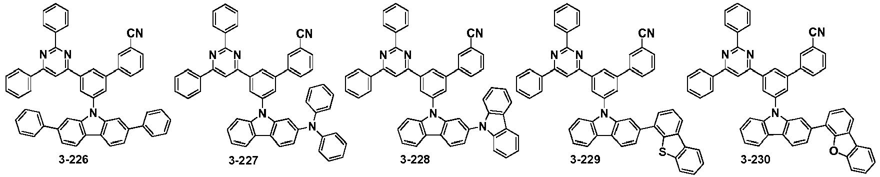

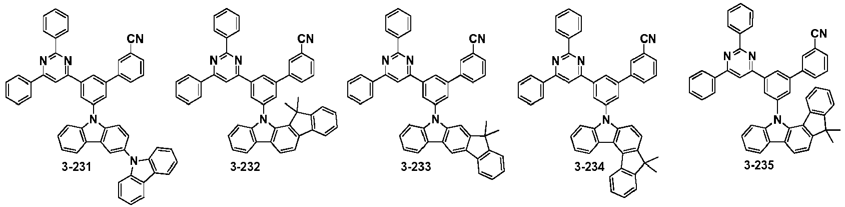

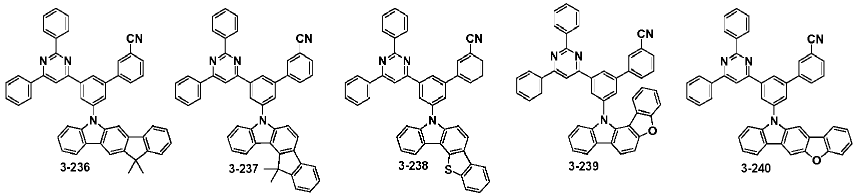

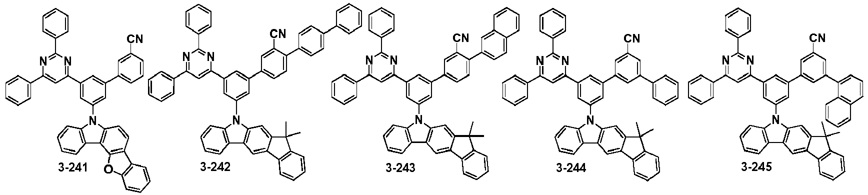

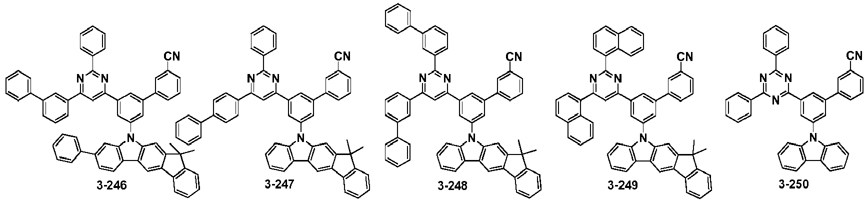

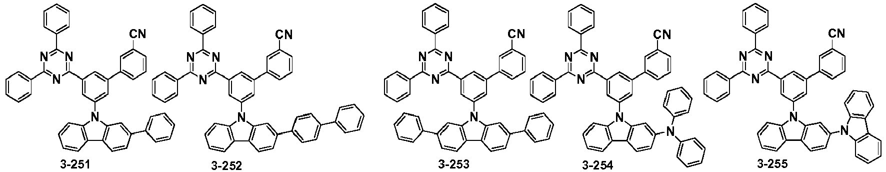

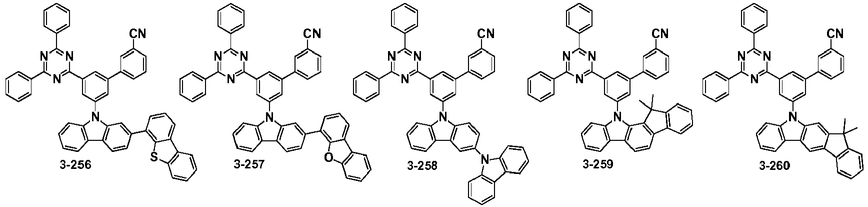

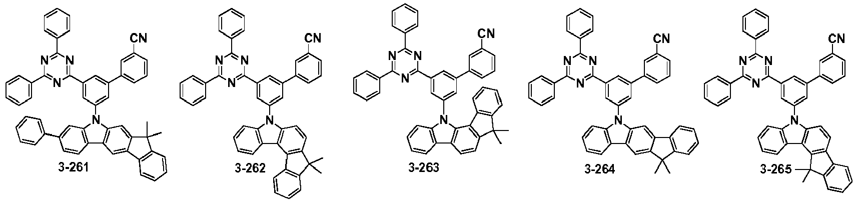

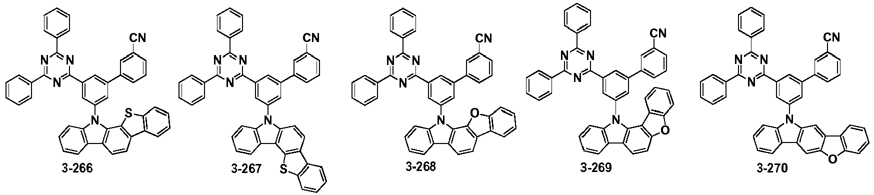

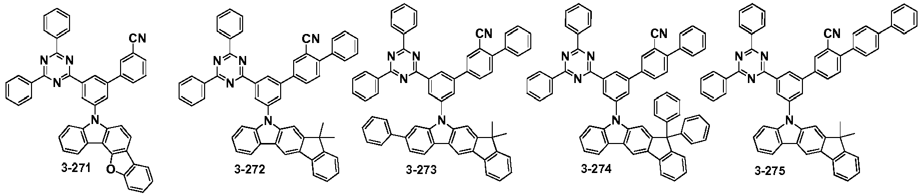

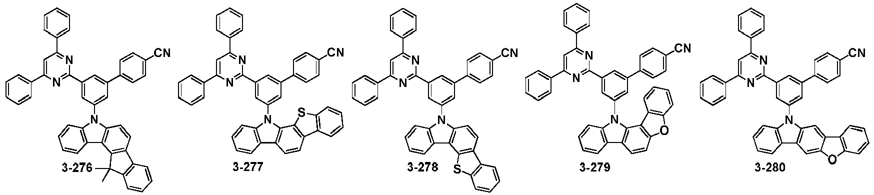

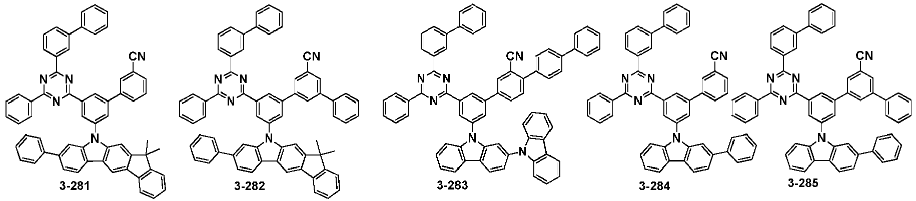

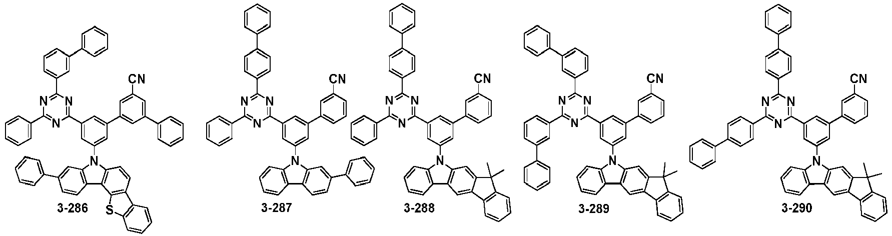

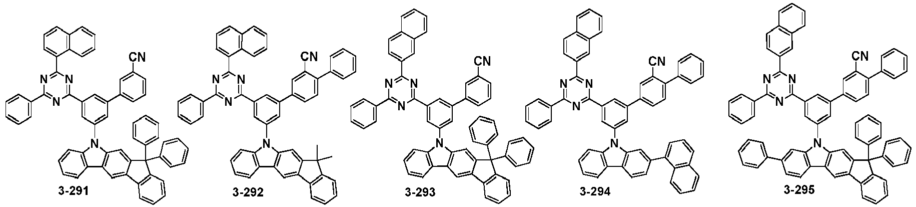

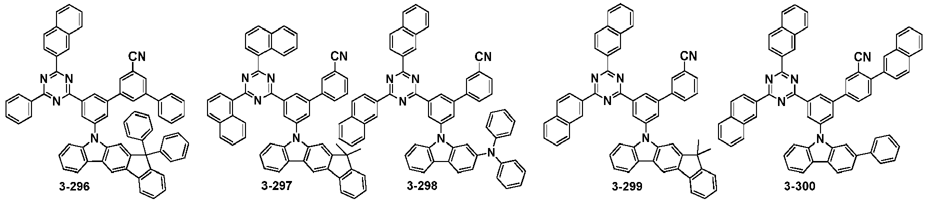

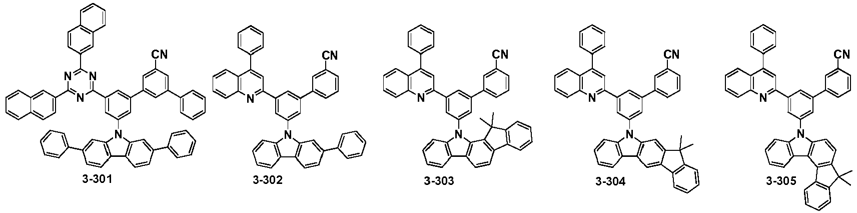

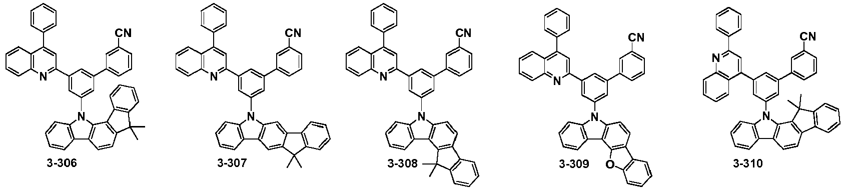

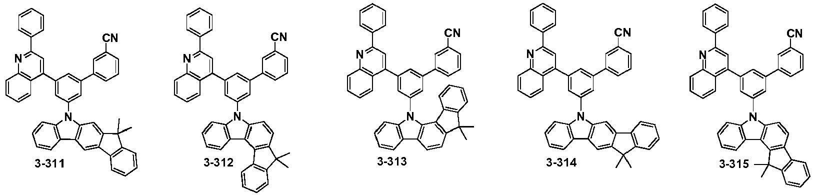

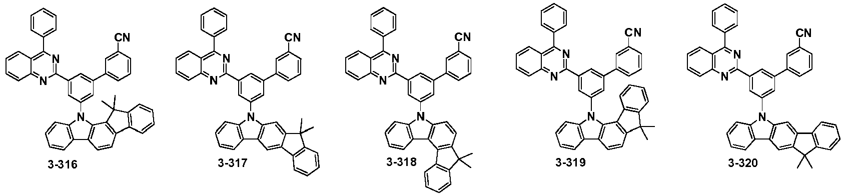

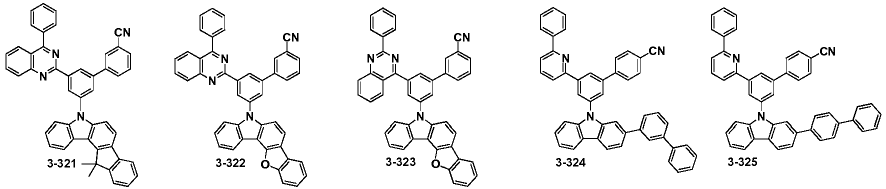

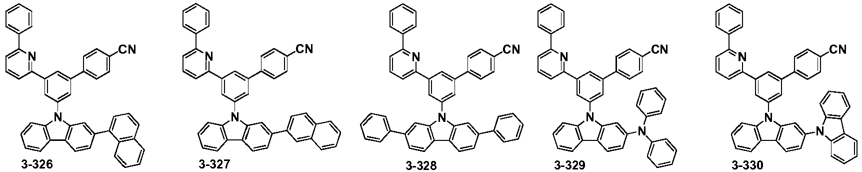

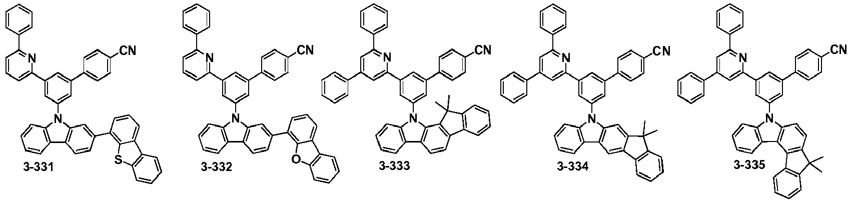

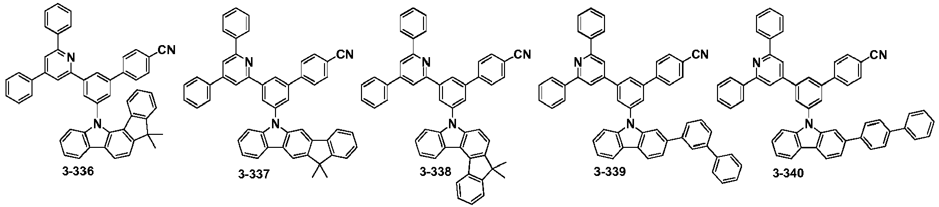

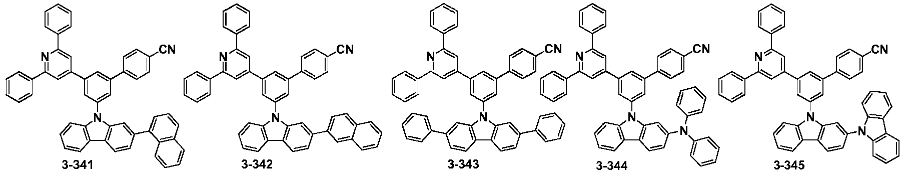

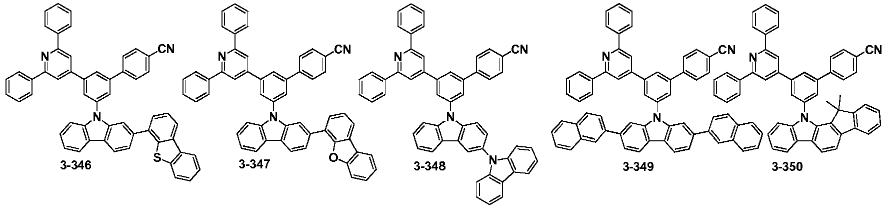

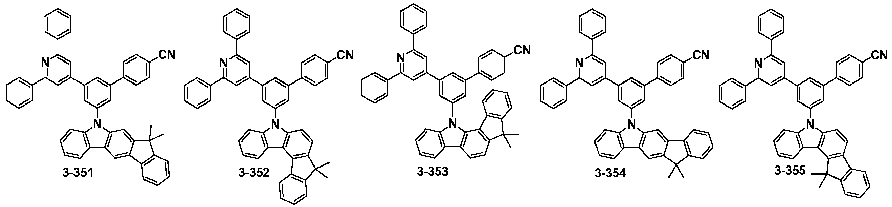

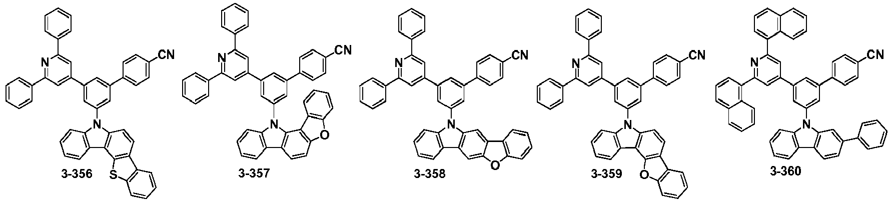

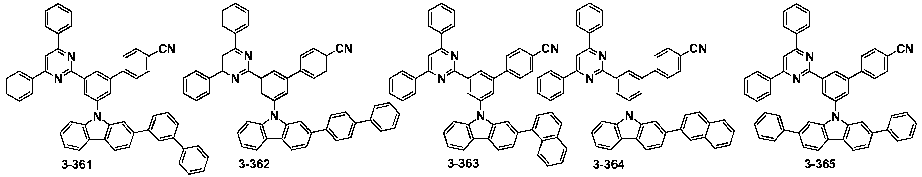

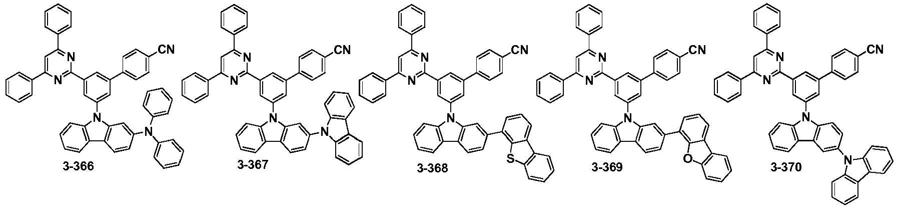

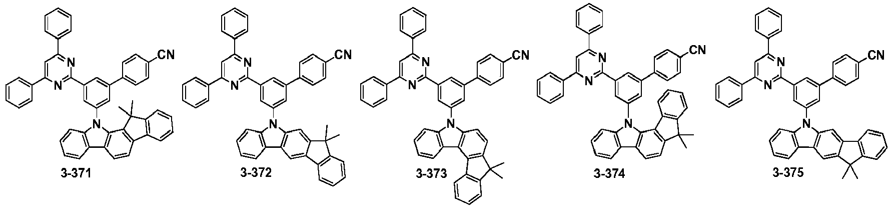

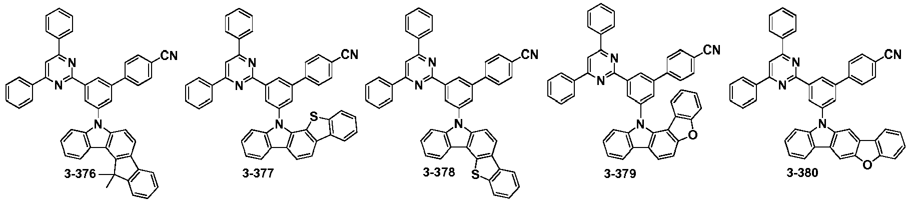

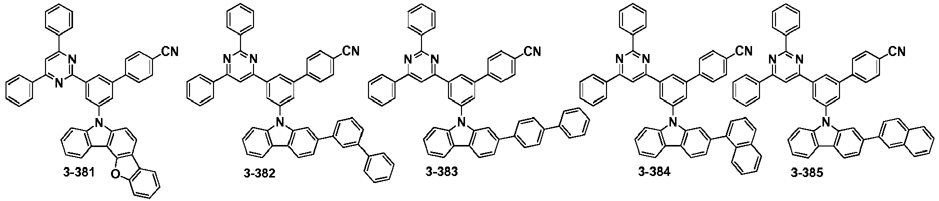

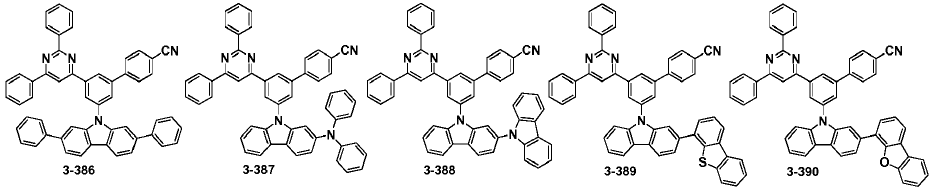

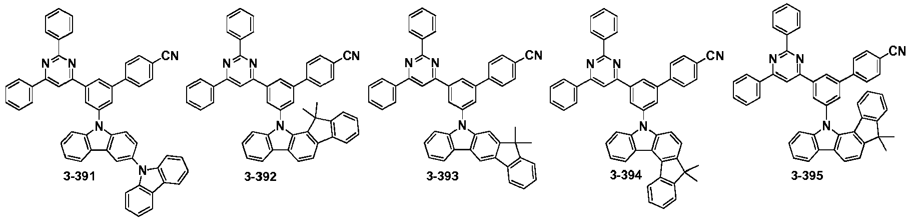

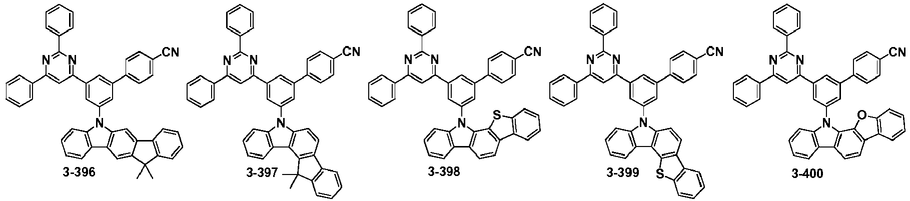

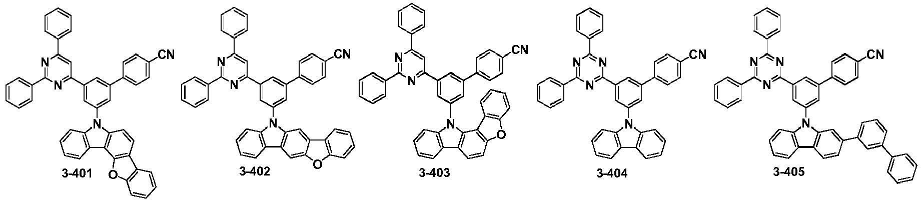

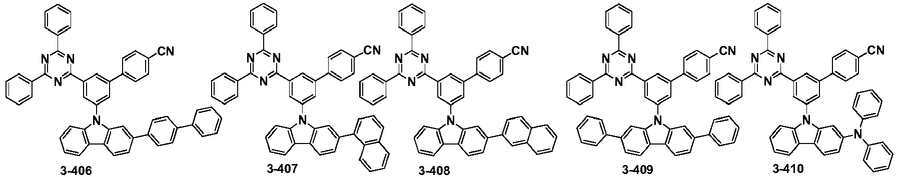

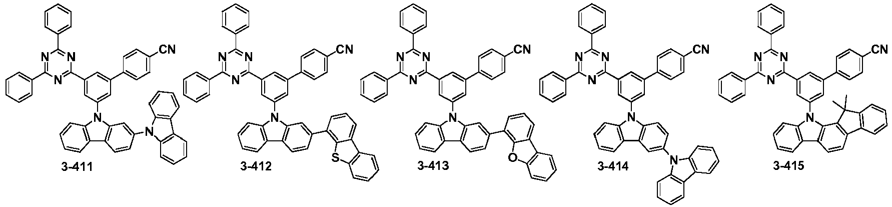

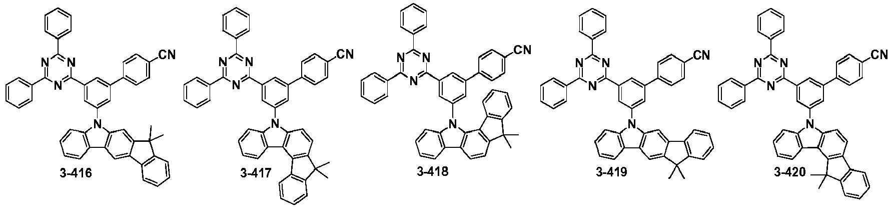

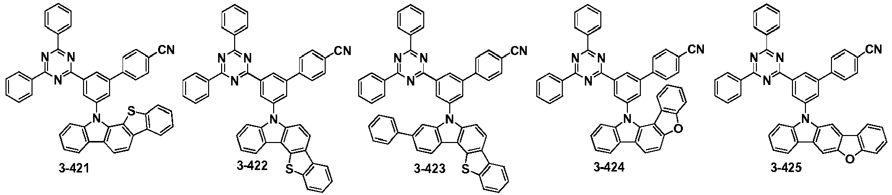

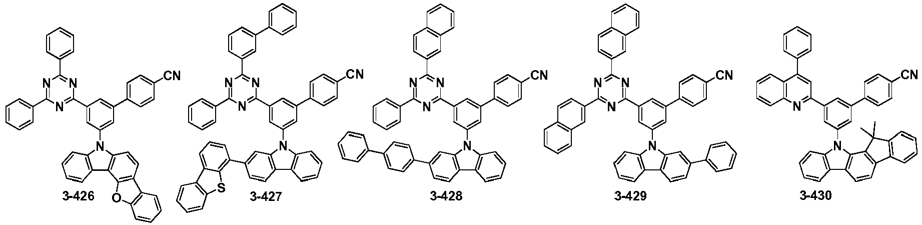

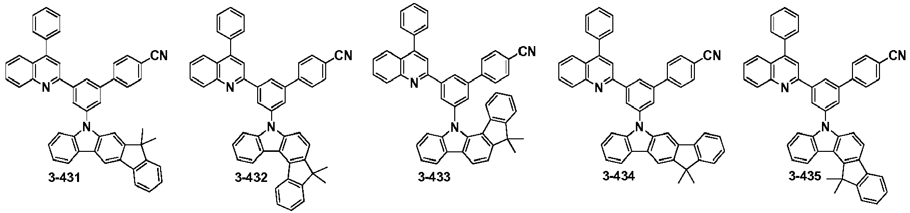

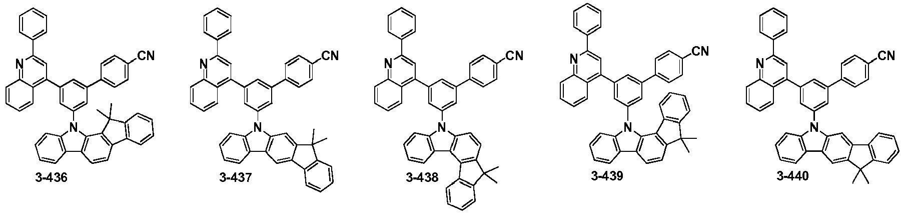

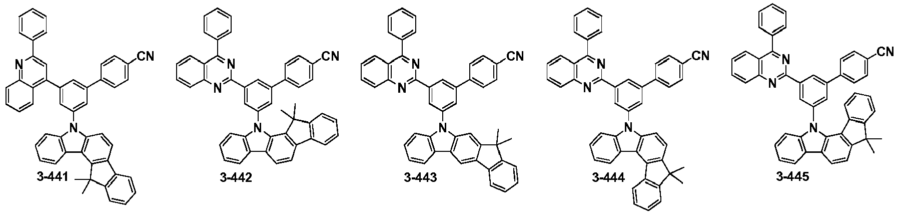

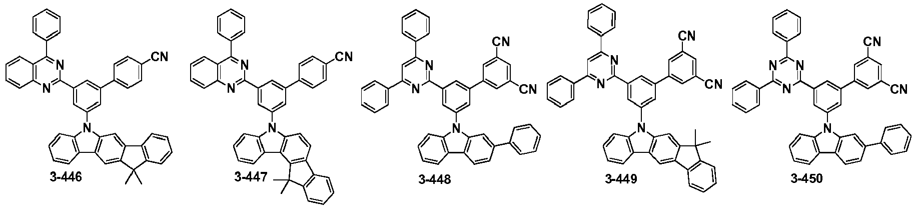

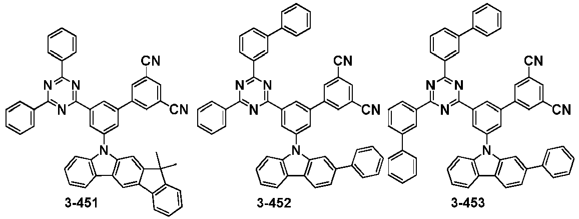

The organic light emitting device according to an exemplary embodiment of the present application may comprise as a host material for a light emitting layer: the hetero-cyclic compound represented by Chemical Formula 1; and the compound represented by Chemical Formula 2 or 3. In this case, a dopant material for the light emitting layer may use a material known in the art.

The weight ratio of the hetero-cyclic compound represented by Chemical Formula 1:the compound represented by Chemical Formula 2 or 3 in the host material may be 1:10 to 10:1, 1:8 to 8:1, 1:5 to 5:1, 1:2 to 2:1, and 1:1, but is not limited thereto.

The host material is in a form in which two or more compounds are simply mixed, and materials in a powder state may be mixed before an organic material layer of an organic light emitting device is formed, and compounds in a liquid state may be mixed at a temperature equal to or greater than a suitable temperature. The host material is in a solid state at a temperature which is equal to or less than the melting point of each material, and may be maintained as a liquid if the temperature is adjusted.

The organic light emitting device according to an exemplary embodiment of the present application may be manufactured by typical methods and materials for manufacturing an organic light emitting device, except that an organic material layer having one or more layers is formed by using the hetero-cyclic compound represented by Chemical Formula 1; and the compound represented by Chemical Formula 2 or 3.

The hetero-cyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The compound represented by Chemical Formula 1 may be used as a material for an electron transporting layer, a hole blocking layer, or a light emitting layer, and the like in an organic light emitting device. As an example, the compound represented by Chemical Formula 1 may be used as a material for an electron transporting layer, a hole transporting layer, or a light emitting layer of an organic light emitting device.

Furthermore, the compound represented by Chemical Formula 1 may be used as a material for a light emitting layer in an organic light emitting device. As an example, the compound represented by Chemical Formula 1 may be used as a material for a phosphorescent host of a light emitting layer in an organic light emitting device.

Further, an organic material layer comprising the compound represented by Chemical Formula 1 may additionally comprise other materials, if necessary.

The compound represented by Chemical Formula 1 may be used as a material for a charge producing layer in an organic light emitting device.

The compound represented by Chemical Formula 1 may be used as a material for an electron transporting layer, a hole blocking layer, or a light emitting layer, and the like in an organic light emitting device. As an example, the compound represented by Chemical Formula 1 may be used as a material for an electron transporting layer, a hole transporting layer, or a light emitting layer of an organic light emitting device.

Furthermore, the compound represented by Chemical Formula 1 may be used as a material for a light emitting layer in an organic light emitting device. As an example, the compound represented by Chemical Formula 1 may be used as a material for a phosphorescent host of a light emitting layer in an organic light emitting device.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

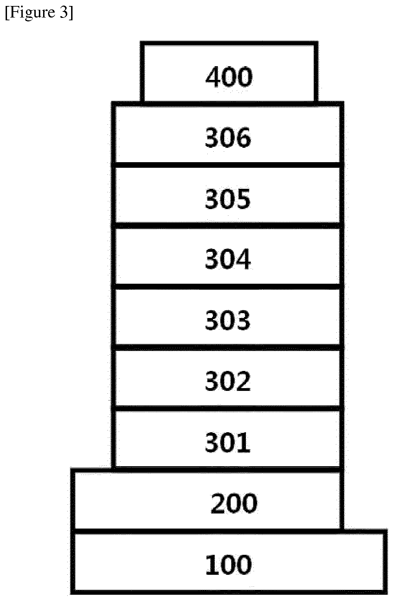

FIG. 3 exemplifies a case where an organic material layer is a multilayer. An organic light emitting device according to FIG. 3 comprises a hole injection layer 301, a hole transporting layer 302, a light emitting layer 303, a hole blocking layer 304, an electron transporting layer 305, and an electron injection layer 306. However, the scope of the present application is not limited by the stacking structure as described above, and if necessary, the other layers except for the light emitting layer may be omitted, and another necessary functional layer may be further added.

In the organic light emitting device according to an exemplary embodiment of the present application, materials other than the compounds of Chemical Formulae 1 to 5 will be exemplified below, but these materials are illustrative only, and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material comprise: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a material for the negative electrode, materials having a relatively low work function may be used, and a metal, a metal oxide, or a conductive polymer, and the like may be used. Specific examples of the negative electrode material comprise: a metal such as magnesium, calcium, sodium, potassium, titanium, indium, yttrium, lithium, gadolinium, aluminum, silver, tin, and lead, or an alloy thereof; a multi-layer structured material such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p.677 (1994)], for example, tris(4-carbazoyl-9-ylphenyl)amine (TCTA), 4,4',4"-tris[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

The organic light emitting device according to an exemplary embodiment of the present application may be a top emission type, a bottom emission type, or a dual emission type according to the material to be used.

The hetero-cyclic compound according to an exemplary embodiment of the present application may act even in organic electronic devices comprising organic solar cells, organic photoconductors, organic transistors, and the like, based on the principle similar to those applied to organic light emitting devices.

### [Mode for Invention]

Hereinafter, the present specification will be described in more detail through Examples, but these Examples are provided only for exemplifying the present application, and are not intended to limit the scope of the present application.

### <Examples>

### [Preparation Example 1-1] Preparation of Compound 1-1

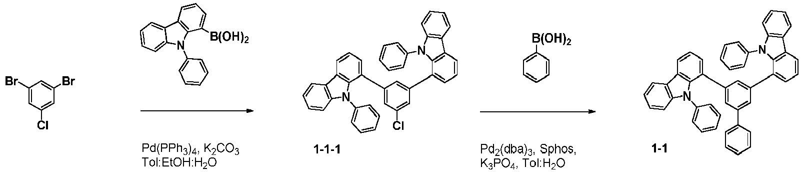

### Preparation of Compound 1-1-1

20 g (73.98 mmol) of 1,3-dibromo-5-chlorobenzene, 46.7 g (162.8 mmol) of (9-phenyl-9H-carbazol-1-yl)boronic acid, 4.27 g (3.7 mmol) of Pd(PPh₃)₄, and 30.67 g (221.94 mmol) of K₂CO₃ were dissolved in 350/70/70 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 37.42 g (85%) of Target Compound 1-1-1.

### Preparation of Compound 1-1

5 g (8.40 mmol) of Compound 1-1-1, 1.23 g (10.08 mmol) of phenylboronic acid, 0.77 g (0.84 mmol) of Pd₂(dba)₃, 0.69 g (1.68 mmol) of SPhos, and 5.35 g (25.2 mmol) of K₃PO₄ were dissolved in 40/4 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 3.5 g (66%) of Target Compound 1-1.

### [Preparation Example 1-2] Preparation of Compound 1-13

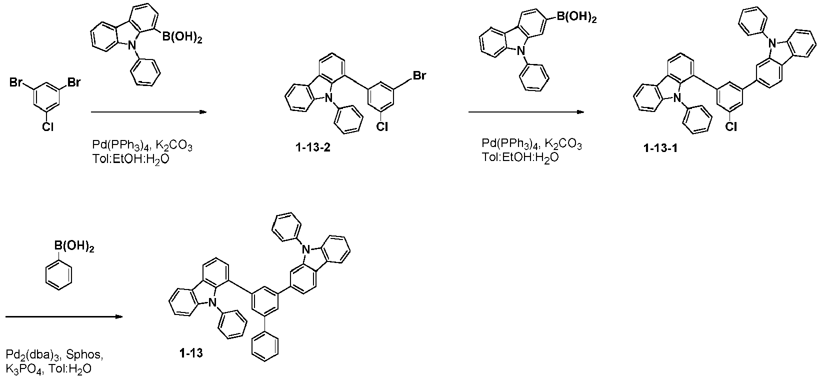

### Preparation of Compound 1-13-2

15 g (55.48 mmol) of 1,3-dibromo-5-chlorobenzene, 15.9 g (55.48 mmol) of (9-phenyl-9H-carbazol-1-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 14.9 g (62%) of Target Compound 1-13-2.

### Preparation of Compound 1-13-1

24 g (55.48 mmol) of Compound 1-13-2, 24.2 g (66.58 mmol) of (9-phenyl-9H-carbazol-2-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-13-1.

### Preparation of Compound 1-13

6.2 g (9.28 mmol) of Compound 1-13-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.5 g (68%) of Target Compound 1-13.

### [Preparation Example 1-3] Preparation of Compound 1-21

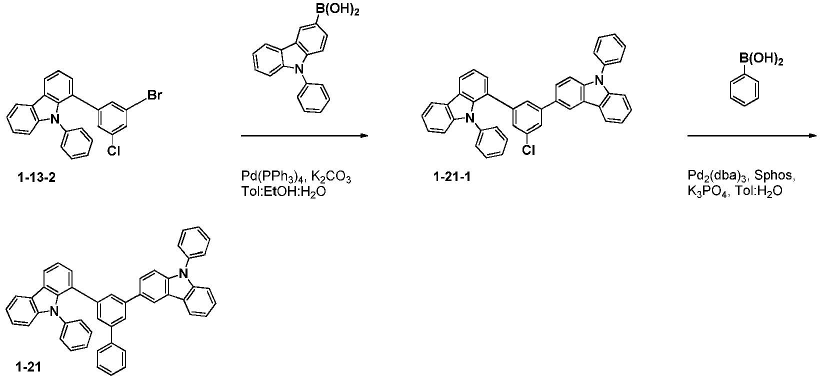

### Preparation of Compound 1-21-1

24 g (55.48 mmol) of Compound 1-13-2, 24.2 g (66.58 mmol) of (9-phenyl-9H-carbazol-3-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-21-1.

### Preparation of Compound 1-21

6.2 g (9.28 mmol) of Compound 1-21-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.5 g (68%) of Target Compound 1-21.

### [Preparation Example 1-4] Preparation of Compound 1-31

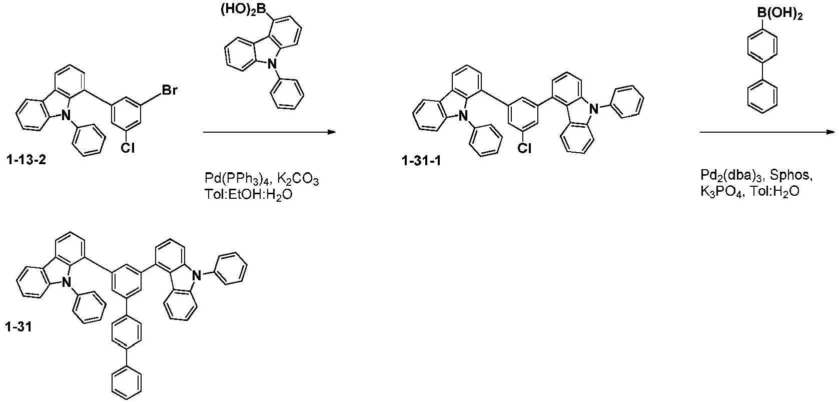

### Preparation of Compound 1-31-1

24 g (55.48 mmol) of Compound 1-13-2, 24.2 g (66.58 mmol) of (9-phenyl-9H-carbazol-4-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-31-1.

### Preparation of Compound 1-31

6.2 g (9.28 mmol) of Compound 1-31-1, 2.2 g (11.14 mmol) of [1,1'-biphenyl]-4-ylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.5 g (68%) of Target Compound 1-31.

### [Preparation Example 1-5] Preparation of Compound 1-41

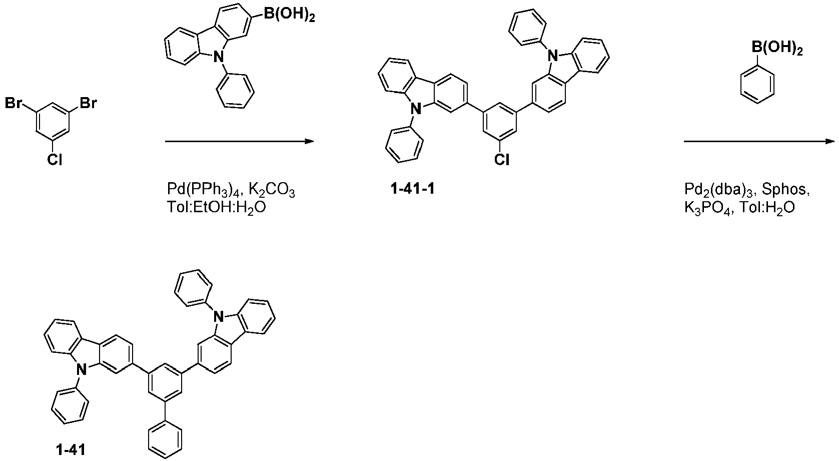

### Preparation of Compound 1-41-1

20 g (73.98 mmol) of 1,3-dibromo-5-chlorobenzene, 46.7 g (162.8 mmol) of (9-phenyl-9H-carbazol-2-yl)boronic acid, 4.27 g (3.7 mmol) of Pd(PPh₃)₄, and 30.67 g (221.94 mmol) of K₂CO₃ were dissolved in 350/70/70 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 37.42 g (85%) of Target Compound 1-41-1.

### Preparation of Compound 1-41

5 g (8.40 mmol) of Compound 1-41-1, 1.23 g (10.08 mmol) of phenylboronic acid, 0.77 g (0.84 mmol) of Pd₂(dba)₃, 0.69 g (1.68 mmol) of SPhos, and 5.35 g (25.2 mmol) of K₃PO₄ were dissolved in 40/4 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 3.5 g (66%) of Target Compound 1-41.

### [Preparation Example 1-6] Preparation of Compound 1-43

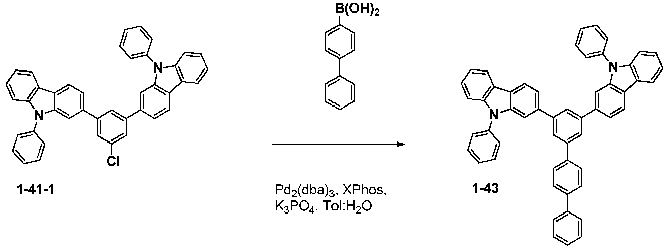

6 g (10.08 mmol) of Compound 1-41-1, 2.99 g (15.12 mmol) of [1,1'-biphenyl]-4-ylboronic acid, 0.93 g (1.0 mmol) of Pd₂(dba)₃, 0.96 g (2.0 mmol) of XPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.3 g (60%) of Target Compound 1-43.

### [Preparation Example 1-7] Preparation of Compound 1-44

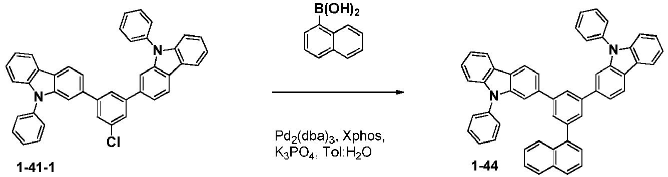

6 g (10.08 mmol) of Compound 1-41-1, 2.6 g (15.12 mmol) of naphthalen-1-ylboronic acid, 0.93 g (1.0 mmol) of Pd₂(dba)₃, 0.96 g (2.0 mmol) of XPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.3 g (62%) of Target Compound 1-44.

### [Preparation Example 1-8] Preparation of Compound 1-45

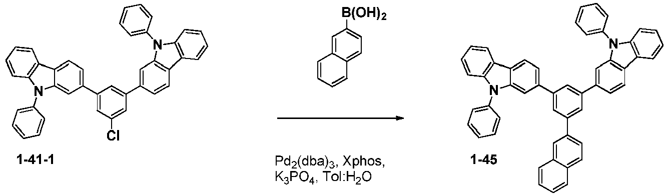

6 g (10.08 mmol) of Compound 1-41-1, 2.6 g (15.12 mmol) of naphthalen-2-ylboronic acid, 0.93 g (1.0 mmol) of Pd₂(dba)₃, 0.96 g (2.0 mmol) of XPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.2 g (61%) of Target Compound 1-45.

### [Preparation Example 1-9] Preparation of Compound 1-48

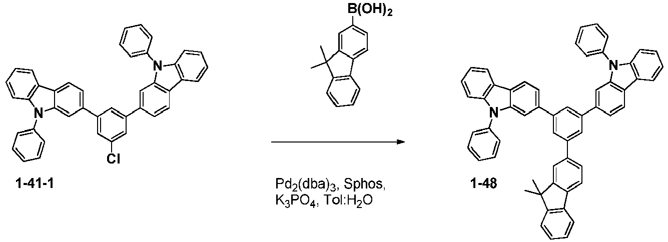

6 g (10.08 mmol) of Compound 1-41-1, 3.6 g (15.12 mmol) of (9,9-dimethyl-9H-fluoren-2-yl)boronic acid, 0.93 g (1.0 mmol) of Pd₂(dba)₃, 0.82 g (2.0 mmol) of SPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.0 g (53%) of Target Compound 1-48.

### [Preparation Example 1-10] Preparation of Compound 1-49

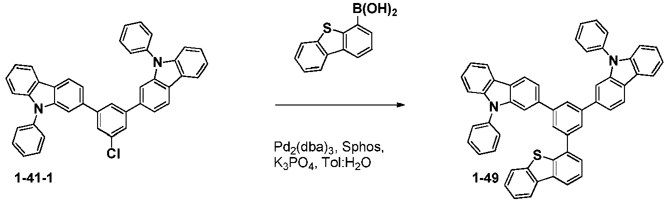

6 g (10.08 mmol) of Compound 1-41, 3.5 g (15.12 mmol) of dibenzo[*b,d*]thiophen-4-ylboronic acid, 0.93 g (1.0 mmol) of Pd₂(dba)₃, 0.82 g (2.0 mmol) of SPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.4 g (58%) of Target Compound 1-49.

### [Preparation Example 1-11] Preparation of Compound 1-50

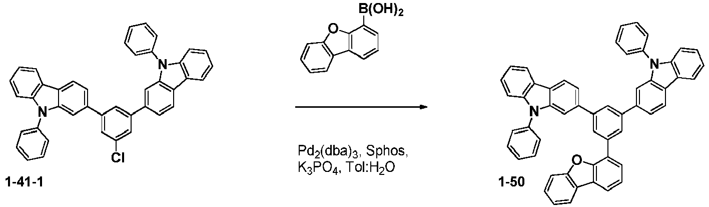

5.5 g (9.24 mmol) of Compound 1-41-1, 2.35 g (11.09 mmol) of dibenzo[*b,d*]furan-4-ylboronic acid, 0.84 g (0.92 mmol) of Pd₂(dba)₃, 0.90 g (1.85 mmol) of SPhos, and 5.88 g (27.72 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.3 g (64%) of Target Compound 1-50.

### [Preparation Example 1-12] Preparation of Compound 1-54

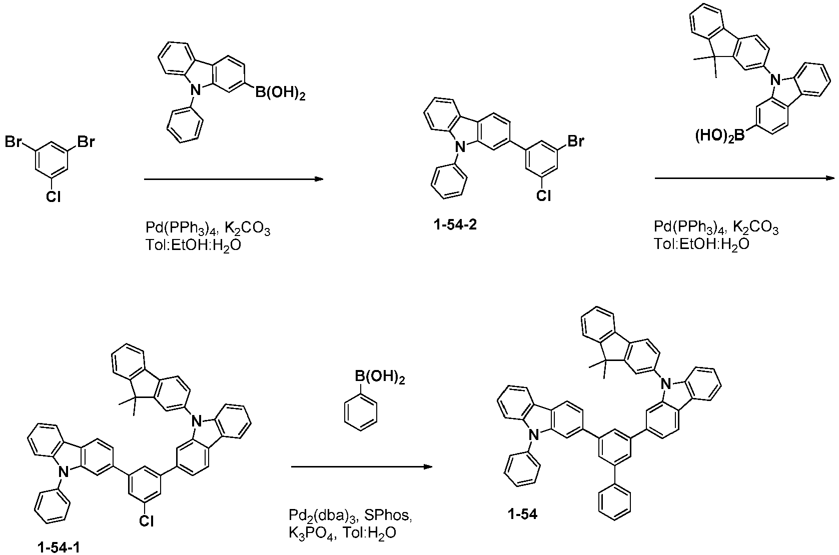

### Preparation of Compound 1-54-2

20 g (73.98 mmol) of 1,3-dibromo-5-chlorobenzene, 21.2 g (73.98 mmol) of (9-phenyl-9H-carbazol-2-yl)boronic acid, 4.27 g (3.7 mmol) of Pd(PPh₃)₄, and 30.67 g (221.94 mmol) of K₂CO₃ were dissolved in 350/70/70 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 19.2 g (60%) of Target Compound 1-54-2.

### Preparation of Compound 1-54-1

24 g (55.48 mmol) of Compound 1-54-2, 26.9 g (66.58 mmol) of (9-(9,9-dimethyl-9H-fluoren-2-yl-9H-carbazol-2-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.8 g (78%) of Target Compound 1-54-1.

### Preparation of Compound 1-54

5.97 g (8.40 mmol) of Compound 1-54-1, 1.23 g (10.08 mmol) of phenylboronic acid, 0.77 g (0.84 mmol) of Pd₂(dba)₃, 0.69 g (1.68 mmol) of SPhos, and 5.35 g (25.2 mmol) of K₃PO₄ were dissolved in 40/4 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.7 g (74%) of Target Compound 1-54.

### [Preparation Example 1-13] Preparation of Compound 1-60

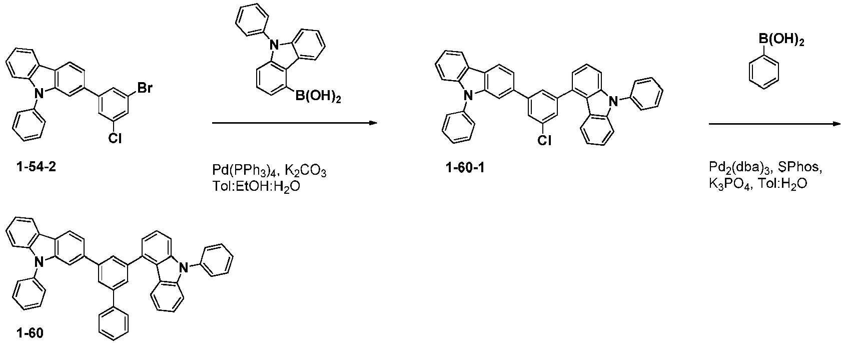

### Preparation of Compound 1-60-1

24 g (55.48 mmol) of Compound 1-54-2, 19.1 g (66.58 mmol) of (9-phenyl-9H-carbazol-4-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 23.1 g (70%) of Target Compound 1-60-1.

### Preparation of Compound 1-60

5.0 g (8.40 mmol) of Compound 1-60-1, 1.23 g (10.08 mmol) of phenylboronic acid, 0.77 g (0.84 mmol) of Pd₂(dba)₃, 0.69 g (1.68 mmol) of SPhos, and 5.35 g (25.2 mmol) of K₃PO₄ were dissolved in 40/4 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 3.5 g (65%) of Target Compound 1-60.

### [Preparation Example 1-14] Preparation of Compound 1-70

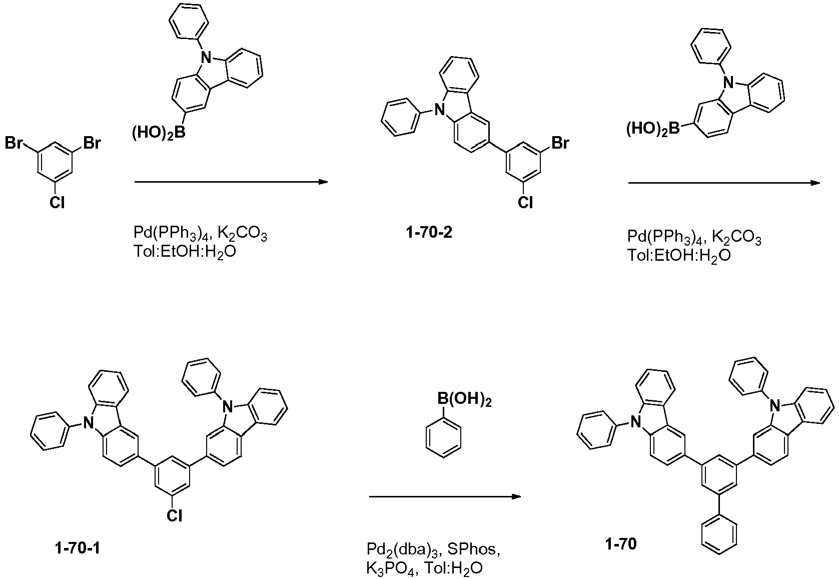

### Preparation of Compound 1-70-2

15 g (55.48 mmol) of 1,3-dibromo-5-chlorobenzene, 15.9 g (55.48 mmol) of (9-phenyl-9H-carbazol-3-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 14.9 g (62%) of Target Compound 1-70-2.

### Preparation of Compound 1-70-1

24 g (55.48 mmol) of Compound 1-70-2, 24.2 g (66.58 mmol) of (9-1,1-biphenyl-9H-carbazol-3-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-70-1.

### Preparation of Compound 1-70

6.2 g (9.28 mmol) of Compound 1-70-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.5 g (68%) of Target Compound 1-70.

### [Preparation Example 1-15] Preparation of Compound 1-82

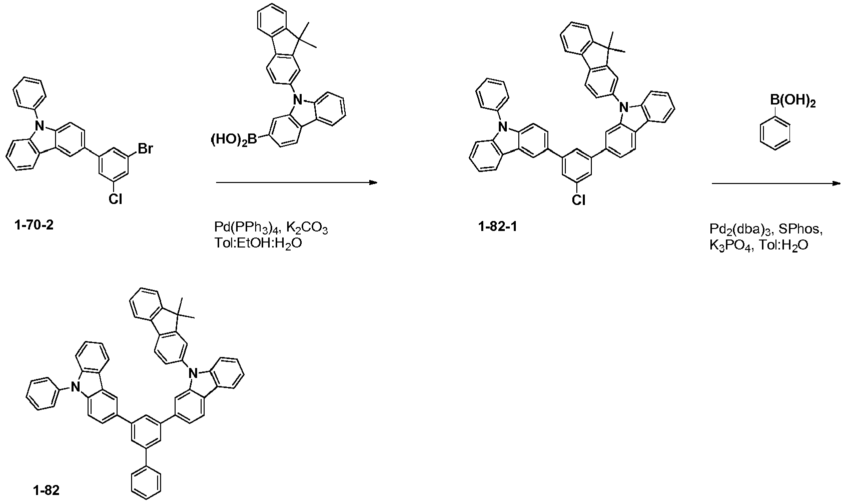

### Preparation of Compound 1-82-1

24 g (55.48 mmol) of Compound 1-70-2, 26.9 g (66.58 mmol) of (9-(9,9-dimethyl-9H-fluoren-2-yl-9H-carbazol-2-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.8 g (78%) of Target Compound 1-82-1.

### Preparation of Compound 1-82

6.2 g (9.28 mmol) of Compound 1-82-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.95 g (75%) of Target Compound 1-82.

### [Preparation Example 1-16] Preparation of Compound 1-90

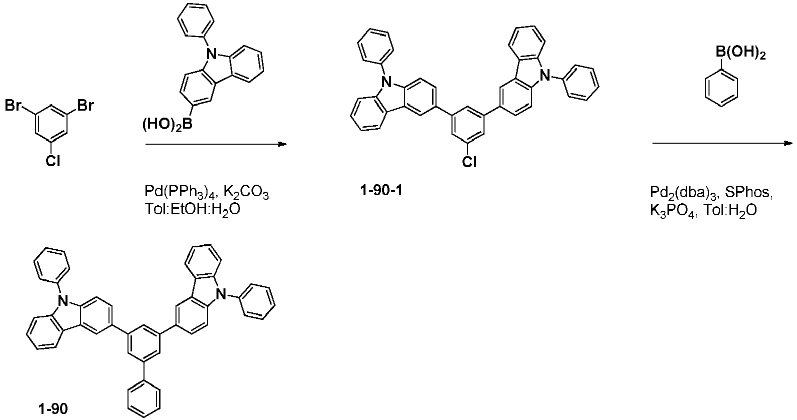

### Preparation of Compound 1-90-1

15 g (55.48 mmol) of 1,3-dibromo-5-chlorobenzene, 35 g (122 mmol) of (9-phenyl-9H-carbazol-3-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 27.07 g (82%) of Target Compound 1-90-1.

### Preparation of Compound 1-90

5.8 g (9.28 mmol) of Compound 1-1-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.2 g (68%) of Target Compound 1-90.

### [Preparation Example 1-17] Preparation of Compound 1-97

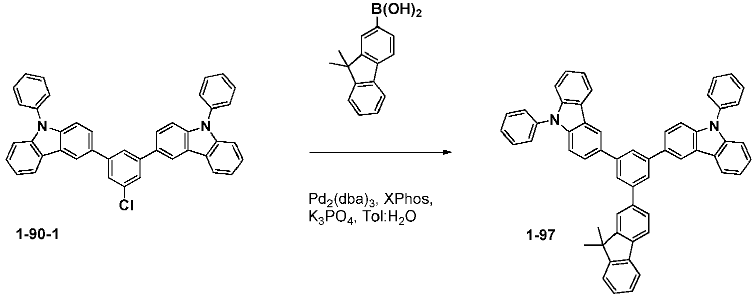

6 g (10.08 mmol) of Compound 1-90-1, 3.6 g (15.12 mmol) of (9,9-dimethyl-9H-fluoren-2-yl)boronic acid, 0.93 g (1.0 mmol) of Pd₂(dba)₃, 0.96 g (2.0 mmol) of XPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 7.4 g (90%) of Target Compound 1-97.

### [Preparation Example 1-18] Preparation of Compound 1-100

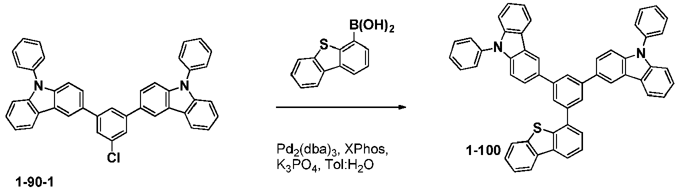

6 g (10.08 mmol) of Compound 1-90-1, 3.5 g (15.12 mmol) of dibenzo[*b,d*]thiophen-4-ylboronic acid, 0.93 g (1.0 mmol) of Pd₂(dba)₃, 1.66 g (4.0 mmol) of XPhos, and 6.42 g (30.2 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 6.7 g (90%) of Target Compound 1-100.

### [Preparation Example 1-19] Preparation of Compound 1-101

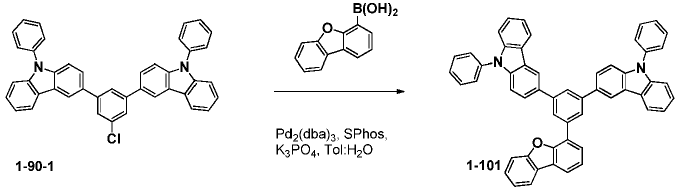

8 g (13.44 mmol) of Compound 1-90-1, 4.28 g (20.16 mmol) of dibenzo[*b,d*]furan-4-ylboronic acid, 1.24 g (1.3 mmol) of Pd₂(dba)₃, 2.2 g (5.4 mmol) of SPhos, and 8.56 g (40.32 mmol) of K₃PO₄ were dissolved in 60/6 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 8.4 g (90%) of Target Compound 1-101.

### [Preparation Example 1-20] Preparation of Compound 1-103

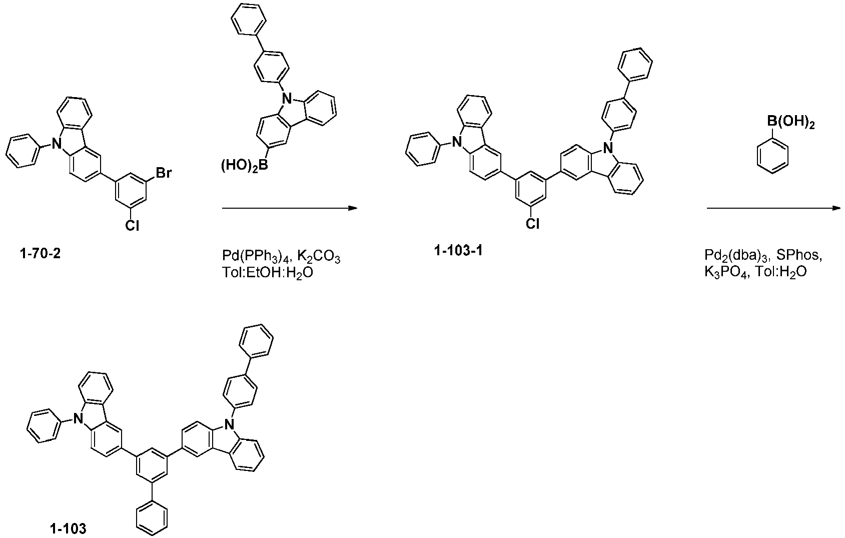

### Preparation of Compound 1-103-1

24 g (55.48 mmol) of Compound 1-70-2, 24.2 g (66.58 mmol) of (9-1,1-biphenyl-9H-carbazol-3-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-103-1.

### Preparation of Compound 1-103

6.2 g (9.28 mmol) of Compound 1-103-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 4.5 g (68%) of Target Compound 1-103.

### [Preparation Example 1-21] Preparation of Compound 1-110

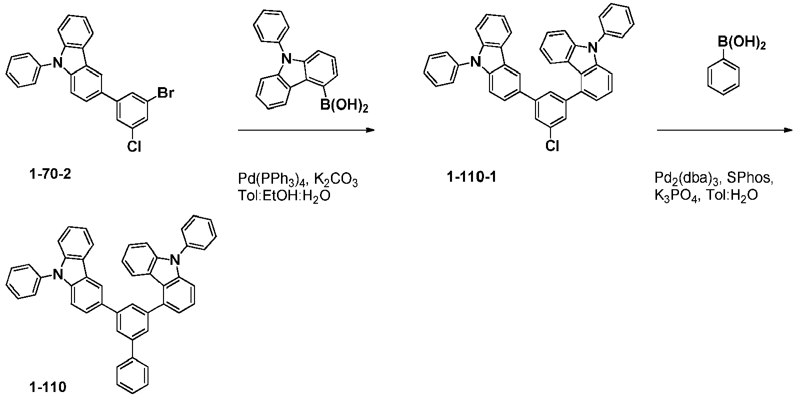

### Preparation of Compound 1-110-1

24 g (55.48 mmol) of Compound 1-70-2, 19.1 g (66.58 mmol) of (9-biphenyl-9H-carbazol-4-yl)boronic acid, 3.2 g (2.77 mmol) of Pd(PPh₃)₄, and 23.01 g (166.44 mmol) of K₂CO₃ were dissolved in 250/50/50 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 30.5 g (82%) of Target Compound 1-110-1.

### Preparation of Compound 1-110

6.2 g (9.28 mmol) of Compound 1-110-1, 1.36 g (11.14 mmol) of phenylboronic acid, 0.43 g (0.46 mmol) of Pd₂(dba)₃, 0.76 g (1.86 mmol) of SPhos, and 5.9 g (27.9 mmol) of K₃PO₄ were dissolved in 50/5 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 3.54 g (60%) of Target Compound 1-110.

### [Preparation Example 1-22] Preparation of Compound 1-120

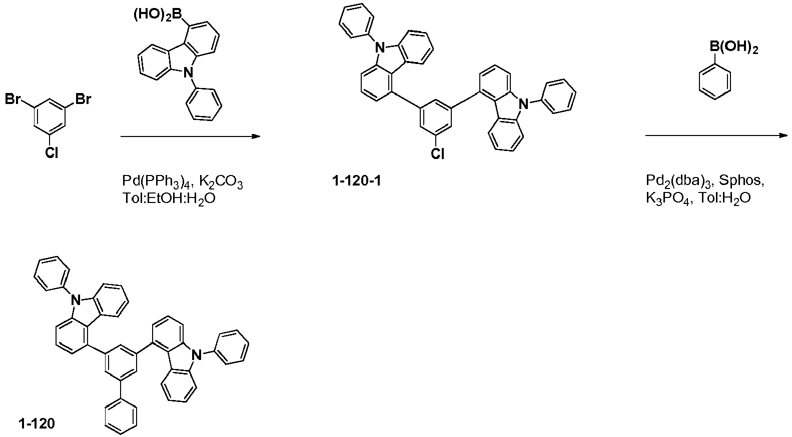

### Preparation of Compound 1-120-1

20 g (73.98 mmol) of 1,3-dibromo-5-chlorobenzene, 46.7 g (162.8mmol) of (9-phenyl-9H-carbazol-4-yl)boronic acid, 4.27 g (3.7 mmol) of Pd(PPh₃)₄, and 30.67 g (221.94 mmol) of K₂CO₃ were dissolved in 350/70/70 mL of toluene/EtOH/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 37.42 g (85%) of Target Compound 1-120-1.

### Preparation of Compound 1-120

5 g (8.40 mmol) of Compound 1-120-1, 1.23 g (10.08 mmol) of phenylboronic acid, 0.77 g (0.84 mmol) of Pd₂(dba)₃, 0.69 g (1.68 mmol) of SPhos, and 5.35 g (25.2 mmol) of K₃PO₄ were dissolved in 40/4 mL of toluene/H₂O, and then the resulting solution was refluxed for 12 hours. The resulting product was cooled to room temperature, and then an extraction was performed using distilled water and dichloromethane. Thereafter, the extract was dissolved in dichloromethane, the resulting solution was filtered using silica gel, celite, and florisil, the solvent was removed, and column chromatography was used to obtain 3.5 g (66%) of Target Compound 1-120.

### [Preparation Example 2-1] Preparation of Compound 2-11

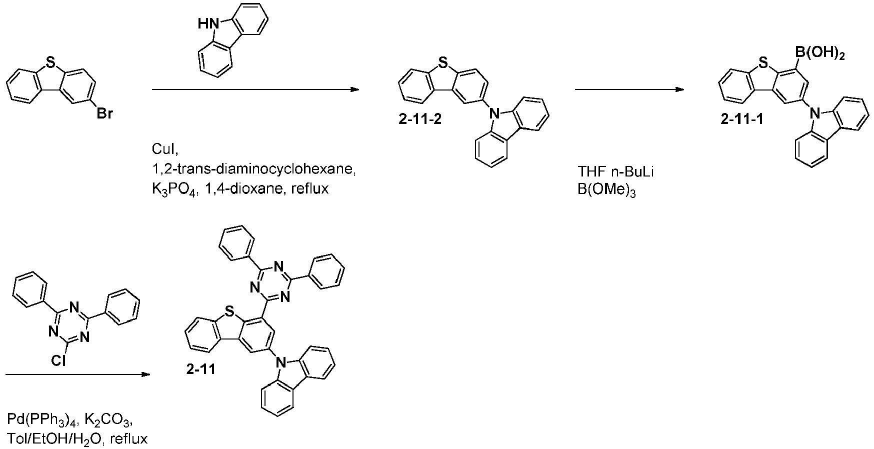

### Preparation of Compound 2-11-2

5.0 g (19.0 mmol) of 2-bromodibenzo[b,d]thiophene, 2.6 g (15.8 mmol) of 9H-carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 4.7 g (85%) of Target Compound 2-11-2.

### Preparation of Compound 2-11-1

7.4 mL (18.6 mmol) of 2.5 M n-BuLi was added dropwise to a mixed solution containing 5 g (14.3 mmol) of Compound 2-11-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 3.9 g (70%) of Target Compound 2-11-1.

### Preparation of Compound 2-11

7.5 g (19.0 mmol) of Compound 2-11-1, 5.1 g (19.0 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 7.7 g (70%) of Target Compound 2-11.

### [Preparation Example 2-2] Preparation of Compound 2-12

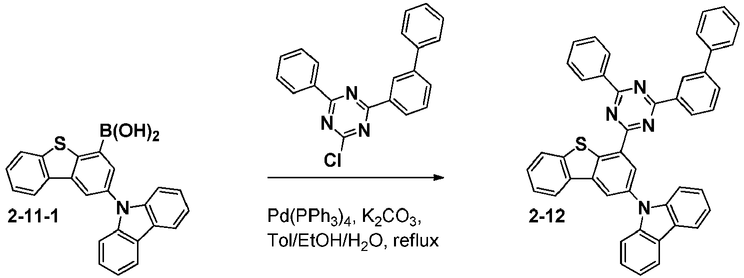

7.5 g (19.0 mmol) of Compound 4-11-1, 6.5 g (19.0 mmol) of 2-([1,1'-biphenyl]-3-yl)-4-chloro-6-phenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.7 g (70%) of Target Compound 2-12.

### [Preparation Example 2-3] Preparation of Compound 2-28

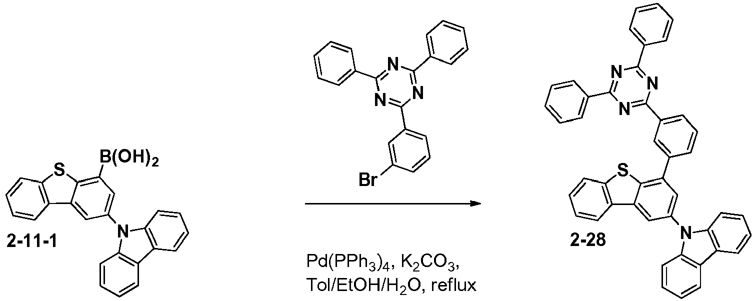

7.5 g (19.0 mmol) of Compound 2-11-1, 7.4 g (19.0 mmol) of 2-(3-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.7 g (70%) of Target Compound 2-28.

### [Preparation Example 2-4] Preparation of Compound 2-36

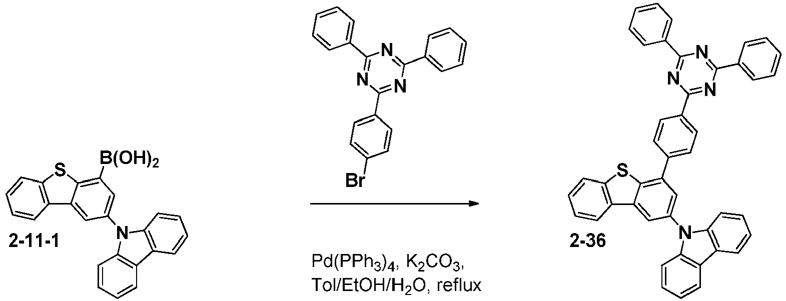

7.5 g (19.0 mmol) of Compound 2-11-1, 7.4 g (19.0 mmol) of 2-(4-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.7 g (70%) of Target Compound 2-36.

### [Preparation Example 2-5] Preparation of Compound 2-39

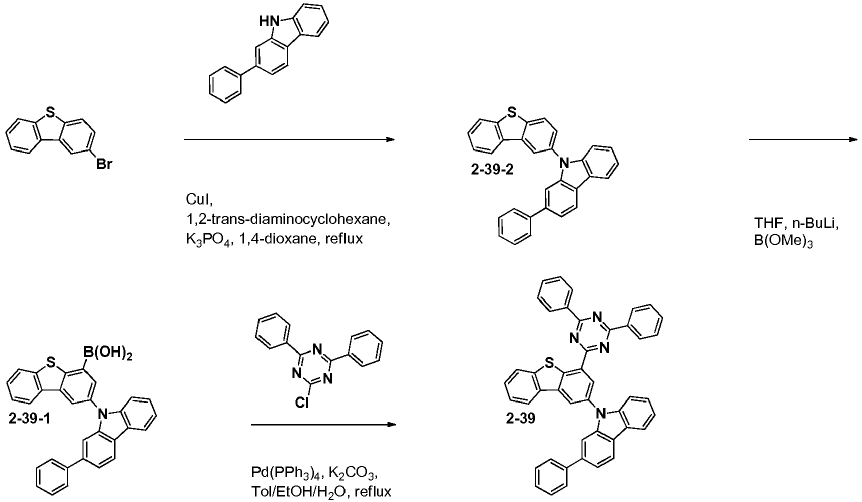

### Preparation of Compound 2-39-2

5.0 g (19.0 mmol) of 2-bromodibenzo[b,d]thiophene, 3.8 g (15.8 mmol) of 2-phenyl-9H-carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 5.7 g (85%) of Target Compound 2-39-2.

### Preparation of Compound 2-39-1

7.4 mL (18.6 mmol) of 2.5 M n-BuLi was added dropwise to a mixed solution containing 6.1 g (14.3 mmol) of Compound 2-39-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 4.7 g (70%) of Target Compound 2-39-1.

### Preparation of Compound 2-39

8.9 g (19.0 mmol) of Compound 2-39-1, 5.1 g (19.0 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.7 g (70%) of Target Compound 2-39.

### [Preparation Example 2-6] Preparation of Compound 2-40

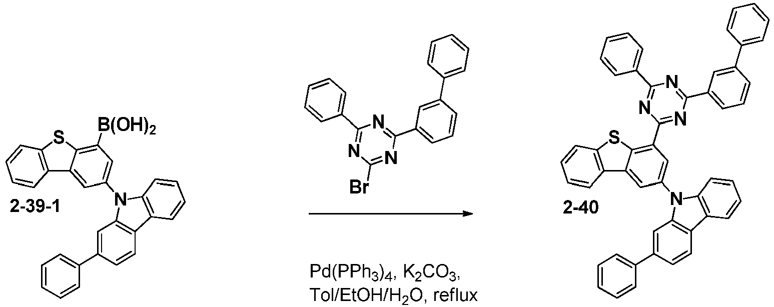

8.9 g (19.0 mmol) of Compound 2-39-1, 7.4 g (19.0 mmol) of 2-([1,1'-biphenyl]-3-yl)-4-bromo-6-phenyl-1,3,5-triazine, 1.1 g (0.95 mmol) of Pd(PPh₃)₄, and 5.2 g (38.0 mM) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.7 g (70%) of Target Compound 2-40.

### [Preparation Example 2-7] Preparation of Compound 2-41

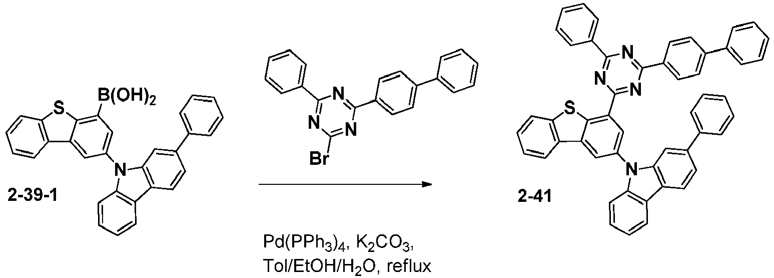

8.9 g (19.0 mmol) of Compound 2-39-1, 7.4 g (19.0 mmol) of 2-([1,1'-biphenyl]-4-yl)-4-bromo-6-phenyl-1,3,5-triazine, 1.1 g (0.95 mM) of Pd(PPh₃)₄, and 5.2 g (38.0 mM) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9 g (65%) of Target Compound 2-41.

### [Preparation Example 2-8] Preparation of Compound 2-42

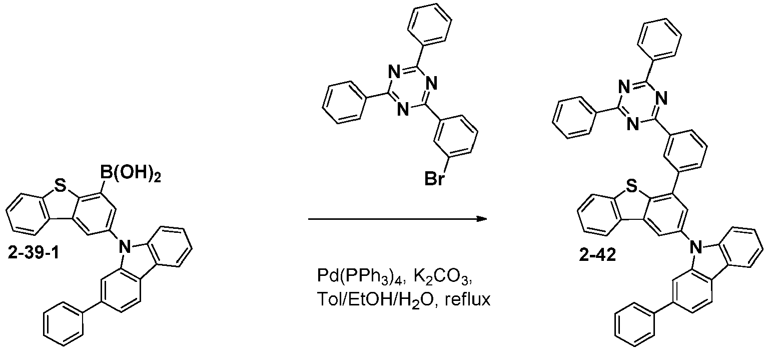

8.9 g (19.0 mmol) of Compound 2-39-1, 7.4 g (19.0 mmol) of 2-(3-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.7 g (70%) of Target Compound 2-42.

### [Preparation Example 2-9] Preparation of Compound 2-43

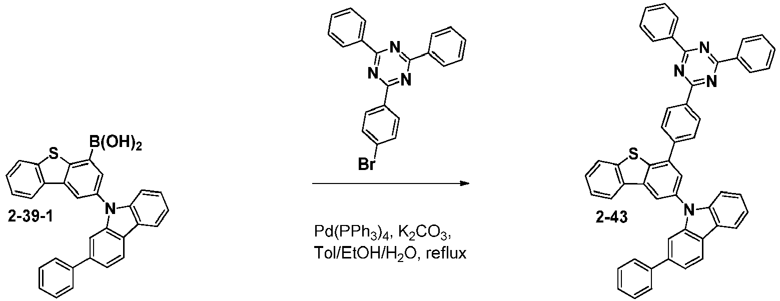

8.9 g (19.0 mmol) of Compound 2-39-1, 7.4 g (19.0 mmol) of 2-(4-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.7 g (70%) of Target Compound 2-43.

### [Preparation Example 2-10] Preparation of Compound 2-47

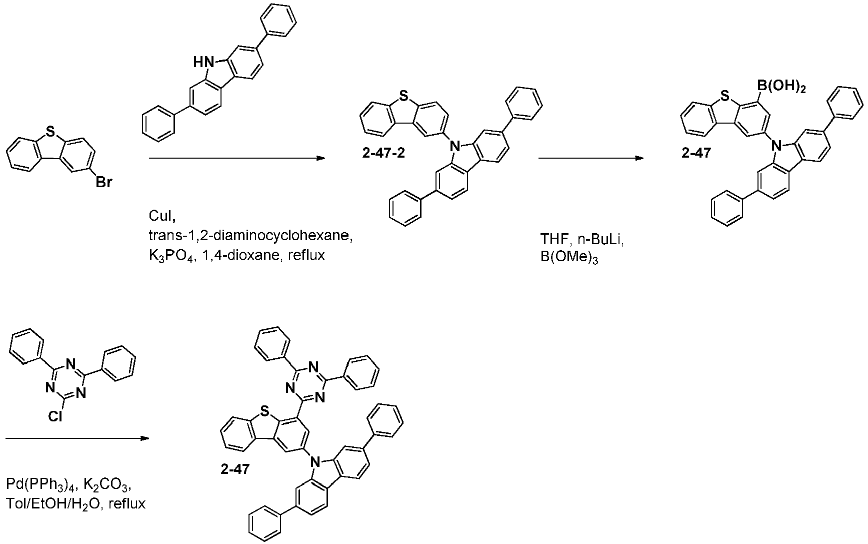

### Preparation of Compound 2-47-2

5.0 g (19.0 mmol) of 2-bromodibenzo[b,d]thiophene, 5.0 g (15.8 mmol) of 2,7-diphenyl-9H-carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 6.7 g (85%) of Target Compound 2-47-2.

### Preparation of Compound 2-47-1

7.4 mL (18.6 mmol) of 2.5 M n-BuLi was added dropwise to a mixed solution containing 7.2 g (14.3 mmol) of Compound 2-47-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain (60%) of Target Compound 2-47-1.

### Preparation of Compound 2-47

10.4 g (19.0 mmol) of Compound 2-47-1, 5.1 g (19.0 mM) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.7 g (70%) of Target Compound 2-47.

### [Preparation Example 2-11] Preparation of Compound 2-66

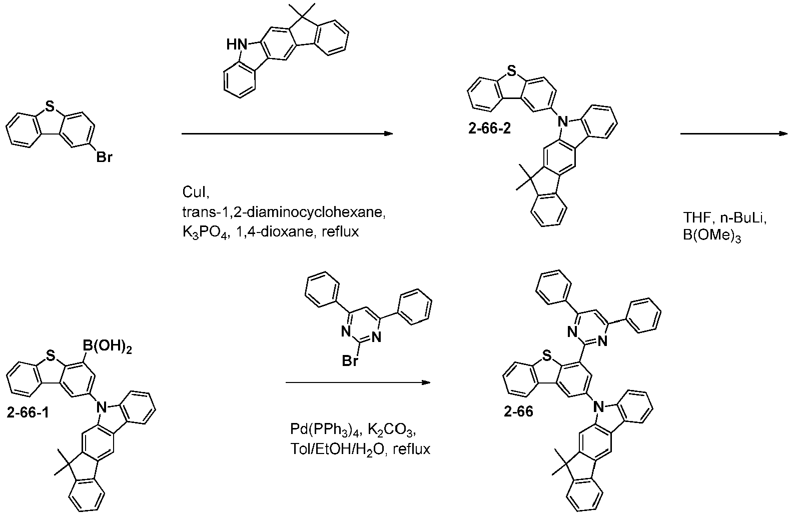

### Preparation of Compound 2-66-2

5.0 g (19.0 mmol) of 2-bromodibenzo[b,d]thiophene, 4.5 g (15.8 mmol) of 7.7-dimethyl-5,7-dihydroindeno[2,1-b]carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 7.3 g (85%) of Target Compound 2-66-2.

### Preparation of Compound 2-66-1

7.4 mL (18.6 mmol) of 2.5 M n-BuLi was added dropwise to a mixed solution containing 6.7 g (14.3 mmol) of Compound 2-66-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 5.1 g (70%) of Target Compound 2-66-1.

### Preparation of Compound 2-66

9.7 g (19.0 mmol) of Compound 2-66-1, 5.9 g (19.0 mmol) of 2-bromo-4,6-diphenylpyrimidine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.3 g (70%) of Target Compound 2-66.

### [Preparation Example 2-12] Preparation of Compound 2-68

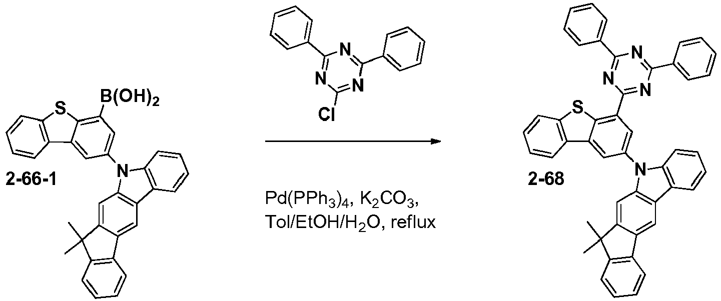

9.7 g (19.0 mmol) of Compound 2-66-1, 5.1 g (19.0 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.3 g (70%) of Target Compound 2-68.

### [Preparation Example 2-13] Preparation of Compound 2-71

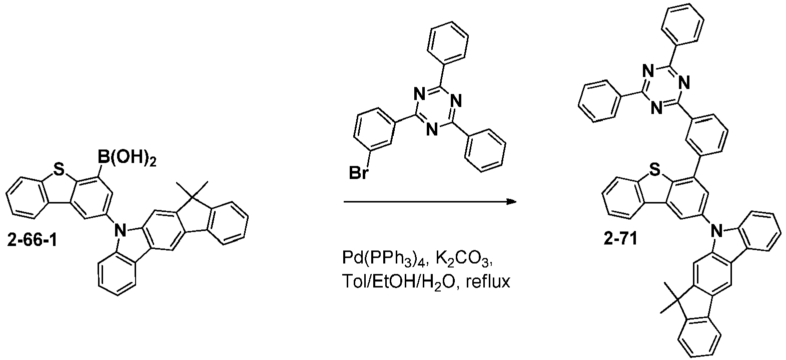

9.7 g (19.0 mmol) of Compound 2-66-1, 7.4 g (19.0 mmol) of 2-(3-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 10.3 g (70%) of Target Compound 2-71.

### [Preparation Example 2-14] Preparation of Compound 2-74

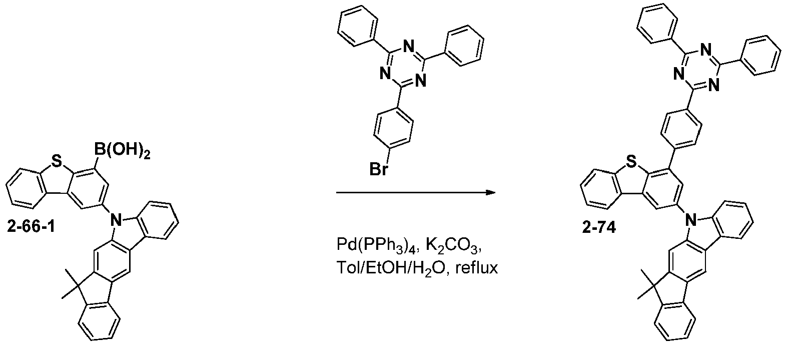

9.7 g (19.0 mmol) of Compound 2-66-1, 7.4 g (19.0 mmol) of 2-(4-bromophenyl)-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 10.3 g (70%) of Target Compound 2-74.

### [Preparation Example 2-15] Preparation of Compound 2-79

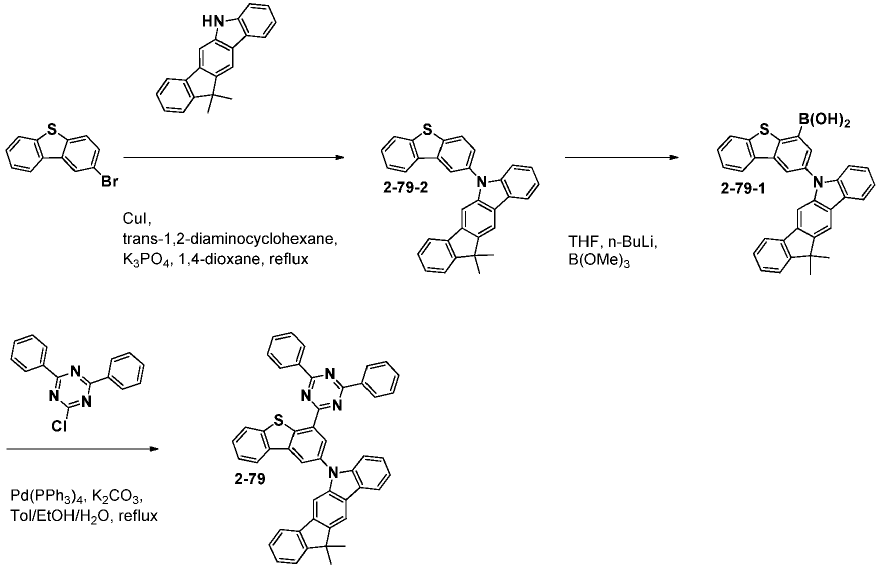

### Preparation of Compound 2-79-2

5.0 g (19.0 mmol) of 2-bromodibenzo[b,d]thiophene, 4.5 g (15.8 mmol) of 11,11-dimethyl-5,11-dihydroindeno[1,2-b]carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 5.9 g (80%) of Target Compound 2-79-2.

### Preparation of Compound 2-79-1

7.4 mL (18.6 mmol) of 2.5 M n-BuLi was added dropwise to a mixed solution containing 6.7 g (14.3 mmol) of Compound 2-79-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 5.1 g (70%) of Target Compound 2-79-1.

### Preparation of Compound 2-79

9.7 g (19.0 mmol) of Compound 2-79-1, 5.1 g (19.0 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.3 g (70%) of Target Compound 2-79.

### [Preparation Example 2-16] Preparation of Compound 2-83

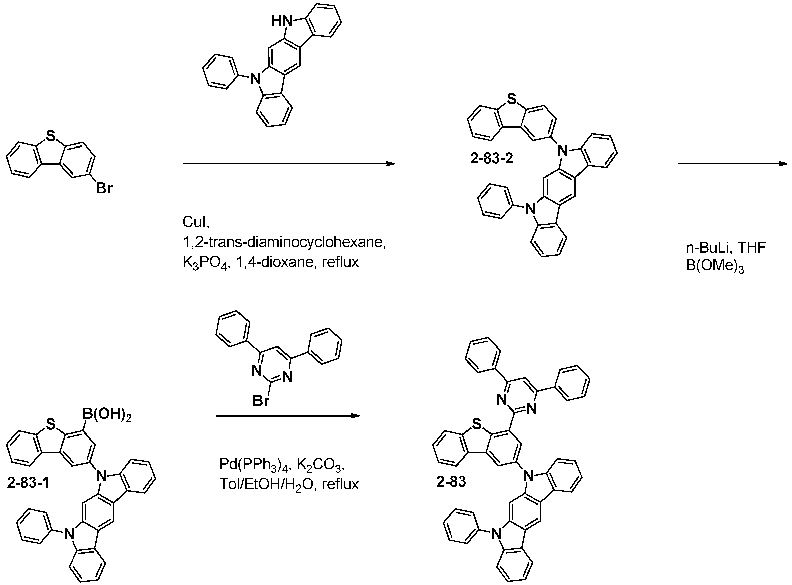

### Preparation of Compound 2-83-2

5.0 g (19.0 mmol) of 2-bromodibenzo[b,d]thiophene, 5.3 g (15.8 mmol) of 5-phenyl-5,7-dihydroindolo[2,3-b]carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 6.9 g (85%) of Target Compound 2-83-2.

### Preparation of Compound 2-83-1

7.4 mL (18.6 mmol) of 2.5 M n-BuLi was added dropwise to a mixed solution containing 7.4 g (14.3 mmol) of Compound 2-83-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 5.6 g (70%) of Target Compound 2-83-1.

### Preparation of Compound 2-83

10.6 g (19.0 mmol) of Compound 2-83-1, 5.9 g (19.0 mmol) of 2-bromo-4,6-diphenylpyrimidine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.3 g (70%) of Target Compound 2-83.

### [Preparation Example 2-17] Preparation of Compound 2-85

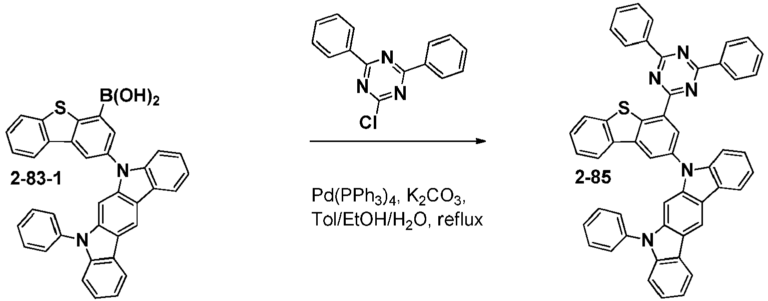

10.6 g (19.0 mmol) of Compound 2-83-1, 5.1 g (19.0 mM) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.3 g (70%) of Target Compound 2-85.

### [Preparation Example 2-18] Preparation of Compound 2-99

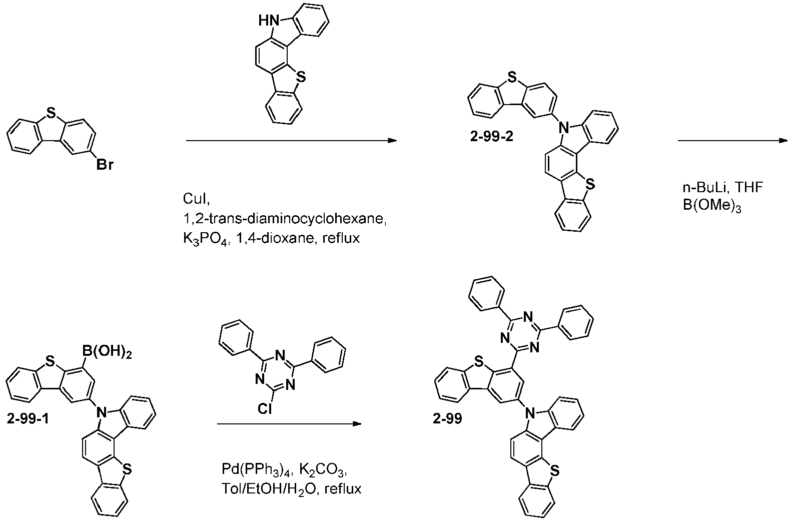

### Preparation of Compound 2-99-2

5.0 g (19.0 mmol) of 2-bromodibenzo[b,d]thiophene, 4.3 g (15.8 mmol) of 5H-benzo[4,5]thieno[3,2-c]carbazole, 3.0 g (15.8 mmol) of CuI, 1.9 mL (15.8 mmol) of trans-1,2-diaminocyclohexane, and 3.3 g (31.6 mmol) of K₃PO₄ were dissolved in 100 mL of 1,4-dioxane, and then the resulting solution was refluxed for 24 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 7.2 g (85%) of Target Compound 2-99-2.

### Preparation of Compound 2-99-1

7.4 mL (18.6 mmol) of 2.5 M n-BuLi was added dropwise to a mixed solution containing 6.5 g (14.3 mmol) of Compound 2-99-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 5.0 g (70%) of Target Compound 2-99-1.

### Preparation of Compound 2-99

9.5 g (19.0 mmol) of Compound 2-99-1, 5.1 g (19.0 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.9 g (70%) of Target Compound 2-99.

### [Preparation Example 2-19] Preparation of Compound 2-164

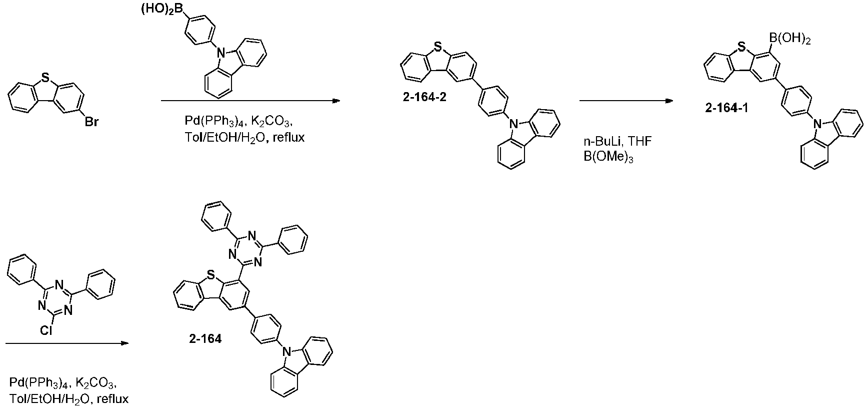

### Preparation of Compound 2-164-2

5 g (19.0 mmol) of 2-bromodibenzo[b,d]thiophene, 5.5 g (19.0 mmol) of (4-(9H-carbazol-9-yl)phenyl)boronic acid, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 8.9 g (70%) of Target Compound 2-164-2.

### Preparation of Compound 2-164-1

7.4 mL (18.6 mmol) of 2.5 M n-BuLi was added dropwise to a mixed solution containing 6.09 g (14.3 mmol) of Compound 2-164-2 and 100 mL of THF at -78°C, and the resulting mixture was stirred at room temperature for 1 hour. 4.8 mL (42.9 mmol) of trimethyl borate (B(OMe)₃) was added dropwise to the reaction mixture, and the resulting mixture was stirred at room temperature for 2 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:MeOH = 100:3) and recrystallized with DCM to obtain 4.7 g (70%) of Target Compound 2-164-1.

### Preparation of Compound 2-164

8.9 g (19.0 mmol) of Compound 2-164-1, 6.1 g (22.8 mmol) of 2-chloro-4,6-diphenyl-1,3,5-triazine, 1.1 g (0.95 mmol) of Pd(PPh₃)₄, and 5.2 g (38.0 mmol) of K₂CO₃ were dissolved in 100/20/20 mL of toluene/EtOH/H₂O, and then the resulting solution was refluxed for 12 hours. After the reaction was completed, distilled water and DCM were added thereto at room temperature, extraction was performed, the organic layer was dried over MgSO₄, and then the solvent was removed by a rotary evaporator. The reactant was purified by column chromatography (DCM:Hex = 1:3) and recrystallized with methanol to obtain 9.0 g (72%) of Target Compound 2-164.

### [Preparation Example 3-1] Preparation of Compound 3-87

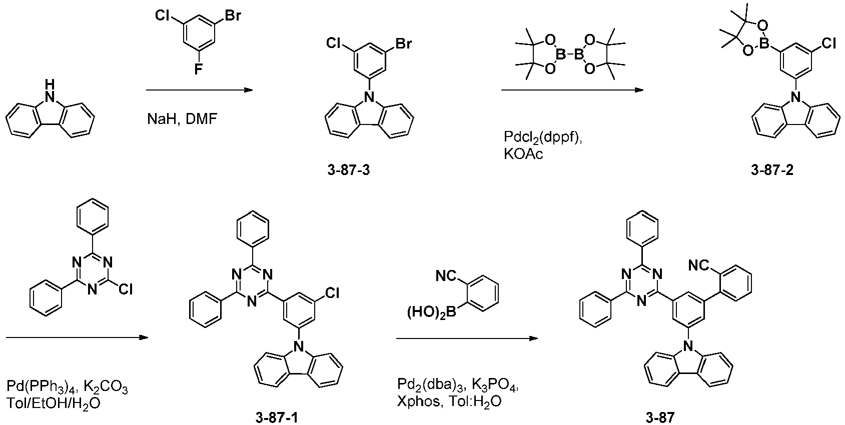

### Preparation of Compound 3-87-3

8.9 g (52.93 mmol) of a compound 9H-carbazole, 22.1 g (105.8 mmol) of 1-bromo-3-chloro-5-fluorobenzene, 1.9 g (79.40 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 13.2 g (70%) of Target Compound 3-87-3.

### Preparation of Compound 3-87-2

14.3 g (40.18 mmol) of Compound 3-87-3, 13.26 g (52.24 mmol) of bis(pinacolato)diboron, 0.8 g (1.205 mmol) of PdCl₂(dppf), 11.8 g (120.5 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 12.7 g (78%) of Target Compound 3-87-2.

### Preparation of Compound 3-87-1

14 g (34.6 mmol) of Compound 3-87-2, 11.2 g (41.5 mmol) of 2-chloro-4,6-diphenyl-1,3,5-triazine, 2 g (1.7 mmol) of Pd(PPh₃)₄, 14.3 g (103.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 14.1 g (80%) of Target Compound 3-87-1.

### Preparation of Compound 3-87

4.9 g (9.582 mmol) of Compound 3-87-1, 2.1 g (14.37 mmol) of (2-cyanophenyl)boronic acid, 0.61 g (0.67 mmol) of Pd₂(dba)₃, 6.1 g (28.7 mmol) of K₃PO₄, 0.91 g (1.91 mmol) of Xphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.0 g (73%) of Target Compound 3-87.

### [Preparation Example 3-2] Preparation of Compound 3-99

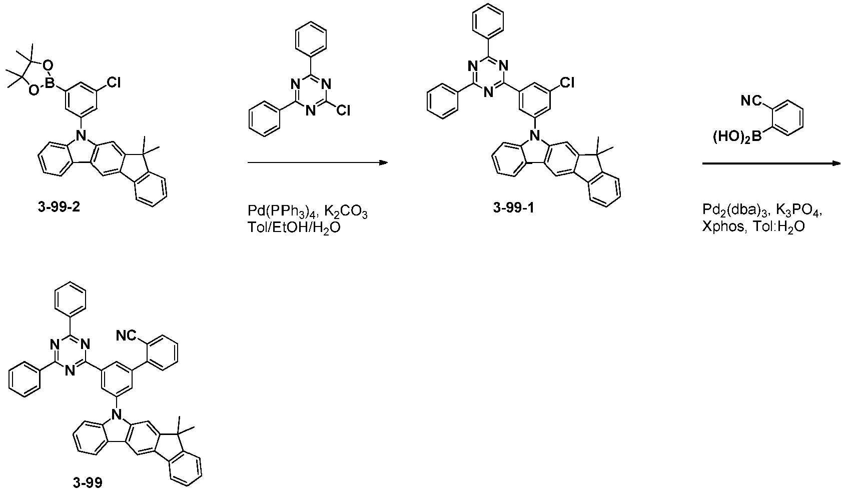

### Preparation of Compound 3-99-1

18 g (34.6 mmol) of Compound 3-203-2, 11.2 g (41.5 mmol) of 2-chloro-4,6-diphenyl-1,3,5-triazine, 2 g (1.7 mmol) of Pd(PPh₃)₄, 14.3 g (103.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 16 g (77%) of Target Compound 3-99-1.

### Preparation of Compound 3-99

6 g (9.58 mmol) of Compound 3-99-1, 2.1 g (14.37 mmol) of (2-cyanophenyl)boronic acid, 0.61 g (67 mmol) of Pd₂(dba)₃, 6.1 g (28.7 mmol) of K₃PO₄, 0.91 g (1.91 mmol) of Xphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.1 g (73%) of Target Compound 3-99.

### [Preparation Example 3-3] Preparation of Compound 3-203

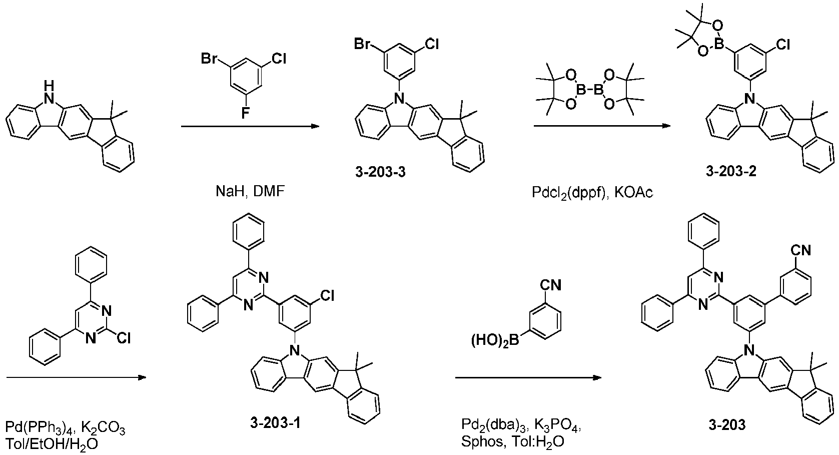

### Preparation of Compound 3-203-3

15 g (52.93 mmol) of a compound 7,7-dimethyl-5,7-dihydroindeno[2,1-b]carbazole, 22.1 g (105.8 mmol) of 1-bromo-3-chloro-5-fluorobenzene, 1.9 g (79.4 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 19 g (76%) of Target Compound 3-203-3.

### Preparation of Compound 3-203-2

19 g (40.18 mmol) of Compound 3-203-3, 13.26 g (52.24 mmol) of bis(pinacolato)diboron, 0.8 g (1.21 mmol) of PdCl₂(dppf), 11.8 g (120.5 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 18 g (76%) of Target Compound 3-203-2.

### Preparation of Compound 3-203-1

18 g (34.6 mmol) of Compound 3-203-2, 11.0 g (41.5 mmol) of 2-chloro-4,6-diphenylpyrimidine, 2 g (1.7 mmol) of Pd(PPh₃)₄, 14.3 g (103.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 15 g (78%) of Target Compound 3-203-1.

### Preparation of Compound 3-203

5 g (7.98 mmol) of Compound 3-203-1, 1.5 g (10.38 mmol) of (3-cyanophenyl)boronic acid, 0.36 g (39 mmol) of Pd₂(dba)₃, 5.2 g (23.9 mmol) of K₃PO₄, 0.33 g (79 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 3.4 g (61%) of Target Compound 3-203.

### [Preparation Example 3-4] Preparation of Compound 3-251

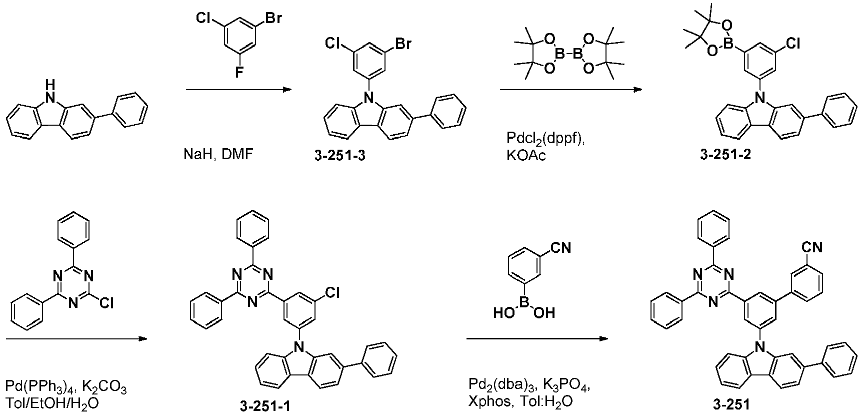

### Preparation of Compound 3-251-3

12.8 g (52.89 mmol) of a compound 2-phenyl-9H-carbazole, 22.1 g (105.8 mmol) of 1-bromo-3-chloro-5-fluorobenzene, 1.9 g (79.40 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 17.2 g (74%) of Target Compound 3-251-3.

### Preparation of Compound 3-251-2

17.3 g (40.09 mmol) of Compound 3-251-3, 13.26 g (52.24 mmol) of bis(pinacolato)diboron, 0.8 g (1.205 mmol) of PdCl₂(dppf), 11.8 g (120.5 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 14.8 g (74%) of Target Compound 3-251-2.

### Preparation of Compound 3-251-1

11.7 g (24.53 mmol) of Compound 3-251-2, 7.9 g (29.48 mmol) of 2-chloro-4,6-diphenyl-1,3,5-triazine, 1.4 g (1.2 mmol) of Pd(PPh₃)₄, 10.1 g (73.7 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 10 g (70%) of Target Compound 3-251-1.

### Preparation of Compound 3-251

5.6 g (9.582 mmol) of Compound 3-251-1, 2.1 g (14.37 mmol) of 3-cyanophenylboronic acid, 0.61 g (0.67 mmol) of Pd₂(dba)₃, 6.1 g (28.7 mmol) of K₃PO₄, 0.91 g (1.91 mmol) of Xphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 3.6 g (58%) of Target Compound 3-251.

### [Preparation Example 3-5] Preparation of Compound 3-260

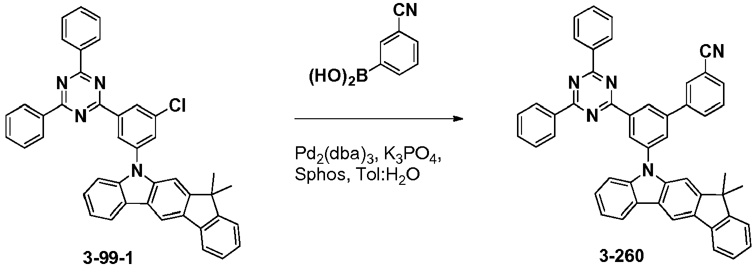

5 g (7.98 mmol) of Compound 3-99-1, 1.5 g (10.38 mmol) of (3-cyanophenyl)boronic acid, 0.36 g (39 mmol) of Pd₂(dba)₃, 5.2 g (23.9 mmol) of K₃PO₄, 0.33 g (79 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 3.5 g (64%) of Target Compound 3-260.

### [Preparation Example 3-6] Preparation of Compound 3-262

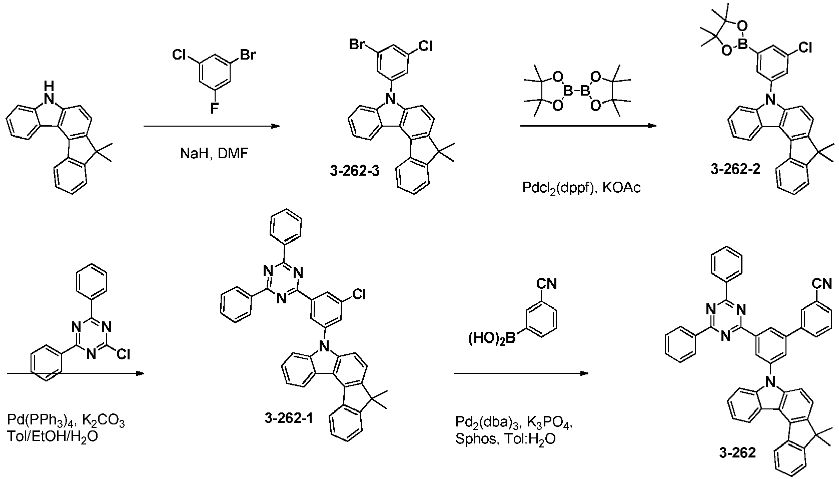

### Preparation of Compound 3-262-3

15 g (53mmol) of a compound 11,11-dimethyl-5,11-dihydroindeno[1,2-b]carbazole, 24.3 g (106.01 mmol) of 1-bromo-3-chloro-5-fluorobenzene, 3.1 g (79.54 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 17 g (71%) of Target Compound 3-262-3.

### Preparation of Compound 3-262-2

17 g (40.25 mmol) of Compound 3-262-3, 15.2 g (60.81 mmol) of bis(pinacolato)diboron, 1.4 g (2.01 mmol) of PdCl₂(dppf), 11.8 g (120.7 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 16 g (72%) of Target Compound 3-262-2.

### Preparation of Compound 3-262-1

16 g (34.68 mmol) of Compound 3-262-2, 19.4 g (52.02 mmol) of 2-chloro-4,6-diphenyl-1,3,5-triazine, 2.6 g (1.7 mmol) of Pd(PPh₃)₄, 14.3 g (109.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 16 g (75%) of Target Compound 3-262-1.

### Preparation of Compound 3-262

6 g (9.64 mmol) of Compound 3-262-1, 2.1 g (14.42 mmol) of (3-cyanophenyl)boronic acid, 0.44 g (48 mmol) of Pd₂(dba)₃, 6.1 g (28.9 mmol) of K₃PO₄, 0.39 g (94 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.6 g (77%) of Target Compound 3-262.

### [Preparation Example 3-7] Preparation of Compound 3-264

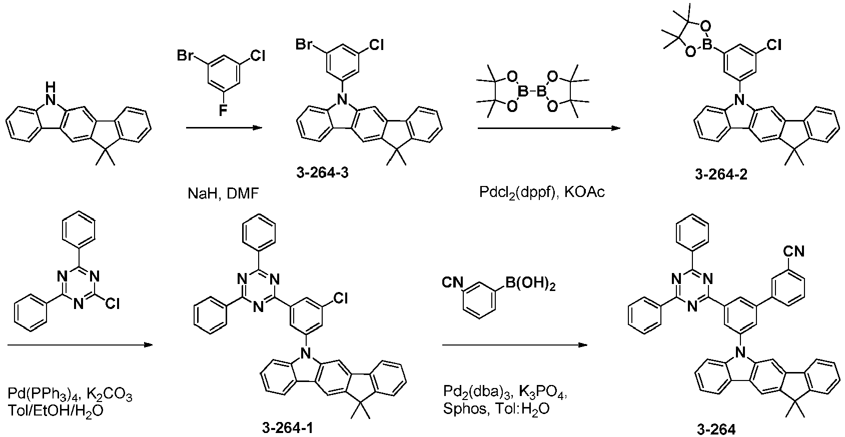

### Preparation of Compound 3-264-3

15 g (53 mmol) of a compound 11,11-dimethyl-5,11-dihydroindeno[1,2-b]carbazole, 24.3 g (106.01 mmol) of 1-bromo-3-chloro-5-fluorobenzene, 3.1 g (79.54 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 19 g (78%) of Target Compound 3-264-3.

### Preparation of Compound 3-264-2

19 g (40.25 mmol) of Compound 3-264-3, 15.2 g (60.81 mmol) of bis(pinacolato)diboron, 1.4 g (2.01 mmol) of PdCl₂(dppf), 11.8 g (120.7 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 18 g (73%) of Target Compound 3-264-2.

### Preparation of Compound 3-264-1

18 g (34.68 mmol) of Compound 3-264-2, 19.4 g (52.02 mmol) of 2-chloro-4,6-diphenyl-1,3,5-triazine, 2.6 g (1.7 mmol) of Pd(PPh₃)₄, 14.3 g (109.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 16 g (74%) of Target Compound 3-264-1.

### Preparation of Compound 3-264

6 g (9.64 mmol) of Compound 3-264-1, 2.1 g (14.42 mmol) of (3-cyanophenyl)boronic acid, 0.44 g (48 mmol) of Pd₂(dba)₃, 6.1 g (28.9 mmol) of K₃PO₄, 0.39 g (94 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.6 g (75%) of Target Compound 3-264.

### [Preparation Example 3-8] Preparation of Compound 3-267

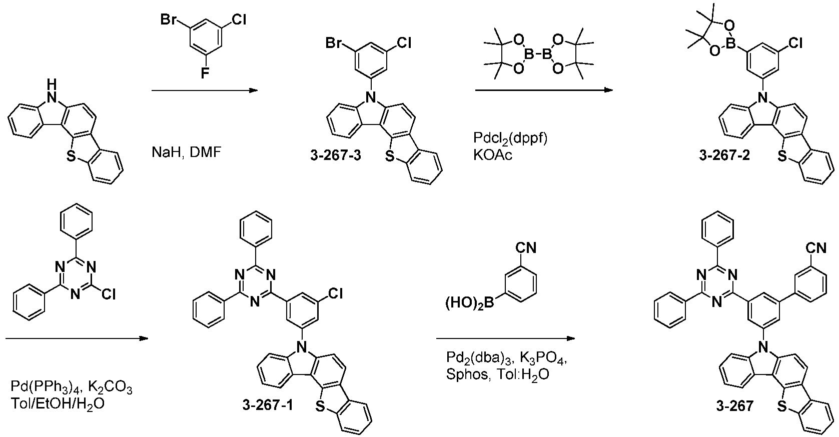

### Preparation of Compound 3-267-3

15 g (54.94 mmol) of a compound 5H-benzo[4,5]thieno[3,2-c]carbazole, 22.9 g (109.89 mmol) of 1-bromo-3-chloro-5-fluorobenzene, 3.2 g (82.41 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 17 g (76%) of Target Compound 3-267-3.

### Preparation of Compound 3-267-2

17 g (41.12 mmol) of Compound 3-267-3, 15.6 g (61.68 mmol) of bis(pinacolato)diboron, 1.5 g (2.056 mmol) of PdCl₂(dppf), 12.08 g (123.3 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 19 g (76%) of Target Compound 3-267-2.

### Preparation of Compound 3-267-1

17.7 g (34.68 mmol) of Compound 3-267-2, 19.4 g (52.02 mmol) of 2-chloro-4,6-diphenyl-1,3,5-triazine, 2.6 g (1.7 mmol) of Pd(PPh₃)₄, 14.3 g (109.8 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 15.8 g (74%) of Target Compound 3-267-1.

### Preparation of Compound 3-267

5.9 g (9.64 mmol) of Compound 3-267-1, 2.1 g (14.42 mmol) of (3-cyanophenyl)boronic acid, 0.44 g (48 mmol) of Pd₂(dba)₃, 6.1 g (28.9 mmol) of K₃PO₄, 0.39 g (94 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.9 g (75%) of Target Compound 3-267.

### [Preparation Example 3-9] Preparation of Compound 3-271

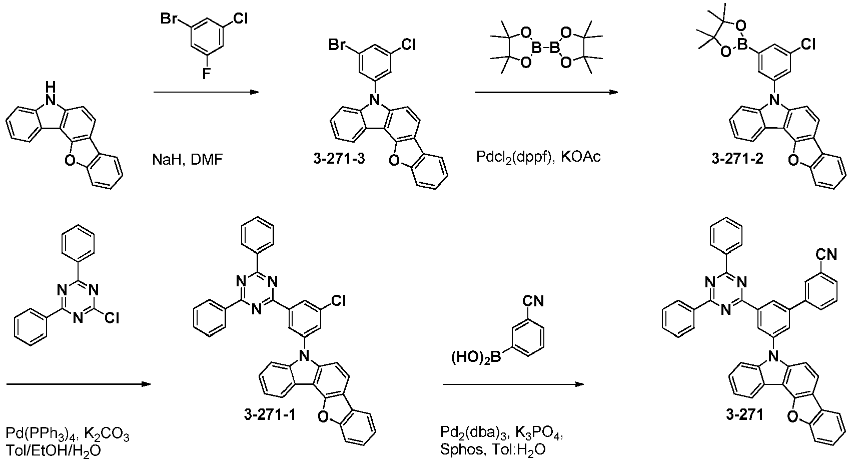

### Preparation of Compound 3-271-3

15 g (58.36 mmol) of a compound 5H-benzofuro[3,2-c]carbazole, 24.3 g (116.73 mmol) of 1-bromo-3-chloro-5-fluorobenzene, 3.5 g (87.54 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 18 g (73%) of Target Compound 3-271-3.

### Preparation of Compound 3-271-2

18 g (42.6 mmol) of Compound 3-271-2, 15.6 g (63.90 mmol) of bis(pinacolato)diboron, 1.5 g (2.130 mmol) of PdCl₂(dppf), 12.08 g (127.9 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 17 g (78%) of Target Compound 3-271-2.

### Preparation of Compound 3-271-1

17 g (36.51 mmol) of Compound 3-271-2, 19.4 g (73.02 mmol) of 2-chloro-4,6-diphenyl-1,3,5-triazine, 2.6 g (1.7 mmol) of Pd(PPh₃)₄, 14.3 g (106.0 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 18 g (71%) of Target Compound 3-271-1.

### Preparation of Compound 3-271

6 g (8.34 mmol) of Compound 3-271-1, 1.8 g (12.52 mmol) of (3-cyanophenyl)boronic acid, 0.38 g (0.41 mmol) of Pd₂(dba)₃, 5.3 g (25.1 mmol) of K₃PO₄, 0.34 g (0.83 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.6 g (72%) of Target Compound 3-271.

### [Preparation Example 3-10] Preparation of Compound 3-416

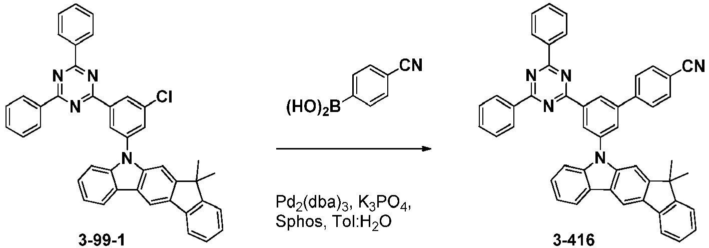

5 g (7.98 mmol) of Compound 3-99-1, 1.5 g (10.38 mmol) of (4-cyanophenyl)boronic acid, 0.36 g (39 mmol) of Pd₂(dba)₃, 5.2 g (23.9 mmol) of K₃PO₄, 0.33 g (79 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.1 g (73%) of Target Compound 3-416.

### [Preparation Example 3-11] Preparation of Compound 3-421

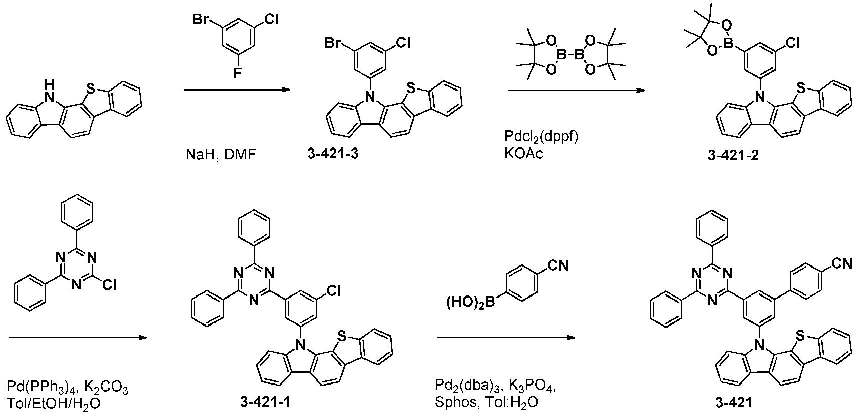

### Preparation of Compound 3-421-3

15 g (54.94 mmol) of a compound 12H-benzo[4,5]thieno[2,3-a]carbazole, 22.9 g (109.89 mmol) of 1-bromo-3-chloro-5-fluorobenzene, 3.2 g (82.41 mmol) of sodium hydride, and 200 mL of DMF were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel and florisil, and the solvent was removed to obtain 17 g (76%) of Target Compound 3-421-3.

### Preparation of Compound 3-421-2

17 g (41.12 mmol) of Compound 3-421-3, 15.6 g (61.68 mmol) of bis(pinacolato)diboron, 1.5 g (2.056 mmol) of PdCl₂(dppf), 12.08 g (123.3 mmol) of KOAc, and 150 mL of 1,4-dioxane were put into a reactor, and the resulting mixture was reacted at 120°C for 5 hours. The resulting product was cooled to room temperature, and then an extraction was performed using distilled water and dichloromethane. Thereafter, the extract was dissolved in dichloromethane, the resulting solution was filtered using silica gel, celite, and florisil, and the solvent was removed to obtain 19 g (76%) of Target Compound 3-421-2.

### Preparation of Compound 3-421-1

19 g (35.36 mmol) of Compound 3-421-2, 14.1 g (53.04 mmol) of 2-chloro-4,6-diphenyl-1,3,5-triazine, 2 g (1.7 mmol) of Pd(PPh₃)₄, 14.3 g (106.0 mmol) of K₂CO₃, 120 mL of toluene, 20 mL of ethanol, and 20 mL of water were put into a reactor, and the resulting mixture was reacted at 120°C for 8 hours. The resulting product was cooled to room temperature, and then an extraction was performed using distilled water and dichloromethane. Thereafter, the extract was dissolved in dichloromethane, the resulting solution was filtered using silica gel, celite, and florisil, and the filtrate was recrystallized with dichloromethane and methanol to obtain 15 g (77%) of Target Compound 3-421-1.

### Preparation of Compound 3-421

5 g (8.13 mmol) of Compound 3-421-1, 1.7 g (12.19 mmol) of (4-cyanophenyl)boronic acid, 0.36 g (0.40 mmol) of Pd₂(dba)₃, 5.8 g (24.39 mmol) of K₃PO₄, 0.33 g (0.79 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.5 g (78%) of Target Compound 3-421.

### [Preparation Example 3-12] Preparation of Compound 3-422

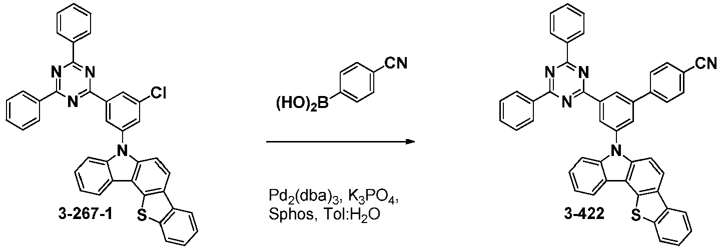

### Preparation of Compound 3-422

5 g (8.13 mmol) of Compound 3-267-1, 1.7 g (12.19 mmol) of (4-cyanophenyl)boronic acid, 0.36 g (0.40 mmol) of Pd₂(dba)₃, 5.8 g (24.39 mmol) of K₃PO₄, 0.33 g (0.79 mmol) of Sphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.3 g (72%) of Target Compound 3-422.

### [Preparation Example 3-13] Preparation of Compound 3-450

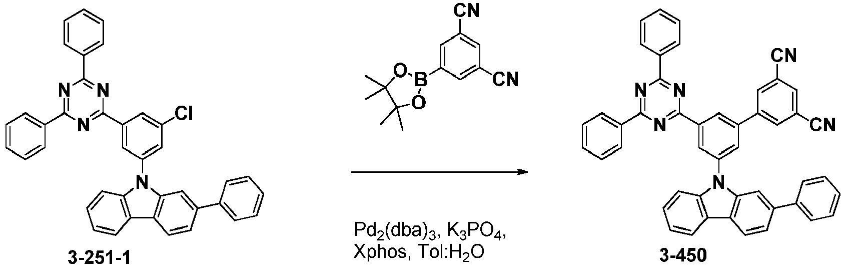

5.6 g (9.582 mmol) of Compound 3-251-1, 3.7 g (14.37 mmol) of 3,5-dicyanophenylboronic acid pinacol ester, 0.61 g (0.67 mmol) of Pd₂(dba)₃, 6.1 g (28.7 mmol) of K₃PO₄, 0.91 g (1.91 mmol) of Xphos, 60 mL of toluene, and 10 mL of water were put into a reactor, and the resulting mixture was subjected to nitrogen substitution. The resulting product was refluxed and reacted for 12 hours, and an extraction was performed using distilled water and dichloromethane. After the organic layer was dried over anhydrous MgSO₄, the solvent was removed by a rotary evaporator, and then the resulting product was column-purified at a ratio of dichloromethane and hexane of 1:1 to obtain 4.4 g (68%) of Target Compound 3-450.

Compounds were prepared in the same manner as in the Preparation Examples, and the synthesis confirmation results thereof are shown in the following Tables 1 to 4. Table 1 shows NMR values, and Table 2 shows measured values by field desorption mass spectrometry (FD-MS).

**[Table 1]**

| Compound | ¹H NMR(CDCl₃, 400Mz) |
|---|---|
| 1-1 | 8.55(d, 2H), 8.08(d, 2H), 7.87∼7.94(m, 4H), 7.25-7.66(m, 24H), |
| 1-13 | 8.49∼8.55(m, 2H), 8.08∼8.12(m, 3H), 7.87∼7.94(m, 2H), 7.25∼7.63(m, 25H) |
| 1-21 | 8.55(m, 2H), 8.08(d, 1H), 7.87∼7.94(m, 4H), 7.77(s, 1H), 7.25∼7.69(m, 24H) |
| 1-31 | 8.55(d, 1H), 8.08∼8.12(m, 3H), 7.87∼7.94(m, 3H), 7.25∼7.66(m, 29H) |
| 1-41 | 8.20(dd, 4H), 7.82(s, 1H), 7.78(s, 2H), 7.68(d, 2H), 7.64-7.58(m, 12H), 7.48-7.36(m, 9H), 7.32-7.29(m, 2H) |
| 1-43 | 8.19(dd, 4H), 7.81(s, 3H), 7.72(dd, 4H), 7.63-7.57(m, 14H), 7.47-7.35(m, 9H), 7.32-7.28(m, 2H) |
| 1-44 | 8.17(dd, 4H), 8.02(d, 1H), 7.92-7.86(m, 3H), 7.71(s, 2H), 7.64-7.61(m, 4H), 7.57-7.52(m, 10H), 7.48-7.38(m, 8H), 7.30-7.27(m, 2H) |
| 1-45 | 8.19(dd, 4H), 8.11(s, 1H), 7.93-7.78(m, 7H), 7.64-7.55(m, 11H), 7.49-7.39(m, 9H), 7.30-7.27(m, 2H) |
| 1-48 | 8.20(dd, 4H), 7.83-7.79(m, 4H), 7.74(d,4H 1H), 7.71(s, 1H), 7.66-7.62(m, 5H), 7.58-7.55(m, 8H), 7.45-7.41(m, 7H), 7.35-7.28(m, 4H), 1.53(s, 6H) |
| 1-49 | 8.23-8.14(m, 6H), 7.96(s, 2H), 7.91(s, 1H), 7.80(t, 1H), 7.69(s, 2H), 7.65(d, 2H), 7.60-7.55(m, 10H), 7.47-7.40(m, 8H), 7.31-7.28(m, 2H) |
| 1-50 | 8.25-8.17(m, 6H), 8.06(s, 2H), 7.85(s, 1H), 7.80(d, 2H), 7.76(s, 1H), 7.61(d, 2H), 7.54-7.45(m, 9H), 7.40-7.30(m, 9H), 7.27-7.23(m, 2H) |
| 1-54 | 8.49∼8.50(m, 2H), 8.10∼8.18(m, 3H), 7.87∼7.94(m, 3H), 7.79(d, 1H), 7.17∼7.66(m, 24H), 7.07(t, 1H), 1.72(s, 6H) |
| 1-60 | 8.52(d, 1H), 8.10∼8.18(m, 3H), 7.90∼7.94(m, 2H), 7.25∼7.66(m, 26H) |
| 1-70 | 8.49(d, 1H), 8.10∼8.18(m, 4H), 8.00(d, 1H), 7.77(s, 1H), 7.41∼7.58(m, 23H), 7.29(m, 2H) |
| 1-82 | 8.49(d, 1H), 8.10∼8.18(m, 4H), 8.00(d, 1H), 7.87∼7.89(m, 2H), 7.7(s, 1H) 7.29∼7.63(m, 22H), 7.17∼7.20(m, 2H), 7.07(t, 1H), 1.72(s, 6H) |
| 1-90 | 8.50(s, 2H), 8.24(d, 2H), 8.03(s, 1H), 7.91(s, 2H), 7.80(dd, 4H), 7.66-7.62(m, 8H), 7.53-7.40(m, 11H), 7.33-7.31(m, 2H) |
| 1-97 | 8.52(s, 2H), 8.25(d, 2H), 8.04(s, 1H), 7.96(d, 2H), 7.87-7.77(m, 6H), 7.66-7.61(m, 9H), 7.55-7.44(m, 10H), 7.37-7.30(m, 4H), 1.58(s, 6H) |
| 1-100 | 8.54(s, 2H), 8.20(t, 4H), 8.13(s, 1H), 8.08(s, 2H), 7.85(t, 3H), 7.71(d, 1H), 7.64-7.61(m, 9H), 7.54-7.42(m, 10H), 7.32-7.29(m, 3H), 7.17(d, 1H) |
| 1-101 | 8.55(s, 2H), 8.23(d, 4H), 8.12(s, 1H), 8.02(t, 2H), 7.86(dd, 2H), 7.80(d, 2H), 7.66-7.61(m, 9H), 7.55-7.43(m, 10H), 7.41-7.30(m, 3H) |
| 1-103 | 8.55(d, 1H), 8.12∼8.18(m, 2H), 7.87∼8.00(m, 3H), 7.77∼7.79(m, 4H), 7.25∼7.68(m, 26H) |
| 1-110 | 8.10∼8.18(m, 4H), 8.00(d, 1H), 7.90(d, 1H), 7.77(s. 1H), 7.29∼7.66(m, 25H) |
| 1-120 | 8.52(d, 1H), 8.10∼8.12(m, 2H), 7.90∼7.94(d, 1H), 7.79(d, 1H), 7.25∼7.66(m, 27H) |
| 2-11 | 9.32(1H, d), 8.90∼8.88(4H, m), 8.61(1H, d), 8.25(2H, d), 8.21(1H, d), 8.12(1H, d), 7.65∼7.45(12H, m), 7.37(2H, t) |
| 2-12 | 8.55(1H, d), 8.45∼8.36(4H, m), 8.19(1H, d), 7.93∼8.00(4H, m), 7.73∼7.77(4H, m), 7.35∼7.61(12H, m), 7.20∼7.20(2H, m) |
| 2-28 | 9.27(1H, s), 8.89(1H, d), 8.79(4H, m), 8.41(1H, d), 8.21(3H, m), 8.05(1H, d), 7.93(1H, d), 7.87(1H, d), 7.77(1H, t), 7.64∼7.46(12H, m), 7.32(2H, t) |
| 2-36 | 8.55(d, 1H), 8.45(d, 1H), 8.28(d, 4H), 8.12(d, 1H), 7.85∼7.98(m, 5H), 7.63∼7.69(m, 2H), 7.25∼7.52(m, 14H) |
| 2-39 | 9.35(1H, s), 8.90(4H, d), 8.64(1H, s), 8.30∼8.20(3H, m), 8.13(1H, d), 7.71(1H, s), 7.66∼7.45(14H, m), 7.38∼7.33(3H, m) |
| 2-40 | 9.38(1H, s), 9.24(1H, s), 8.87(3H, d), 8.64(1H, s), 8.30∼8.21(3H, m), 8.08(1H, d), 7.90(1H, d), 7.80(2H, d), 7.72∼7.32(19H, d) |
| 2-41 | 8.55(1H, d), 8.45(1H, d), 8.28(2H, d), 8.18(1H, d), 7.79∼7.98(6H, m), 7.69(1H, s), 7.62(1H, s), 7.25∼7.52(15H, m) |
| 2-42 | 9.30(1H, s), 8.90(1H, d), 8.80(4H, d), 8.43(1H, s), 8.26∼8.20(3H, m), 8.03(1H, d), 7.93(1H, d), 7.87(1H, s), 7.77(1H, t), 7.72(1H, s), 7.67(1H, d), 7.60∼7.47(11H, m), 7.41∼7.29(4H, m) |
| 2-43 | 8.55(1H, d), 8.45(1H, d), 8.36∼8.31(5H, m), 8.00∼7.91(6H, m), 7.77∼7.74(4H, m), 7.56∼7.35(12H, m), 7.25(2H, d), 7.16(1H, t) |
| 2-47 | 8.62(1H, d), 8.45(1H, d), 8.36∼8.31(5H, m), 8.22(1H, m), 8.00(1H, s), 7.93∼7.91(2H, m), 7.77∼7.74(7H, m), 7.50∼7.41(14H, m) |
| 2-66 | 9.33(1H, d), 8.59(1H, d), 8.53(1H, s), 8.42(4H, m), 8.28(1H, d), 8.22(1H, d), 8.15(1H, s), 8.09(1H, d), 7.90(1H, d), 7.59∼7.51(10H, m), 7.50∼7.34(4H, m), 7.17(1H, m), 1.53∼1.50(6H, d) |
| 2-68 | 9.35(1H, s), 8.90(4H, d), 8.64(1H, s), 8.30∼8.20(3H, m), 8.13(1H, d), 7.71(1H, s), 7.66∼7.45(14H, m), 7.38∼7.33(3H, m) |
| 2-71 | 9.26(1H, s), 8.84(1H, d), 8.49(1H, s), 8.43(1H, s), 8.31(4H, m), 8.25∼8.20(2H, m), 8.07(1H, s), 7.71(1H, t), 7.55(10H, m), 7.45∼7.28(5H, m), 1.53∼1.50(6H, d) |
| 2-74 | 8.55(1H, d), 8.45(1H, d), 8.28(4H, d), 8.09(1H, d), 7.85∼7.94(5H, m), 7.69∼7.70(2H, m), 7.24∼7.52(15H, m) |
| 2-79 | 9.36 (1H, s), 8.89(2H, d), 8.64(1H, s), 8.27∼8.21(3H, m), 8.13 (1H, d), 7.79(1H, s), 7.67∼7.50(9H, m), 7.47∼7.35(4H, m), 7.30∼7.17(2H, m), 1.69(6H, s) |
| 2-83 | 9.31(1H, s), 8.92(1H, s), 8.57(1H, s), 8.43(4H, m), 8.35(1H, d), 8.31(1H, d), 8.18(2H, m), 8.05(1H, d), 7.63∼7.47(11H, m), 7.45∼7.28(7H, m), 7.20(1H, m) |
| 2-85 | 8.38(s, 1H), 8.92(s, 1H), 8.87(d, 4H), 8.62(s, 1H), 8.35(dd, 1H), 8.17(dd, 1H), 7.18∼7.65(m, 22H) |
| 2-99 | 8.55(1H, d), 8.45(1H, d), 8.36(4H, d), 8.05∼7.93(5H, m), 7.77(1H, s), 7.56∼7.49(10H, m), 7.35∼7.33(2H, m), 7.16(1H, t) |
| 2-164 | 8.55(d, 2H), 8.45(d, 1H), 8.28(d, 4H), 7.94∼7.98(m, 3H), 7.75∼7.79(m, 3H), 7.63∼7.68(m, 3H), 7.25∼7.52(m, 12H) |
| 3-99 | 9.12(1H,s), 9.00(1H,s), 8.76(4H,d), 8.47(1H,s), 8.24(1H,d), 8.05(1H,s), 7.89(2H,d), 7.78(3H,t), 7.65(1H,d),7.58(7H,m), 7.74(2H,t), 7.38(2H,m), 7.30(1H,t), 1.56(6H,s) |
| 3-203 | 8.55(1H,d), 8.31(2H,s), 8.24(1H,d), 8.23(1H,s), 8.02(1H,s), 7.94(5H,m), 7.90(1H,s), 7.88(1H,s), 7.85(1H,s), 7.79(1H,d), 7.74(1H,d), 7.72(1H,t), 7.57(1H,t), 7.55(4H,t), 7.49(3H,t), 7.38(1H,t), 7.35(1H,t), 7.16(1H,t), 1.69(6H,s) |
| 3-251 | 8.55(d, 1H), 8.28(d, 4H), 8.18(d, 1H), 7.88∼8.05(m, 5H), 7.62∼7.80(m, 5H), 7.25∼7.51(m, 13H) |
| 3-262 | 8.55(1H,s), 8.36(4H,d), 8.31(2H,s), 8.24(1H,d), 7.94(1H,d), 7.90(1H,s), 7.85(1H,s), 7.79(1H,d), 7.74(1H,d), 7.72(1H,t), 7.67(1H,d), 7.64(1H,d), 7.57(1H,t), 7.50(6H,m), 7.38(1H,t), 7.16(1H,t) |
| 3-260 | 9.11(1H,s), 9.06(1H,s), 8.79(4H,d), 8.51(1H,s), 8.27(1H,d), 8.12 (1H,s), 8.07 (2H,t), 7.91(1H,d), 7.78(1H,d), 7.70(1H,t), 7.62(8H,m), 7.46(2H,d), 7.38(2H,t), 7.32(1H,t), 1.55(6H,s) |
| 3-264 | 8.55(1H,d), 8.36(4H,d), 8.31(2H,s), 8.24(2H,d), 8.02(1H,d), 7.94(1H,d), 7.90(1H,s), 7.85(1H,s), 7.79(1H,d), 7.74(1H,d), 7.72(1H,t), 7.57(1H,t), 7.50(6H,m), 7.38(1H,t), 7.35(1H,t), 7.16(1H,t) |
| 3-267 | 8.55(1H, d), 8.45(1H, d), 8.28(4H, d), 7.88∼8.05(7H, m), 7.69∼7.73(2H, m), 7.25∼7.52(11H, m) |
| 3-271 | 8.55(1H,d), 8.36(4H,d), 8.31(2H,s), 8.02(1H,d), 7.98(1H,d), 7.94(1H,s), 7.90(1H,s), 7.85(1H,s), 7.84(1H,d), 7.79(1H,d), 7.72(1H,t), 7.54(1H,d), 7.50(6H,m), 7.39(1H,t), 7.35(1H,t), 7.31(1H,t), 7.16(1H,t), 7.13(1H,d) |
| 3-416 | δ= 9.12(1H,s), 9.03(1H,s), 8.79(4H,d), 8.15(1H,s), 8.27(1H,d), 8.11(1H,s), 7.90 (1H,d), 7.85(2H,d), 7.62(10H,m), 7.49(3H,t), 7.40(1H,t), 7.26(1H,d), 1.57(6H,s) |
| 3-421 | 8.70(1H,s), 8.55(1H,d), 8.31(1H,s), 8.29(3H,d), 8.20(2H,s), 8.19(1H,d), 7.94(1H,d), 7.84(4H,d), 7.75(2H,d), 7.62(2H,t), 7.58(2H,t), 7.55(3H,t), 7.50(3H,t), 7.49(3H,t), 7.41(1H,t), 7.40(1H,s), 7.35(1H,t), 7.20(1H,t), 7.16(1H,t) |
| 3-422 | 8.55(1m, d), 8.45(1H, d), 8.28(4H, d), 7.82∼8.05(10H, m), 7.25∼7.52(11H, m) |
| 3-450 | 8.55(1H,d), 8.28(4H,d), 8.18(1H,d), 8.01∼8.05(4H,m), 7.79∼7.94(3H,m), 7.62(1H,s), 7.25∼7.52(14H,m) |

**[Table 2]**

| Compound | FD-MS | Compound | FD-MS |
|---|---|---|---|
| 1-1 | m/z= 636.78(C₄₈H₃₂N₂ =636.26) | 1-13 | m/z= 636.78(C₄₈H₃₂N₂= 636.26) |
| 1-21 | m/z= 636.78(C₄gH₃₂N₂ = 636.26) | 1-31 | m/z= 712.88(C₅₄H₃₆N₂ = 712.29) |
| 1-41 | m/z= 637.26(C₄₈H₃₂N₂ = 636.26) | 1-43 | m/z= 713.36(C₅₄H₃₆N₂ = 712.29) |
| 1-44 | m/z= 867.40(C₅₂H₃₄N₂ = 686.27) | 1-45 | m/z= 687.41(C₅₂H₃₄N₂ = 686.27) |
| 1-48 | m/z= 753.32(C₅₂H₃₄N₂ = 752.32) | 1-49 | m/z= 743.17(C₅₄H₃₄N₂S = 742.24) |
| 1-50 | m/z= 727.04(C₅₄H₃₄N₂O = 726.27) | 1-54 | m/z= 752.94(C₅₇H₄₀N₂ = 752.32) |
| 1-60 | m/z= 636.78(C₄₈H₃₂N₂= 636.26) | 1-70 | m/z= 636.78(C₄₈H₃₂N₂= 636.26) |
| 1-82 | m/z= 752.94(C₅₇H₄₀N₂ = 752.32) | 1-110 | m/z= 636.78(C₄₈H₃₂N₂= 636.26) |
| 1-120 | m/z= 636.78(C₄₈H₃₂N₂= 636.26) | 1-90 | m/z= 637.24(C₄₈H₃₂N₂ = 636.20) |
| 1-97 | m/z= 753.17(C₅₇H₄₀N₂ = 752.32) | 1-100 | m/z= 743.29(C₅₄H₃₄N₂S = 742.24) |
| 1-101 | m/z= 727.27(C₅₄H₃₄N₂O = 726.27) | 1-103 | m/z= 712.88(C₅₄H₃₆N₂ = 712.29) |
| 2-11 | m/z= 580.70(C₃₉H₂₄N₄S = 580.17) | 2-12 | m/z= 656.80(C₄₅H₂₈N₄S = 656.20) |
| 2-28 | m/z= 656.80(C₄₅H₂₈N₄S = 656.20) | 2-36 | m/z= 656.80(C₄₅H₂₈N₄S = 656.20) |
| 2-39 | m/z= 656.80(C₄₅H₂₈N₄S = 656.20) | 2-40 | m/z= 732.89(C₅₁H₃₂N₄S = 732.23) |
| 2-41 | m/z= 732.89(C₅₁H₃₂N₄S = 732.23) | 2-42 | m/z= 732.89(C₅₁H₃₂N₄S = 732.23) |
| 2-43 | m/z= 732.89(C₅₁H₃₂N₄S = 732.23) | 2-47 | m/z= 732.89(C₅₁H₃₂N₄S = 732.23) |
| 2-66 | m/z= 695.87(C₄₉H₃₃N₃S = 695.24) | 2-68 | m/z= 696.86(C₄₈H₃₂N₄S = 696.23) |
| 2-71 | m/z= 772.96(C₅₄H₃₁N₄S = 772.27) | 2-74 | m/z= 772.96(C₅₄H₃₆N₄S= 772.27) |
| 2-79 | m/z= 698.86(C₄₈H₃₂N₄S = 696.23) | 2-83 | m/z= 744.90(C₅₂H₃₂N₄S = 744.23) |
| 2-85 | m/z= 745.89(C₅₁H₃₁N₅S = 745.23) | 2-99 | m/z= 686.84(C₄₅H₂₆N₄S₂ = 686.16) |
| 2-164 | m/z= 656.80(C₄₅H₂₈N₄S= 656.20) | 3-87 | m/z= 575.66(C₄₀H₂₅N₅ = 575.21) |
| 3-99 | m/z= 691.27(C₄₉H₃₃N₅ = 691.27) | 3-203 | m/z= 690.83(C₅₀H₃₄N₄ = 690.28) |
| 3-251 | m/z= 651.76(C₄₆H₂₉N₅= 651.24) | 3-260 | m/z= 691.82(C₄₉H₃₃N₅ = 691.27) |
| 3-262 | m/z= 691.82(C₄₉H₃₃N₅ = 691.27) | 3-264 | m/z= 691.82(C₄₉H₃₃N₅ = 691.27) |
| 3-267 | m/z= 681.81(C₄₆H₂₇N₅S = 681.20) | 3-271 | m/z= 665.74(C₄₆H₂₇N₅O = 665.22) |
| 3-416 | m/z= 691.82(C₄₉H₃₃N₅ = 691.27) | 3-421 | m/z= 681.81(C₄₆H₂₇N₅S = 681.20) |
| 3-422 | m/z= 681.80(C₄₆H₂₇N₅S = 681.20) | 3-450 | m/z= 676.77(C₄₇H₂₈N₆ = 676.24) |

**[Table 3]**

| | Eox (V) of NPB | Eox (V) of Compound x | UV absorption edge (nm) | HOMO (eV) | Band gap (eV) | LUMO (eV) |
|---|---|---|---|---|---|---|
| Compound 1-41 | 0.78 | 1.39 | 361 | -6.12 | 3.43 | -2.69 |
| Compound 1-43 | 0.78 | 1.40 | 362 | -6.12 | 3.43 | -2.69 |
| Compound 1-45 | 0.78 | 1.41 | 361 | -6.13 | 3.43 | -2.70 |
| Compound 1-49 | 0.78 | 1.39 | 362 | -6.10 | 3.43 | -2.67 |
| Compound 1-50 | 0.78 | 1.36 | 362 | -6.08 | 3.43 | -2.65 |
| Compound 1-90 | 0.78 | 1.30 | 359 | -6.01 | 3.45 | -2.56 |
| Compound 1-97 | 0.78 | 1.28 | 412 | -6.00 | 3.01 | -2.99 |
| Compound 1-100 | 0.79 | 1.35 | 360 | -6.06 | 3.45 | -2.61 |
| Compound 1-101 | 0.78 | 1.31 | 359 | -6.02 | 3.45 | -2.57 |
| Compound 2-11 | 0.80 | 1.48 | 432 | -6.18 | 2.87 | -3.31 |
| Compound 2-28 | 0.80 | 1.37 | 369 | -6.07 | 3.36 | -2.71 |
| Compound 2-36 | 0.79 | 1.34 | 410 | -6.06 | 3.02 | -3.04 |
| Compound 2-39 | 0.76 | 1.41 | 433 | -6.15 | 2.86 | -3.29 |
| Compound 2-40 | 0.78 | 1.37 | 435 | -6.80 | 2.85 | -3.23 |
| Compound 2-41 | 0.79 | 1.55 | 433 | -6.26 | 2.86 | -3.4 |
| Compound 2-42 | 0.78 | 1.40 | 366 | -6.12 | 3.39 | -2.73 |
| Compound 2-66 | 0.79 | 1.22 | 416 | -5.93 | 2.98 | -2.95 |
| Compound 2-68 | 0.76 | 1.16 | 452 | -5.89 | 2.74 | -3.15 |
| Compound 2-71 | 0.76 | 1.14 | 371 | -5.88 | 3.34 | -2.54 |
| Compound 2-79 | 0.78 | 1.30 | 438 | -6.02 | 2.83 | -3.19 |
| Compound 2-83 | 0.78 | 1.09 | 426 | -5.81 | 2.91 | -2.90 |
| Compound 2-84 | 0.77 | 1.08 | 463 | -5.80 | 2.68 | -3.12 |
| Compound 2-99 | 0.78 | 1.31 | 436 | -5.94 | 2.84 | -3.1 |
| Compound 2-164 | 0.78 | 1.39 | 362 | -6.1 | 3.43 | -2.67 |
| Compound 3-260 | 0.78 | 1.18 | 424 | -5.90 | 2.92 | -2.98 |
| Compound 3-416 | 0.78 | 1.26 | 425 | -5.98 | 2.92 | -3.06 |
| HOMO = -5.5-(Eox(Compound x)-Eox(NPB)) (eV) | | | | | | |
| Band gap = 1240/UV absorption edge (eV) | | | | | | |

**[Table 4]**

| | T₁ (eV) | Tg(°C) | Tm(°C) | Td(°C) |
|---|---|---|---|---|
| Compound 1-45 | 2.52 | 133 | ND | 511 |
| Compound 1-49 | 2.65 | 152 | ND | 528 |
| Compound 1-50 | 2.66 | 144 | ND | 520 |
| Compound 1-70 | 2.67 | 128 | ND | 478 |
| Compound 1-72 | 2.63 | 141 | ND | 517 |
| Compound 1-73 | 2.62 | 138 | ND | 509 |
| Compound 1-90 | 2.75 | 127 | ND | 494 |
| Compound 1-97 | 2.57 | 1.51 | 349, 354 | 511 |
| Compound 1-100 | 2.74 | 151 | ND | 543 |
| Compound 1-101 | 2.76 | 145 | ND | 530 |
| Compound 2-11 | 2.50 | ND | 321 | 453 |
| Compound 2-28 | 2.72 | ND | 294 | 464 |
| Compound 2-36 | 2.58 | 145 | 289, 307 | 507 |
| Compound 2-39 | 2.50 | 152 | 338 | 495 |
| Compound 2-40 | 2.50 | 147 | 300 | 519 |
| Compound 2-41 | 2.53 | 164 | 310 | 511 |
| Compound 2-42 | 2.64 | 158 | ND | 516 |
| Compound 2-66 | 2.59 | 179 | ND | 475 |
| Compound 2-68 | 2.50 | 185 | ND | 475 |
| Compound 2-71 | 2.77 | 195 | ND | 494 |
| Compound 2-79 | 2.48 | 181 | 330 | 485 |
| Compound 2-83 | 2.59 | ND | 327 | 529 |
| Compound 2-85 | 2.46 | 190 | 314 | 517 |
| Compound 2-99 | 2.55 | 177 | 400 | 521 |
| Compound 2-164 | 2.65 | 152 | ND | 528 |
| Compound 3-260 | 2.61 | 177 | ND | 471 |
| Compound 3-416 | 2.67 | 160 | ND | 472 |

### <Experimental Examples>

### 1) Manufacture of Organic Light Emitting Device

A glass substrate thinly coated with ITO to have a thickness of 1,500 Å was ultrasonically washed with distilled water. When the washing with distilled water is finished, the glass substrate was ultrasonically washed with a solvent such as acetone, methanol, and isopropyl alcohol, dried and then was subjected to UVO treatment for 5 minutes by using UV in a UV washing machine. Thereafter, the substrate was transferred to a plasma washing machine (PT), and then was subjected to plasma treatment in order to implement an ITO work function in a vacuum state and remove a residual film, and thus, was transferred to a thermal deposition equipment for organic deposition.

On the ITO prepared as described above, 4,4',4"-tris[2-naphthyl(phenyl)amino]triphenylamine (2-TNATA) was formed as a hole injection layer, and N,N'-di(1-naphthyl)-N,N'-diphenyl-(1,1'-biphenyl)-4,4'-diamine (NPB) was formed as a hole transporting layer. A light emitting layer was formed to have a thickness of 400 Å by performing thermal vacuum deposition on the hole transporting layer.

For the light emitting layer, the compound described in the following Table, which is a host, was deposited at a speed ratio of 1:1 at each cell, and the light emitting layer was used by being doped with 7% of tris(2-phenylpyridine)iridium (Ir(ppy)₃) as a phosphorescent dopant. Thereafter, BCP was deposited to have a thickness of 60 Å as a hole blocking layer, and Alq₃ was deposited to have a thickness of 200 Å as an electron transporting layer thereon. Finally, lithium fluoride (LiF) was deposited to have a thickness of 10 Å on the electron transporting layer to form an electron injection layer, and then aluminum (Al) was deposited to have a thickness of 1,200 Å on the electron injection layer to form a negative electrode, thereby manufacturing an organic electroluminescence device.

Meanwhile, all the organic compounds required for manufacturing an OLED were subjected to vacuum sublimed purification under 10⁻⁶ to 10⁻⁸ torr for each material, and then used for the manufacture of OLED.

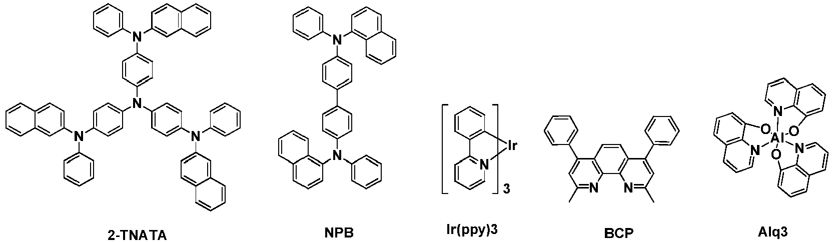

### 2) Driving Voltage and Light Emitting Efficiency of Organic Electroluminescence

### Device

For the organic electroluminescence device manufactured as described above, electroluminescence (EL) characteristics were measured by M7000 manufactured by McScience Inc., and based on the measurement result thereof, T₉₀ was measured by a service life measurement equipment (M6000) manufactured by McScience Inc., when the reference luminance was 5,000 cd/m². The characteristics of the organic electroluminescence device of the present invention are as shown in the following Table 5 and Table 6.

**[Table 5]**

| | Host (Compound No.) (Weight ratio) | Driving voltage (V) | Efficiency (cd/A) | Color coordinate (x, y) | Service life hr (T80) |
|---|---|---|---|---|---|
| Example 1 | 1-1 | 7.62 | 19.5 | (0.296, 0.647) | 215 |
| Example 2 | 1-13 | 6.92 | 11.2 | (0.306, 0.705) | 211 |
| Example 3 | 1-21 | 6.43 | 13.1 | (0.297, 0.659) | 108 |
| Example 4 | 1-31 | 7.22 | 8.8 | (0.276, 0.657) | 105 |
| Example 5 | 1-60 | 7.81 | 14.5 | (0.286,0.717) | 105 |
| Example 6 | 1-70 | 6.62 | 10.5 | (0.256, 0.647) | 109 |
| Example 7 | 1-72 | 6.62 | 10.1 | (0.287, 0.655) | 211 |
| Example 8 | 1-90 | 7.13 | 13.5 | (0.302, 0.659) | 108 |
| Example 9 | 1-97 | 7.72 | 9.8 | (0.316, 0.647) | 105 |
| Example 10 | 1-100 | 6.81 | 11.5 | (0.276, 0.697) | 105 |
| Example 11 | 1-101 | 6.52 | 7.2 | (0.294, 0.668) | 107 |
| Example 12 | 1-112 | 6.21 | 9.1 | (0.316, 0.712) | 105 |
| Example 13 | 1-120 | 7.92 | 16.2 | (0.284, 0.730) | 107 |
| Comparative Example 1 | CBP | 7.81 | 28.4 | (0.282, 0.717) | 45 |
| Comparative Example 2 | Ref 1 | 6.19 | 28.1 | (0.271,0.710) | 43 |
| Comparative Example 3 | Ref 2 | 6.47 | 32.5 | (0.276, 0.682) | 36 |
| Comparative Example 4 | Ref 3 | 6.58 | 28.4 | (0.312, 0.701) | 46 |

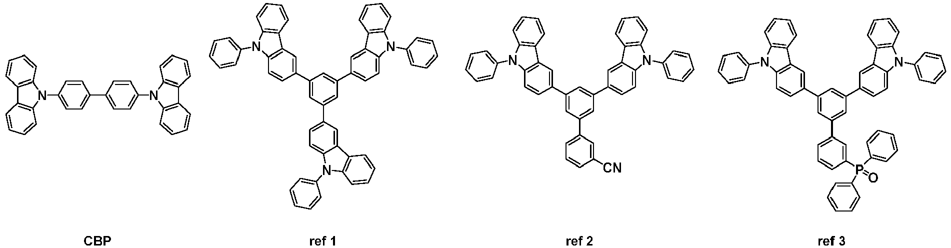

The compounds of the present invention are strong P-type compounds, and it was confirmed that the compounds exhibited better device characteristics when compared to the compounds in the Comparative Examples.

**[Table 6]**

| | Host (Compound No.) (Weight ratio) | Driving voltage (V) | Efficiency (cd/A) | Color coordinate (x, y) | Service life hr (T90) |
|---|---|---|---|---|---|
| Example 14 | 1-1 : 2-39 (1:1) | 3.68 | 61.5 | (0.281, 0.724) | 584 |
| Example 15 | 1-70 : 2-39 (1:1) | 3.77 | 65.8 | (0.295, 0.658) | 621 |
| Example 16 | 1-70 : 2-39 (1:2) | 3.95 | 60.2 | (0.296, 0.698) | 589 |
| Example 17 | 1-70 : 2-39 (1:3) | 4.11 | 62.2 | (0.272, 0.677) | 523 |
| Example 18 | 1-70 : 2-39 (1:4) | 4.53 | 63.2 | (0.286, 0.705) | 487 |
| Example 19 | 1-70 : 2-39 (1:5) | 4.73 | 66.2 | (0.281, 0.698) | 480 |
| Example 20 | 1-70 : 2-39 (2:1) | 3.67 | 61.2 | (0.262, 0.705) | 715 |
| Example 21 | 1-70 : 2-39 (3:1) | 3.62 | 59.3 | (0.279, 0.695) | 685 |
| Example 22 | 1-70 : 2-39 (4:1) | 3.41 | 57.0 | (0.287, 0.685) | 601 |
| Example 23 | 1-70 : 2-39 (5:1) | 3.73 | 54.2 | (0.252, 0.701) | 622 |
| Example 24 | 1-1 : 2-68 (1:1) | 3.36 | 54.9 | (0.286,0.717) | 613 |
| Example 25 | 1-1 : 3-260 (1:1) | 3.88 | 58.5 | (0.276, 0.727) | 698 |
| Example 26 | 1-1 : 3-264 (1:1) | 3.47 | 64.2 | (0.294, 0.657) | 629 |
| Example 27 | 1-13 : 2-39 (1:1) | 3.78 | 59.2 | (0.281, 0.704) | 454 |
| Example 28 | 1-13 : 2-68 (1:1) | 3.86 | 58.9 | (0.286, 0.697) | 651 |
| Example 29 | 1-13 : 3-260 (1:1) | 3.38 | 63.5 | (0.276, 0.687) | 558 |
| Example 30 | 1-13 : 3-264 (1:1) | 3.42 | 64.2 | (0.284, 0.657) | 679 |
| Example 31 | 1-21 : 2-39 (1:1) | 3.68 | 60.5 | (0.271, 0.674) | 496 |
| Example 32 | 1-21 : 2-68 (1:1) | 3.36 | 58.9 | (0.286, 0.697) | 551 |
| Example 33 | 1-21 : 3-260 (1:1) | 3.88 | 63.5 | (0.276, 0.687) | 728 |
| Example 34 | 1-21 : 3-264 (1:1) | 3.57 | 63.4 | (0.284, 0.657) | 559 |
| Example 35 | 1-31 : 2-39 (1:1) | 3.68 | 61.5 | (0.271, 0.674) | 584 |
| Example 36 | 1-31 : 2-68 (1:1) | 3.39 | 57.9 | (0.286, 0.697) | 591 |
| Example 37 | 1-31 : 3-260 (1:1) | 3.48 | 63.5 | (0.276, 0.687) | 548 |
| Example 38 | 1-31 : 3-264 (1:1) | 3.47 | 64.2 | (0.284, 0.657) | 582 |
| Example 39 | 1-50 : 2-39 (1:1) | 3.68 | 61.4 | (0.271, 0.674) | 514 |
| Example 40 | 1-50 : 2-41 (1:1) | 4.01 | 60.2 | (0.284, 0.681) | 595 |
| Example 41 | 1-50 : 2-68 (1:1) | 3.86 | 55.9 | (0.286, 0.697) | 654 |
| Example 42 | 1-50 : 3-251 (1:1) | 3.75 | 57.1 | (0.286, 0.687) | 616 |
| Example 43 | 1-50 : 3-260 (1:1) | 3.58 | 63.1 | (0.276, 0.687) | 498 |
| Example 44 | 1-50 : 3-264 (1:1) | 3.47 | 65.2 | (0.284, 0.657) | 589 |
| Example 45 | 1-50 : 3-416 (1:1) | 3.76 | 48.7 | (0.286, 0.690) | 625 |
| Example 46 | 1-50 : 3-422 (1:1) | 3.60 | 67.1 | (0.276, 0.687) | 656 |
| Example 47 | 1-60 : 2-39 (1:1) | 3.67 | 61.5 | (0.271, 0.674) | 584 |
| Example 48 | 1-60 : 2-68 (1:1) | 3.56 | 58.9 | (0.286, 0.697) | 654 |
| Example 49 | 1-60 : 3-260 (1:1) | 3.98 | 63.5 | (0.276, 0.687) | 598 |
| Example 50 | 1-60 : 3-264 (1:1) | 3.47 | 64.2 | (0.284, 0.657) | 689 |
| Example 51 | 1-70 : 2-39 (1:1) | 3.86 | 60.7 | (0.291, 0.676) | 495 |
| Example 52 | 1-70 : 2-41 (1:1) | 3.92 | 58.1 | (0.281,0.705) | 665 |
| Example 53 | 1-70 : 2-68 (1:1) | 3.78 | 57.8 | (0.311, 0.685) | 456 |
| Example 54 | 1-70 : 3-251 (1:1) | 3.64 | 58.5 | (0.251, 0.691) | 473 |
| Example 55 | 1-70 : 3-260 (1:1) | 3.45 | 66.5 | (0.301, 0.676) | 620 |
| Example 56 | 1-70 : 3-264 (1:1) | 3.98 | 59.5 | (0.281, 0.663) | 512 |
| Example 57 | 1-70 : 3-416 (1:1) | 4.01 | 49.5 | (0.265, 0.678) | 670 |
| Example 58 | 1-70 : 3-422 (1:1) | 3.46 | 53.2 | (0.282, 0.695) | 523 |
| Example 59 | 1-101 : 2-39 (1:1) | 4.24 | 67.7 | (0.284, 0.657) | 412 |
| Example 60 | 1-101 : 2-41 (1:1) | 3.67 | 65.4 | (0.286, 0.687) | 521 |
| Example 61 | 1-101 : 2-68 (1:1) | 3.66 | 62.8 | (0.276, 0.657) | 543 |
| Example 62 | 1-101 : 3-251 (1:1) | 3.59 | 58.5 | (0.255, 0.693) | 513 |
| Example 63 | 1-101 : 3-260 (1:1) | 3.78 | 63.5 | (0.286, 0.705) | 619 |
| Example 64 | 1-101 : 3-264 (1:1) | 3.46 | 59.5 | (0.256, 0.662) | 494 |
| Example 65 | 1-101 : 3-416 (1:1) | 3.52 | 61.5 | (0.301, 0.674) | 625 |
| Example 66 | 1-101 : 3-422 (1:1) | 3.44 | 60.2 | (0.291, 0.647) | 650 |
| Example 67 | 1-112: 2-39 (1:1) | 4.12 | 61.5 | (0.271, 0.674) | 484 |
| Example 68 | 1-112: 2-68 (1:1) | 3.36 | 58.9 | (0.286,0.717) | 641 |
| Example 69 | 1-112 : 3-260 (1:1) | 3.83 | 63.5 | (0.310, 0.687) | 598 |
| Example 70 | 1-112 : 3-264 (1:1) | 3.47 | 64.2 | (0.294, 0.707) | 719 |
| Example 71 | 1-120 : 2-39 (1:1) | 3.68 | 61.5 | (0.271, 0.674) | 484 |
| Example 72 | 1-120 : 2-68 (1:1) | 3.30 | 58.9 | (0.296, 0.727) | 711 |
| Example 73 | 1-120 : 3-260 (1:1) | 3.38 | 63.5 | (0.256, 0.697) | 598 |
| Example 74 | 1-120 : 3-264 (1:1) | 4.17 | 64.2 | (0.284, 0.657) | 629 |
| Comparative Example 5 | 2-39 | 4.21 | 42.9 | (0.276, 0.687) | 130 |
| Comparative Example 6 | 2-41 | 4.51 | 53.5 | (0.255, 0.703) | 102 |
| Comparative Example 7 | 2-68 | 3.95 | 50.2 | (0.291, 0.680) | 210 |
| Comparative Example 8 | 3-251 | 4.59 | 52.5 | (0.286, 0.720) | 182 |
| Comparative Example 9 | 3-260 | 3.93 | 48.1 | (0.284, 0.681) | 245 |
| Comparative Example 10 | 3-264 | 3.75 | 51.7 | (0.280, 0.697) | 234 |
| Comparative Example 11 | 3-416 | 3.82 | 52.5 | (0.271, 0.674) | 185 |
| Comparative Example 12 | 3-422 | 4.14 | 49.2 | (0.316, 0.697) | 191 |

As seen in the results, in particular, the organic light emitting device according to an exemplary embodiment of the present application includes as a host material of a light emitting layer: both the compound represented by Chemical Formula 1; and the compound represented by Chemical Formula 2 or 3, and thus may exhibit significantly improved characteristics in terms of all of the driving, efficiency, and service life as compared to an organic light emitting device to which a single compound is applied as a host material.

This is because a P-N type host material is used to improve the balance between holes and electrons, thereby enhancing the characteristics of the device.

## Claims

1. A compound represented by the following Chemical Formula 1: 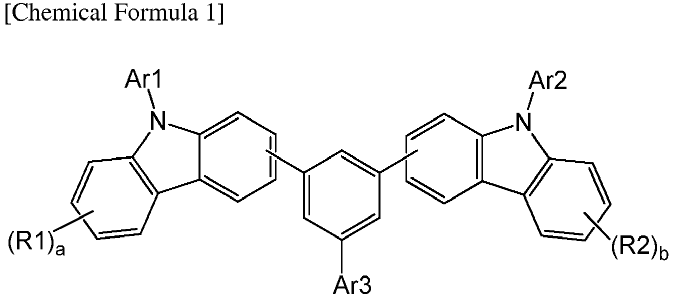 in Chemical Formula 1,
Ar1 to Ar3 are the same as or different from each other, and are each independently a substituted or unsubstituted C₆ to C₆₀ aryl group; or a substituted or unsubstituted C₂ to C₆₀ heteroaryl group,
R1 and R2 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group,
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
a and b are each independently an integer from 0 to 4.

2. The hetero-cyclic compound of claim 1, wherein Chemical Formula 1 is represented by any one of the following Chemical Formulae 1a to 1j: 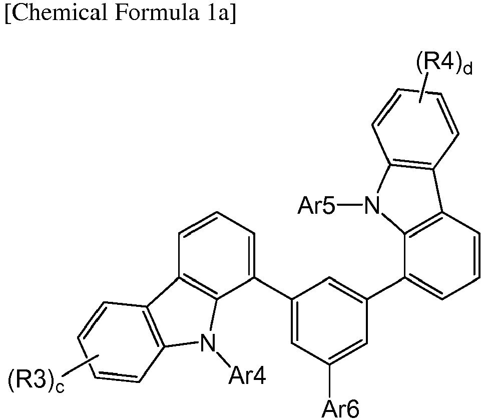 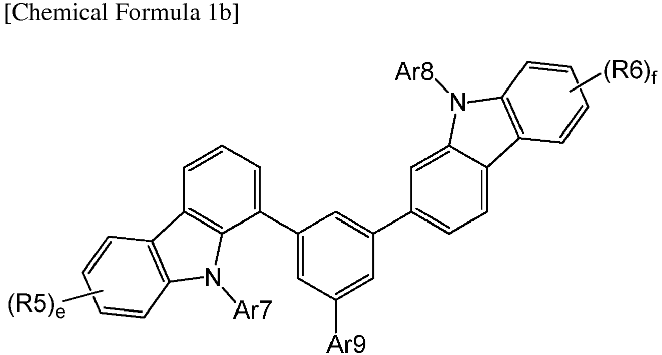 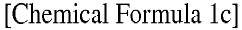 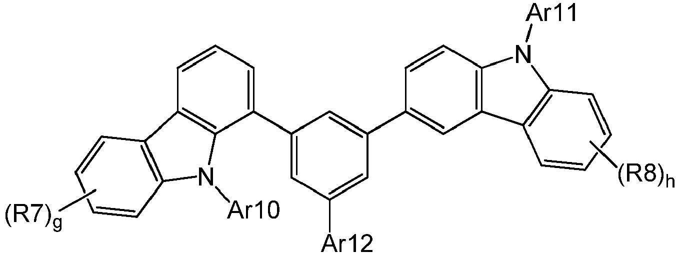 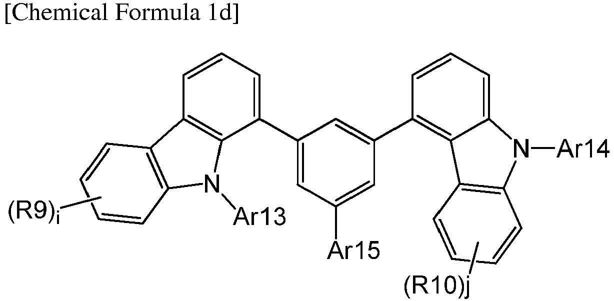 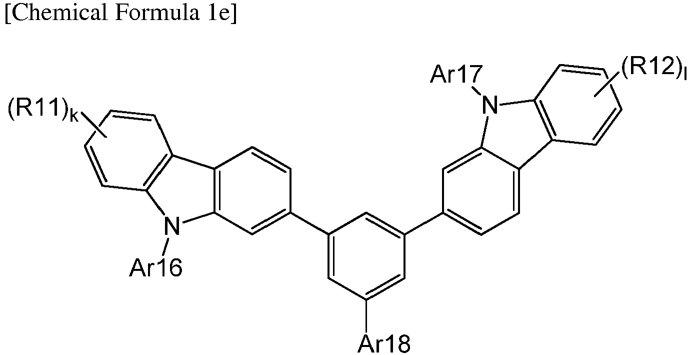 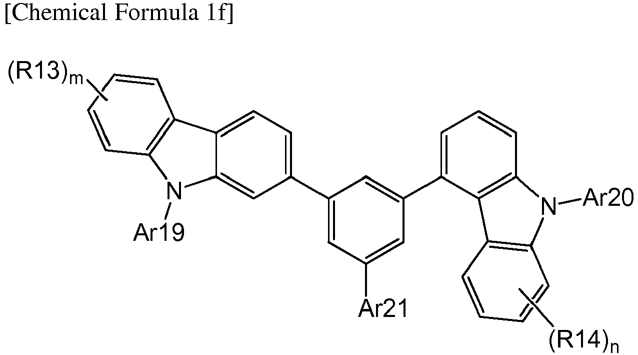 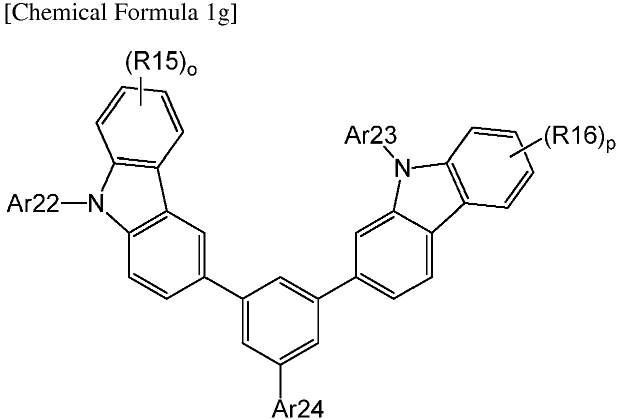 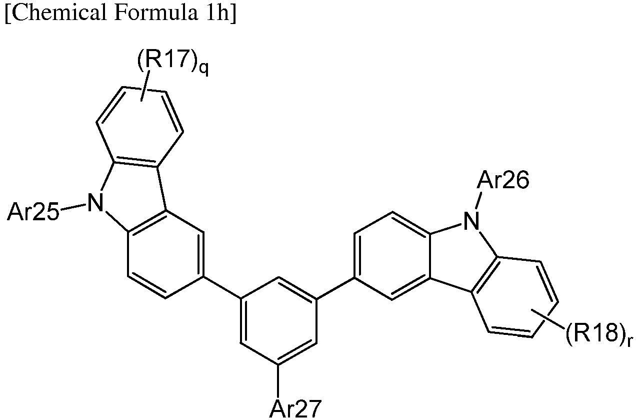 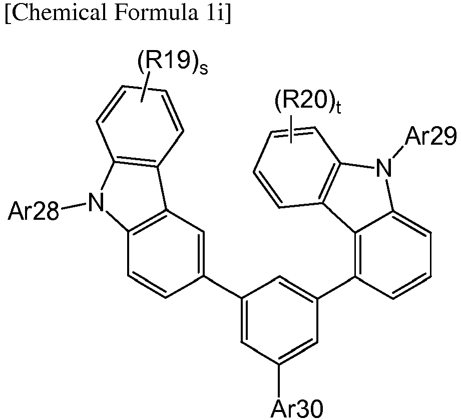 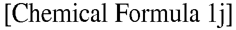 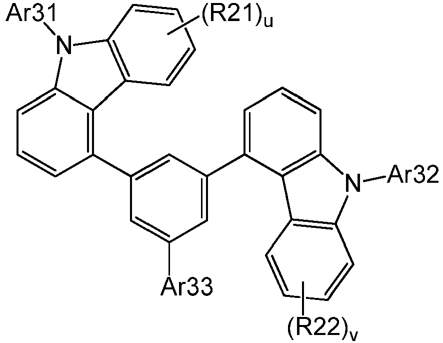 in Chemical Formulae 1a to 1j,
the definitions of Ar4 to Ar32 are the same as those of Ar1 to Ar4 in Chemical Formula 1,
the definitions of R3 to R22 are the same as those of R1 and R2 in Chemical Formula 1, and
the definitions of c, d, e, f, g, h, I, j, k, 1, m, n, o, p, q, r, s, t, u, and v are the same as those of a and b in Chemical Formula 1.

3. The hetero-cyclic compound of claim 1, wherein R1 and R2 of Chemical Formula 1 are each independently hydrogen or deuterium.

4. The hetero-cyclic compound of claim 1, wherein Chemical Formula 1 is represented by any one of the following compounds:

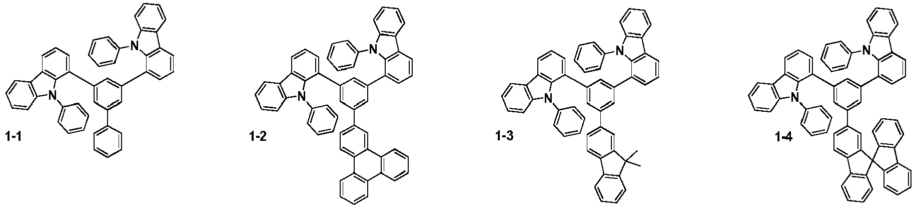

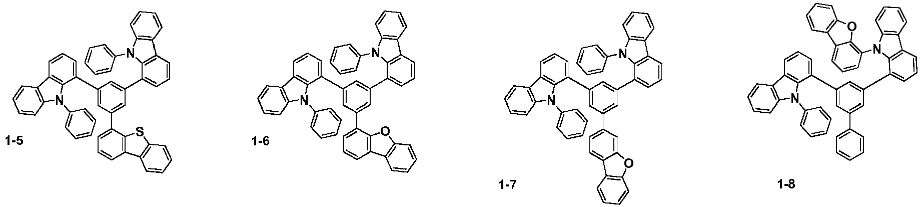

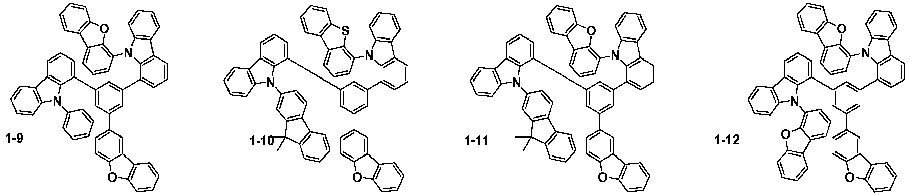

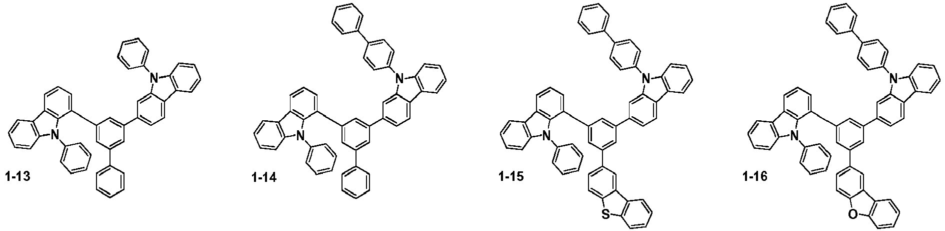

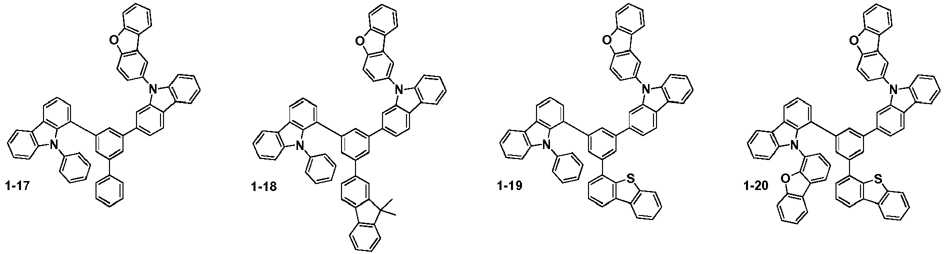

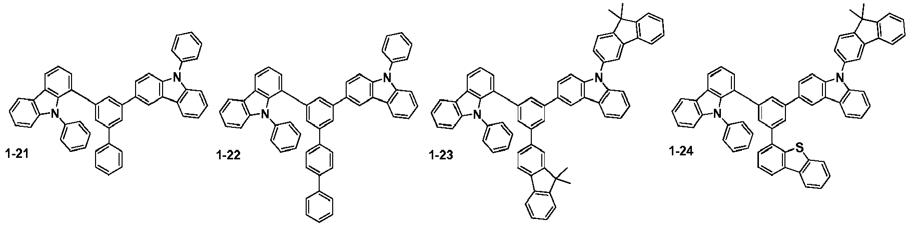

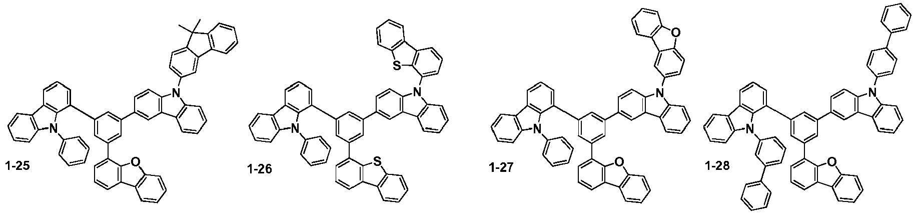

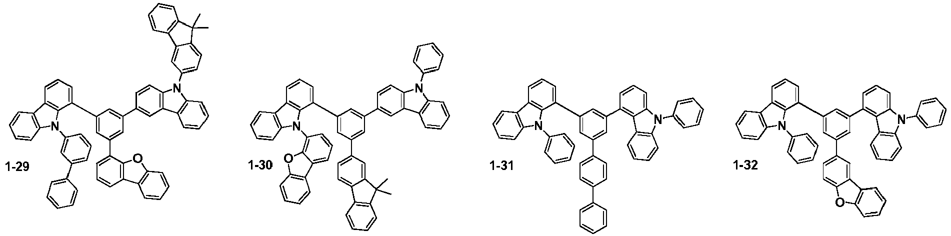

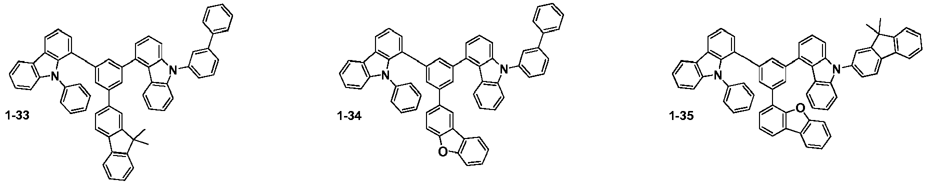

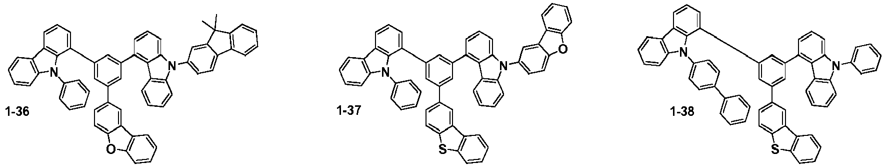

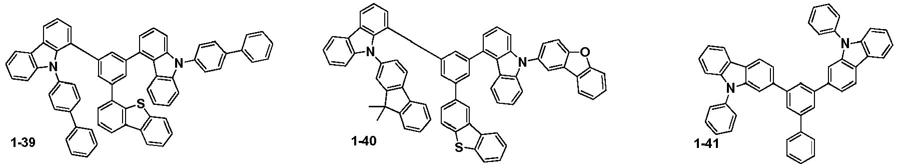

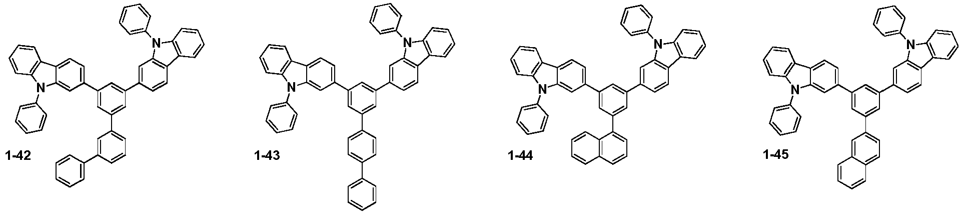

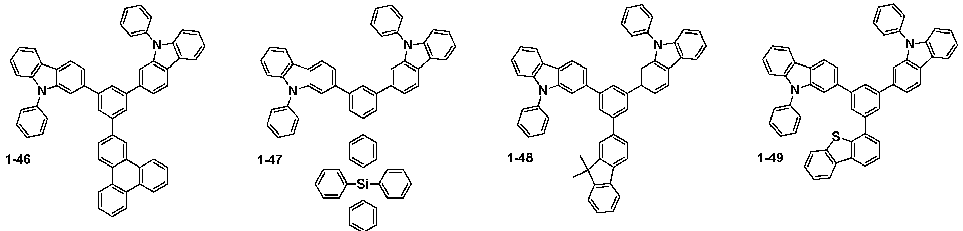

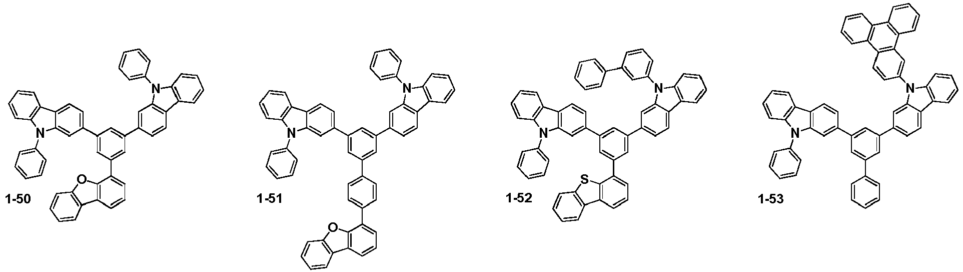

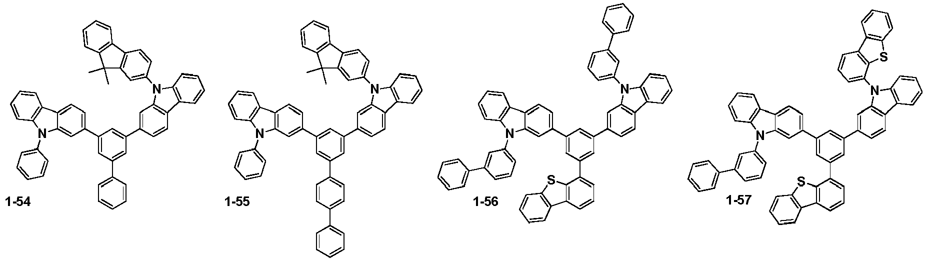

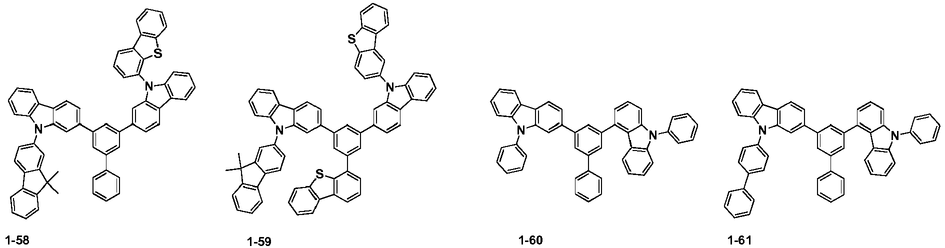

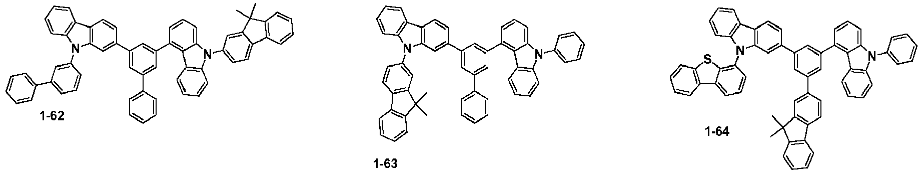

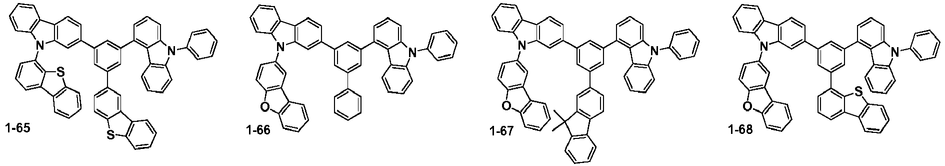

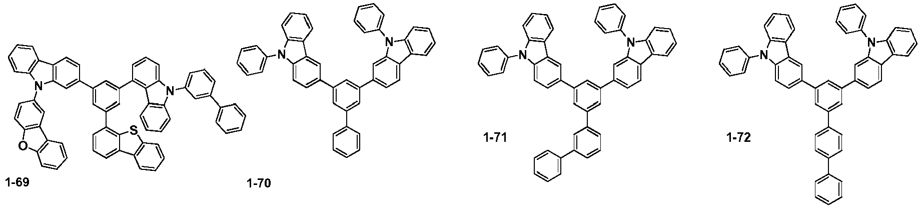

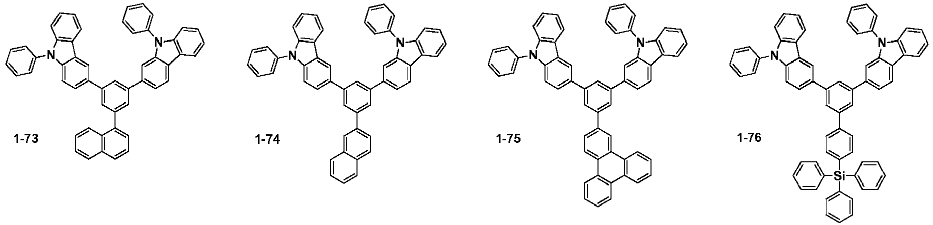

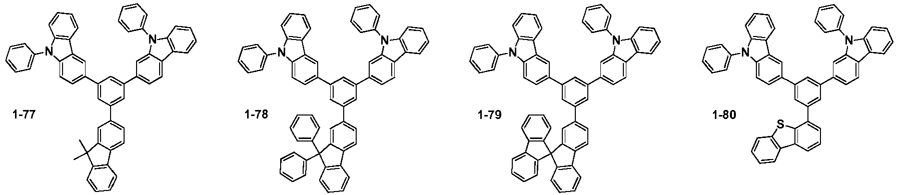

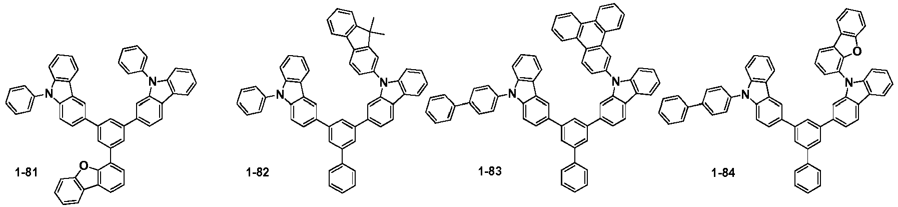

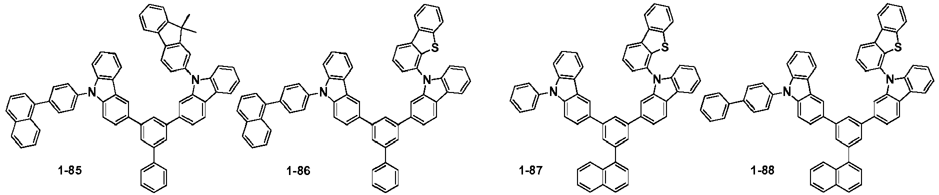

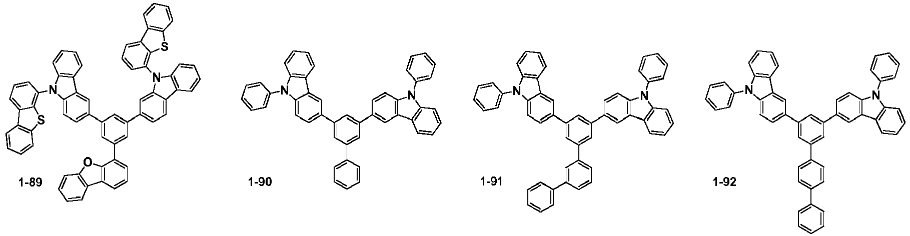

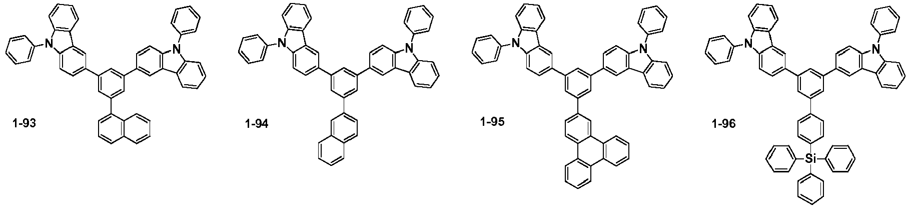

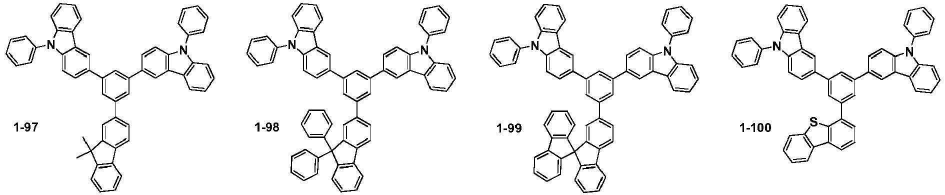

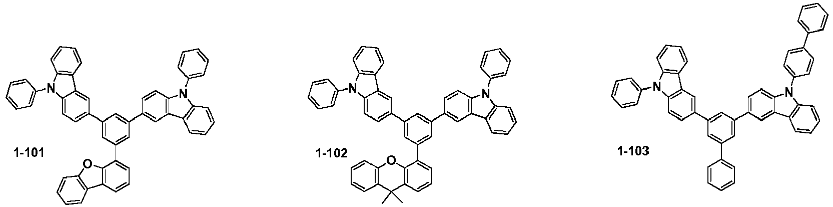

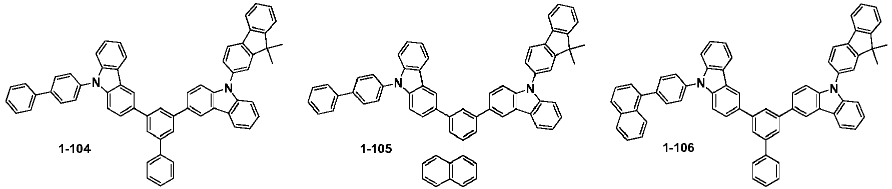

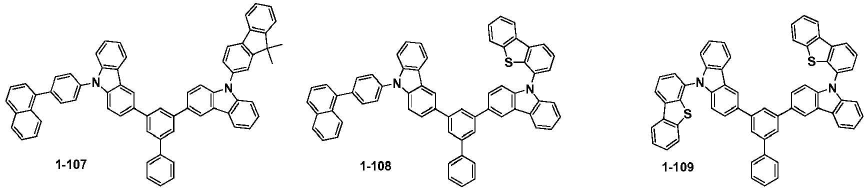

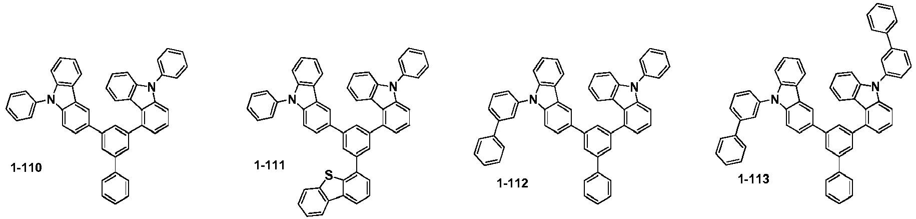

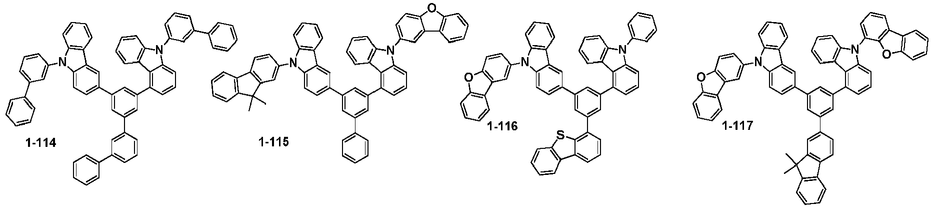

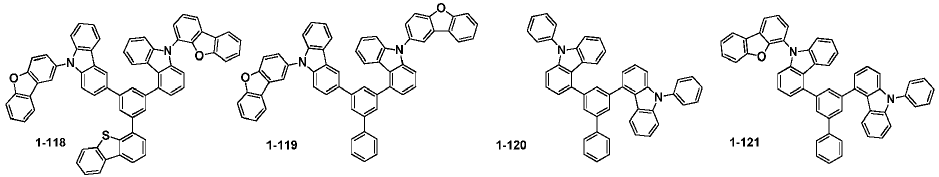

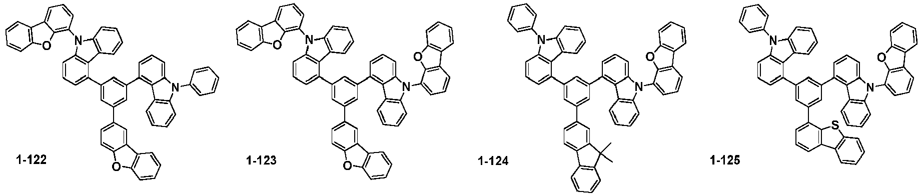

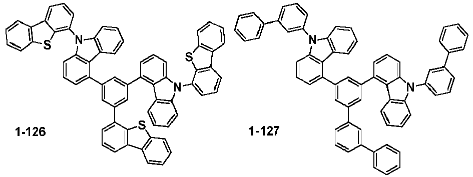

5. An organic light emitting device comprising:
a positive electrode;
a negative electrode; and
an organic material layer having one or more layers disposed between the positive electrode and the negative electrode,
wherein one or more layers of the organic material layer comprise the hetero-cyclic compound of any one of claims 1 to 4.

6. The organic light emitting device of claim 5, wherein the organic material layer comprises at least one layer of a hole blocking layer, an electron injection layer, and an electron transporting layer, and at least one layer of the hole blocking layer, the electron injection layer, and the electron transporting layer comprises the hetero-cyclic compound.

7. The organic light emitting device of claim 5, wherein the organic material layer comprises a light emitting layer, and the light emitting layer comprises the hetero-cyclic compound.

8. The organic light emitting device of claim 5, wherein the organic material layer comprises one or more layers of a hole injection layer, a hole transporting layer, and a layer which injects and transports holes simultaneously, and one layer of the layers comprises the hetero-cyclic compound.

9. The organic light emitting device of claim 5, wherein the organic material layer comprising the hetero-cyclic compound additionally comprises a compound represented by the following Chemical Formula 2 or 3: 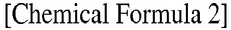 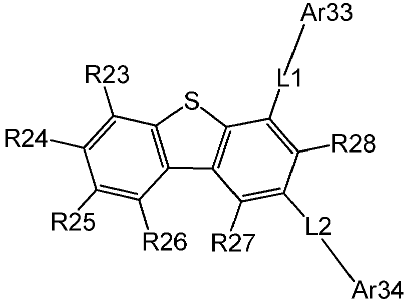 in Chemical Formula 4,
L1 and L2 are the same as or different from each other, and are each independently a direct bond or a substituted or unsubstituted C₆ to C₆₀ arylene group,
Ar33 is a substituted or unsubstituted C₂ to C₆₀ heteroaryl group comprising at least one N,
Ar34 is represented by the following Chemical Formula 4 or 5, 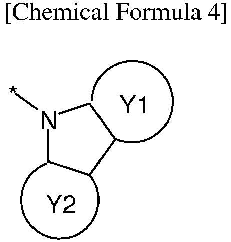 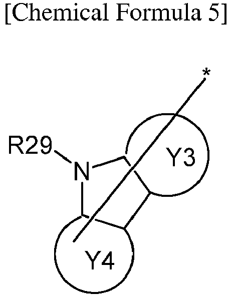 Y1 to Y4 are the same as or different from each other, and are each independently a substituted or unsubstituted C₆ to C₆₀ aromatic hydrocarbon ring; or a substituted or unsubstituted C₂ to C₆₀ aromatic hetero ring,
R23 to R29 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and 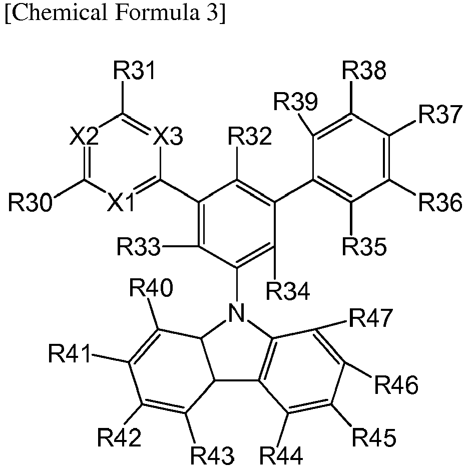 in Chemical Formula 3,
at least one of X1 to X3 is N, and the others are each independently N or CR48,
R31, R32, and R48 are the same as or different from each other, and are each independently selected from the group consisting of hydrogen; deuterium; a halogen group;-CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R";-P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring,
R32 to R34 and R40 to R43 are the same as or different from each other, and are each independently selected from the group consisting of hydrogen; deuterium; a halogen group;-CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R";-P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group,
R44 to R47 are the same as or different from each other, and are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted hydrocarbon ring or hetero ring,
at least one of R35 to R39 is -CN, and the others are each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group which is unsubstituted or substituted with a C₁ to C₂₀ alkyl group, a substituted or unsubstituted C₆ to C₆₀ aryl group, or a C₂ to C₆₀ heteroaryl group, or two or more adjacent groups are bonded to each other to form a substituted or unsubstituted aliphatic or aromatic hydrocarbon ring, and
R, R', and R" are the same as or different from each other, and are each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group.

10. The organic light emitting device of claim 9, wherein the compound represented by Chemical Formula 2 is represented by any one of the following compounds:

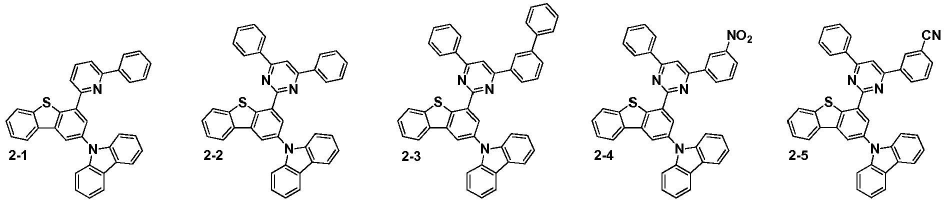

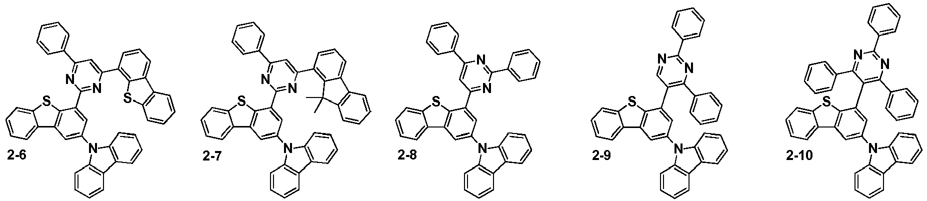

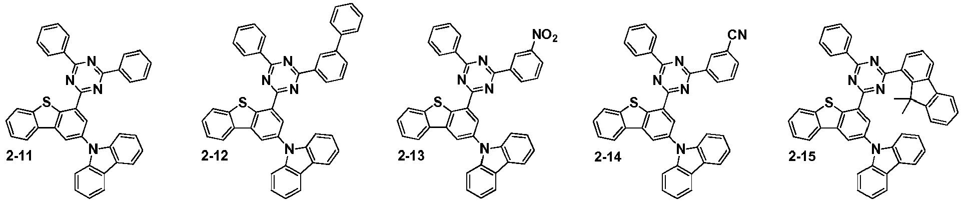

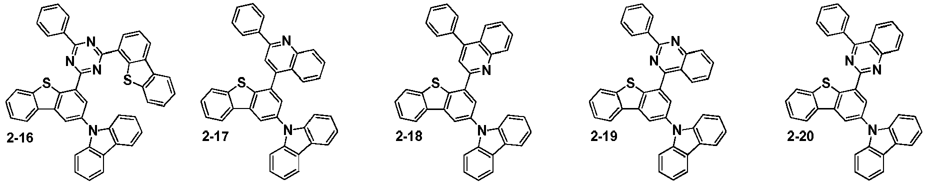

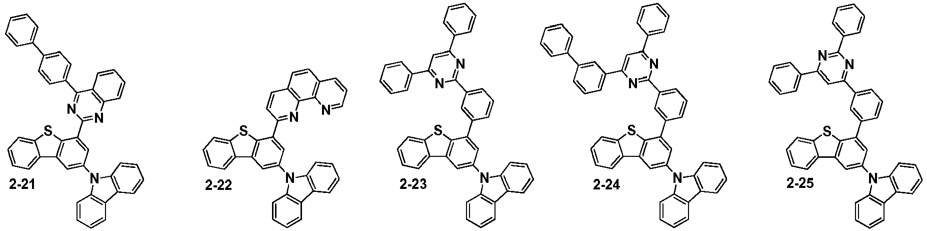

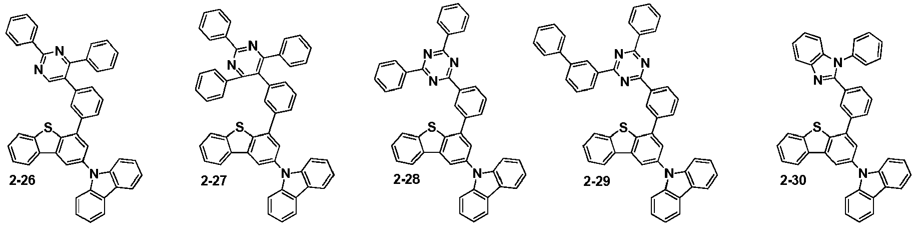

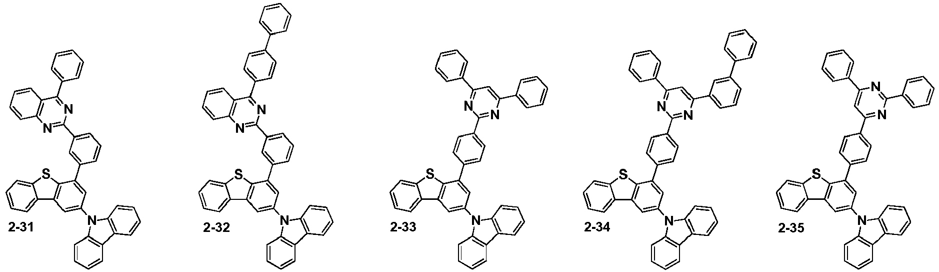

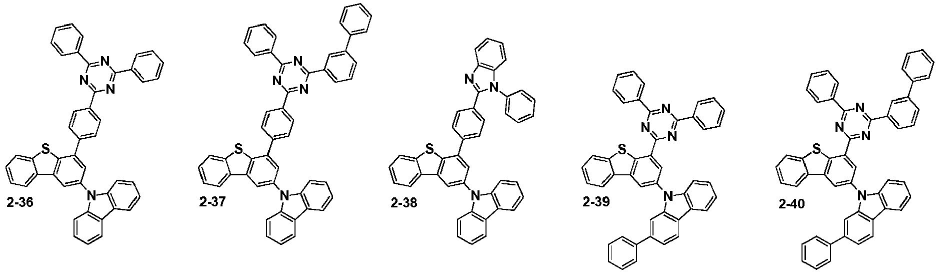

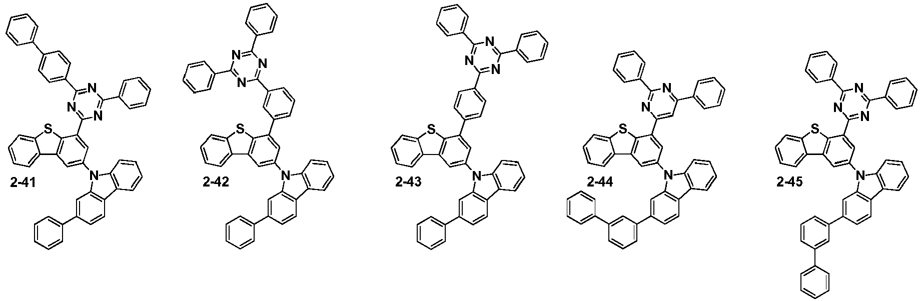

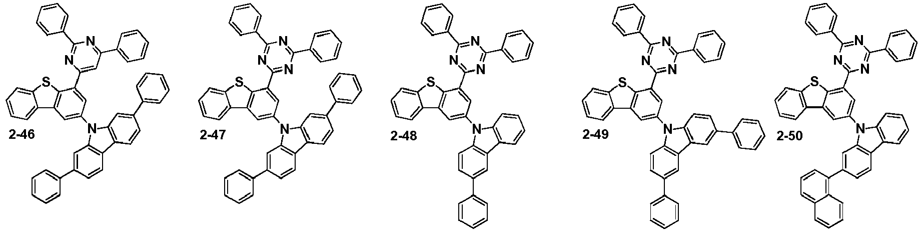

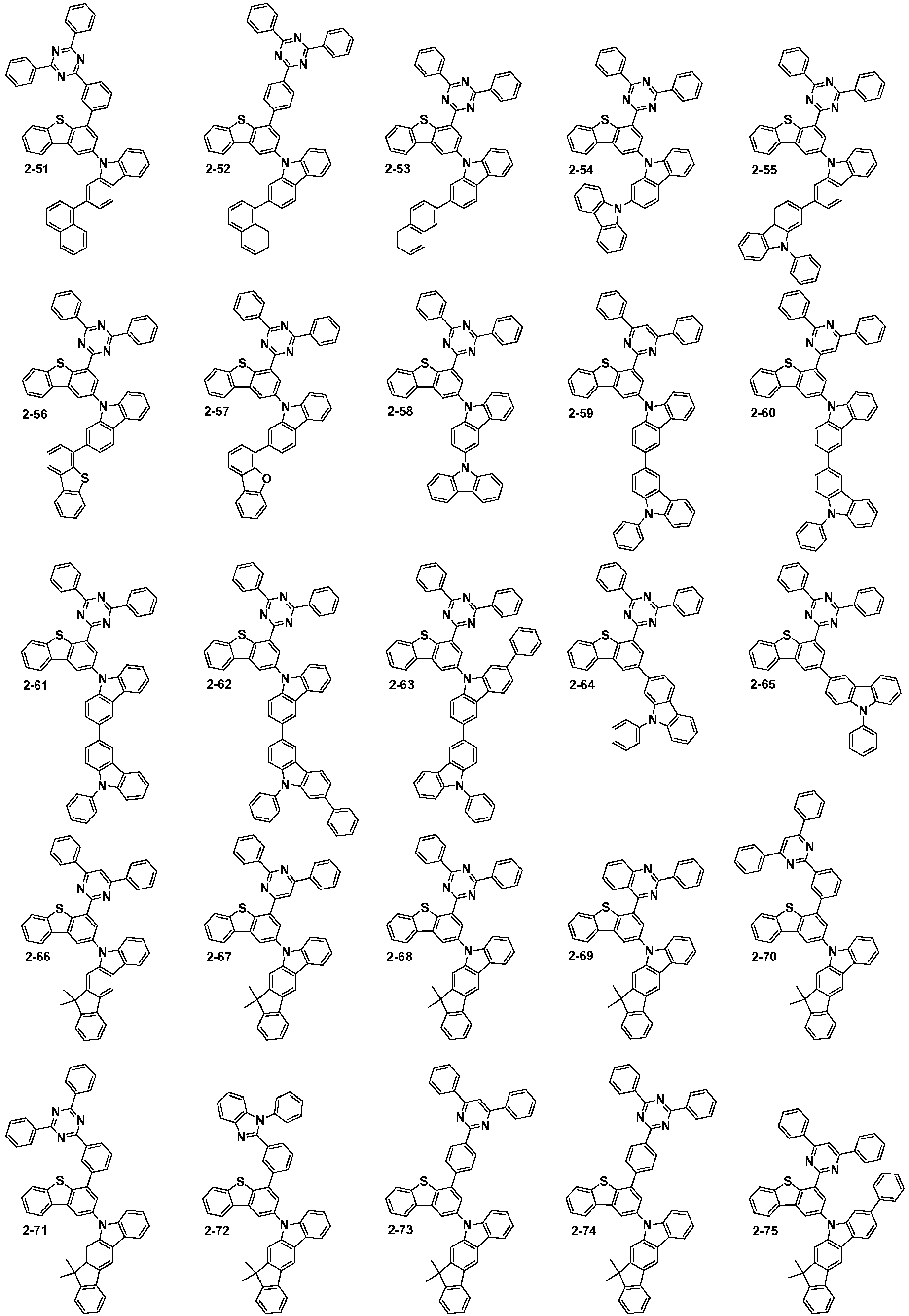

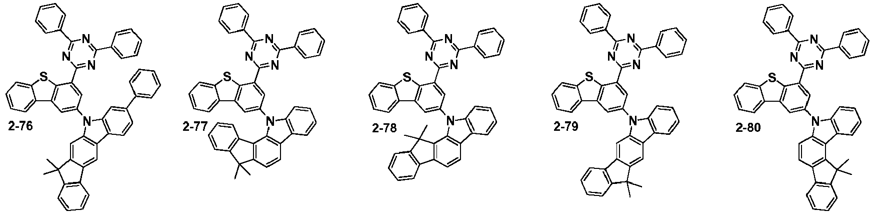

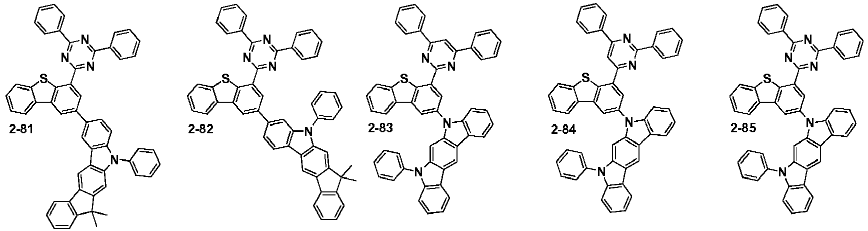

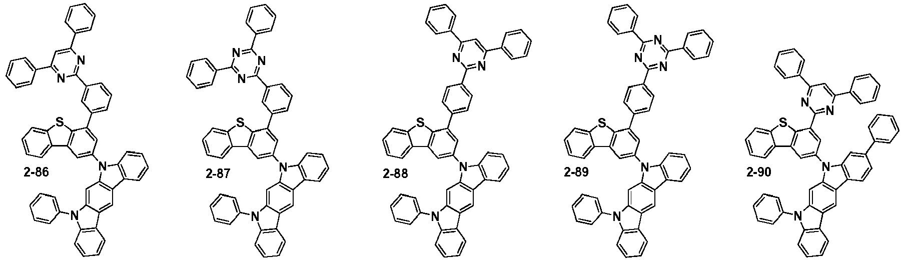

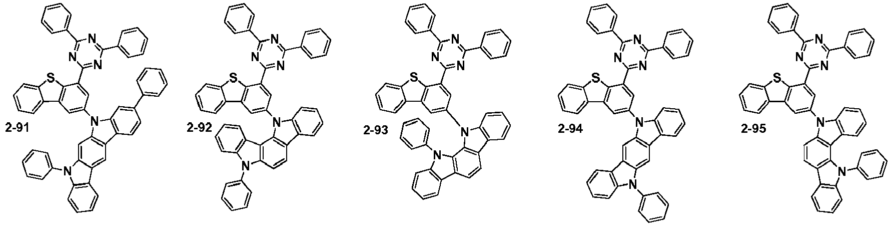

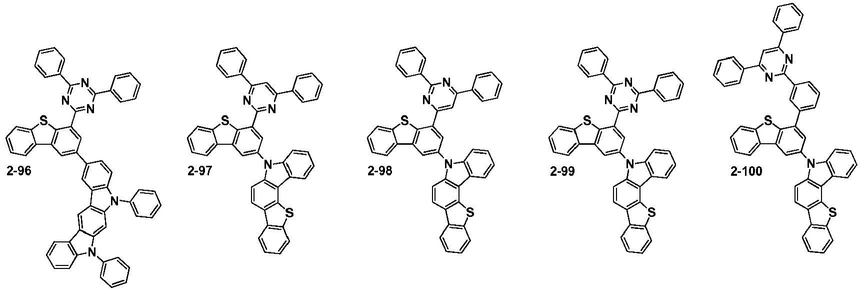

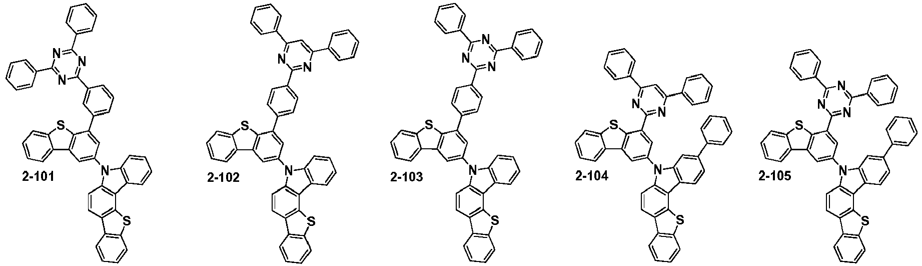

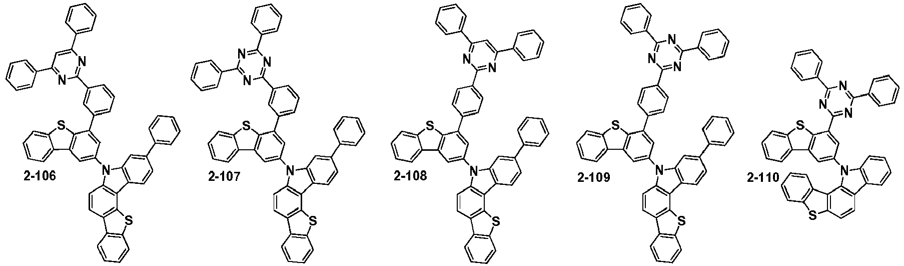

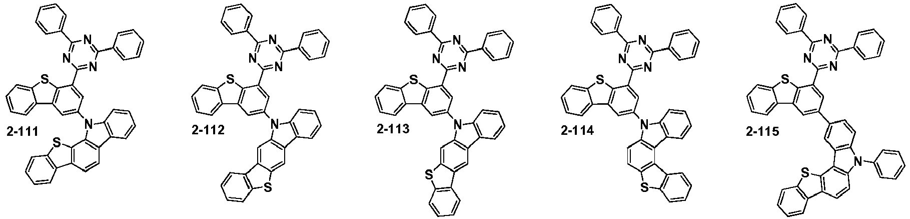

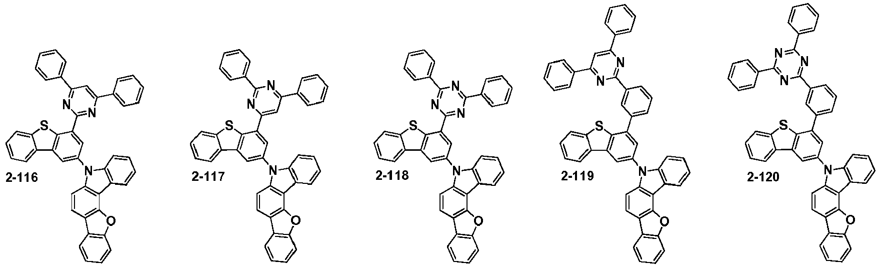

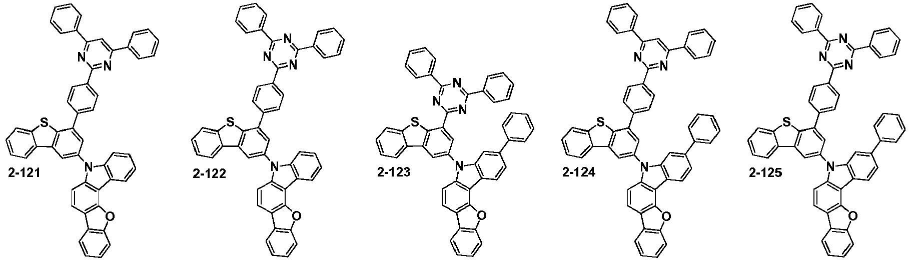

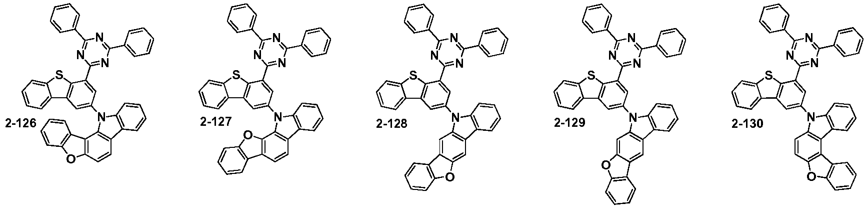

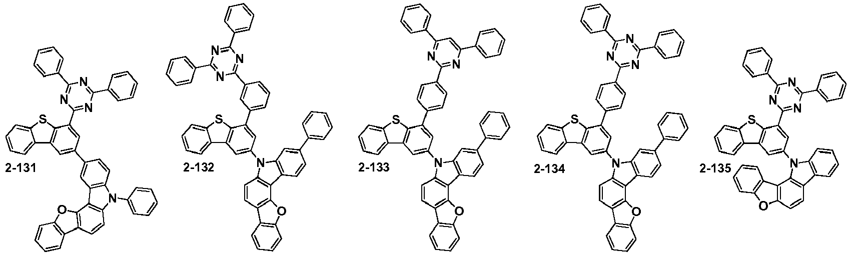

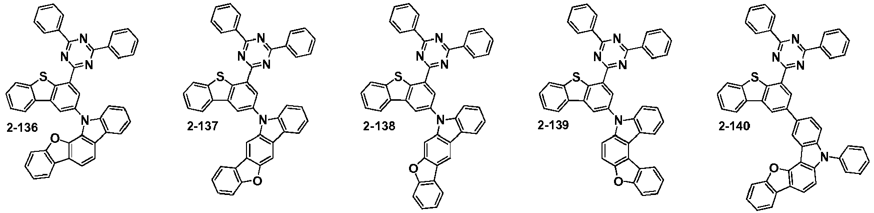

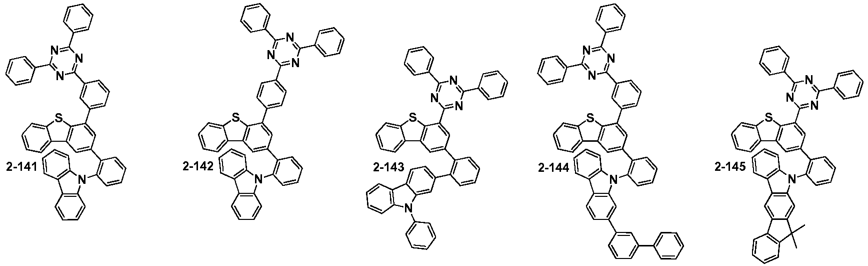

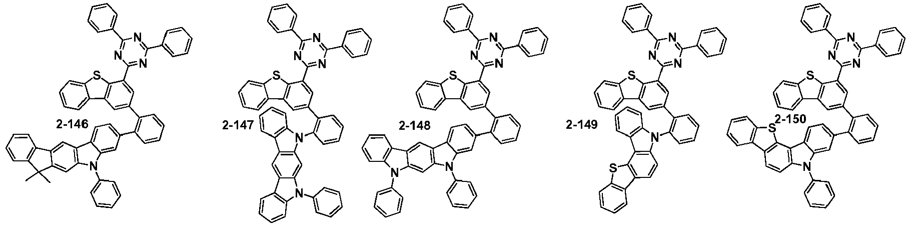

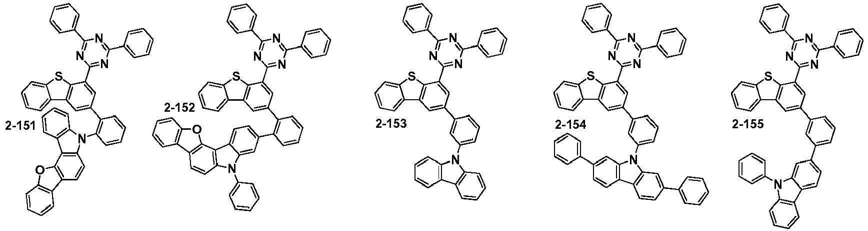

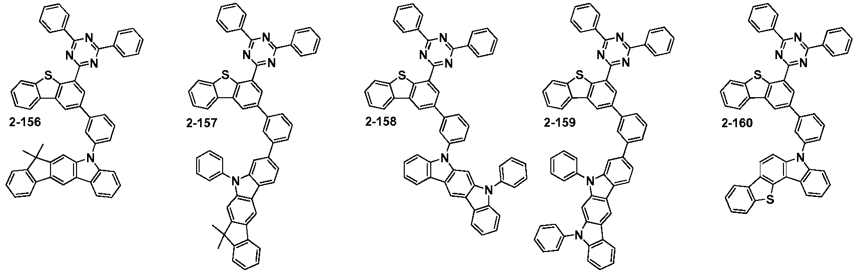

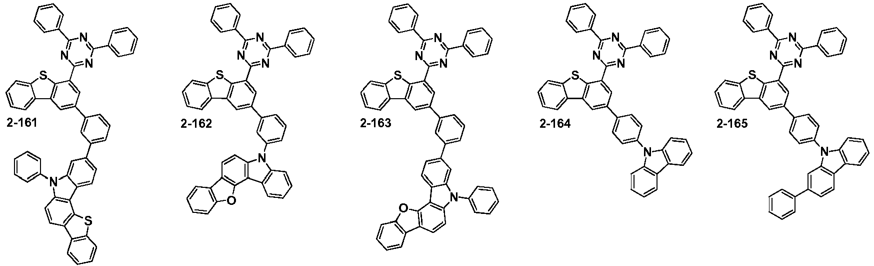

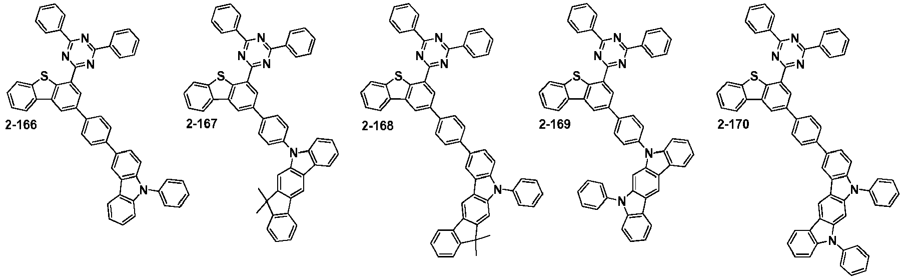

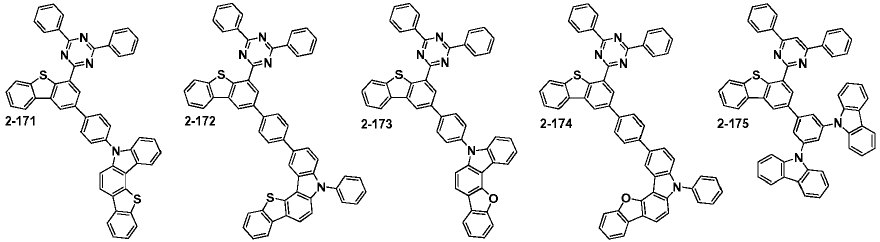

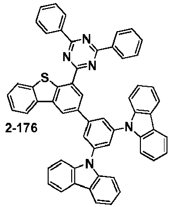

11. The organic light emitting device of claim 9, wherein the compound represented by Chemical Formula 3 is represented by any one of the following compounds:

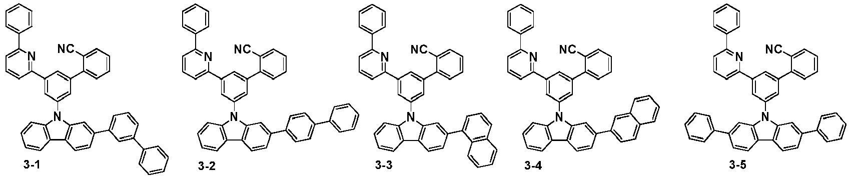

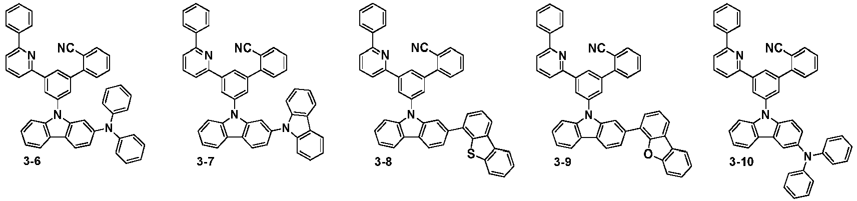

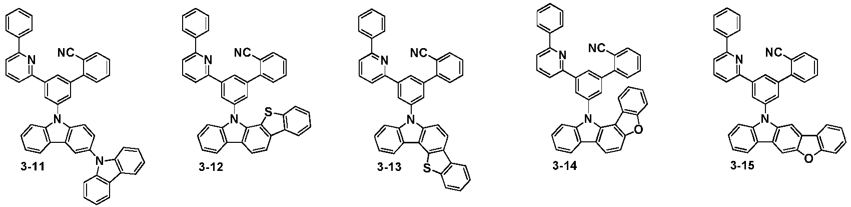

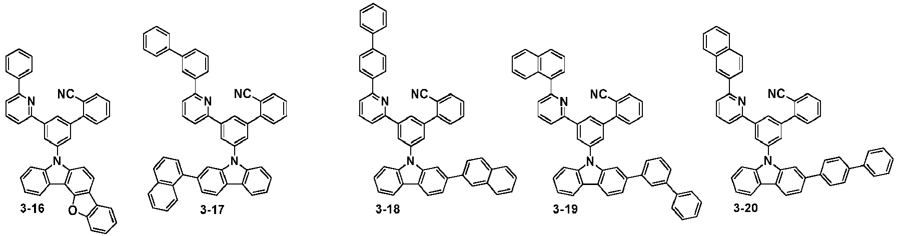

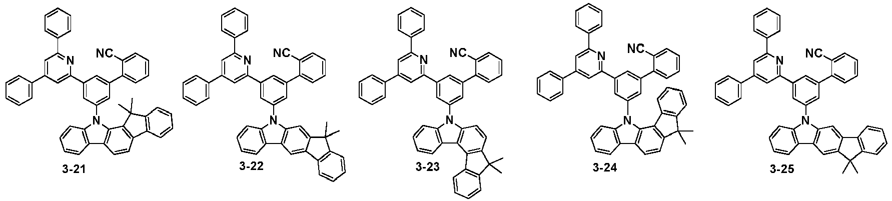

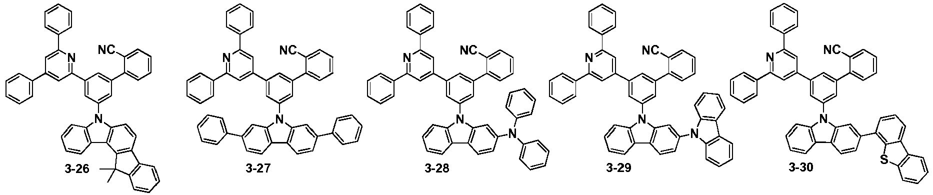

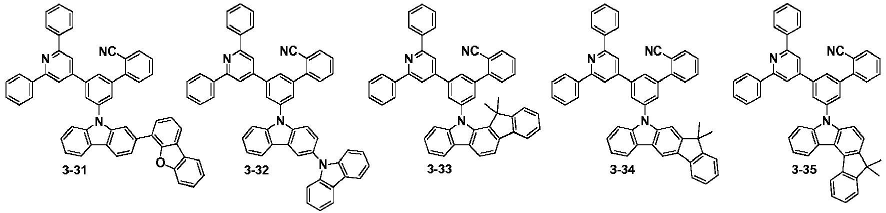

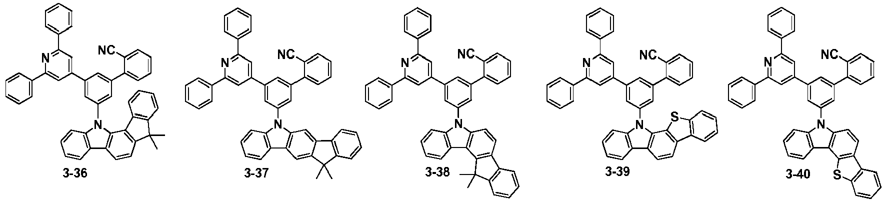

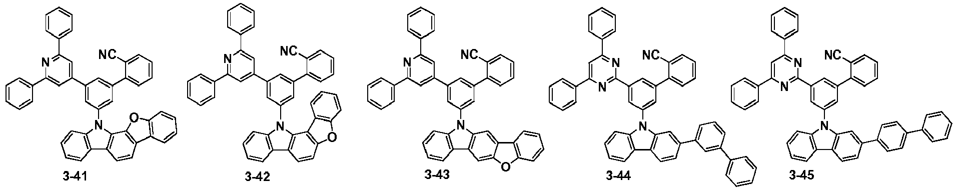

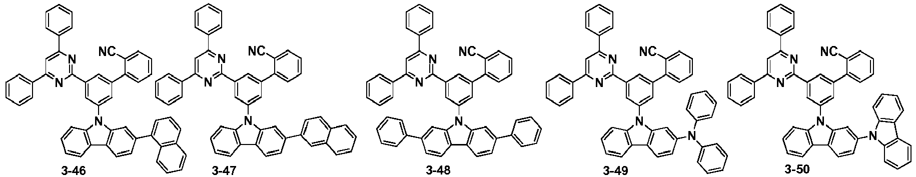

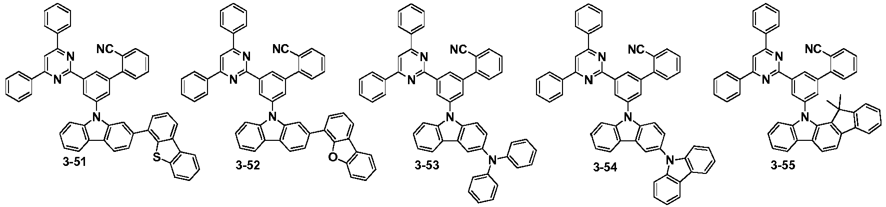

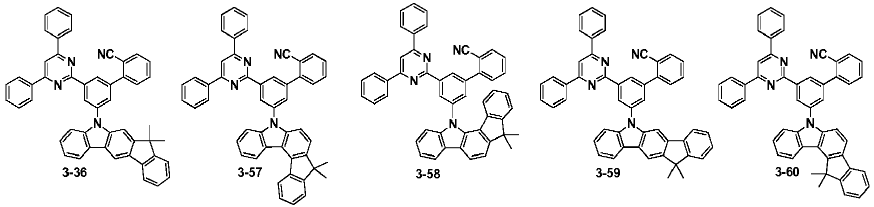

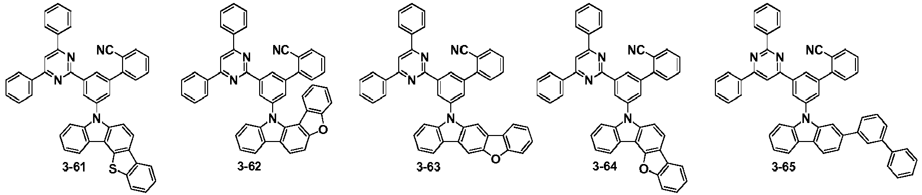

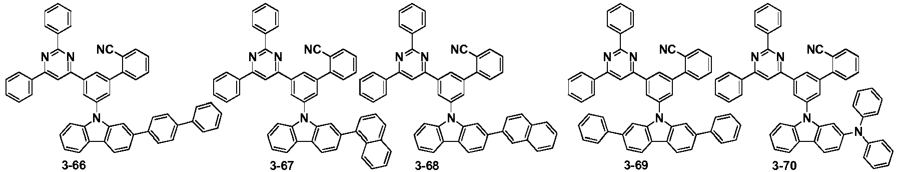

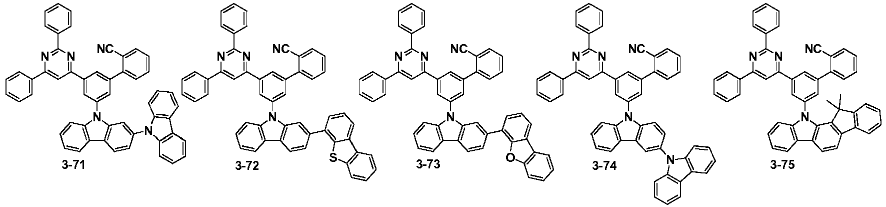

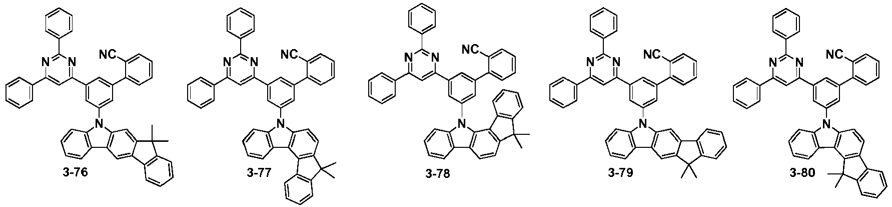

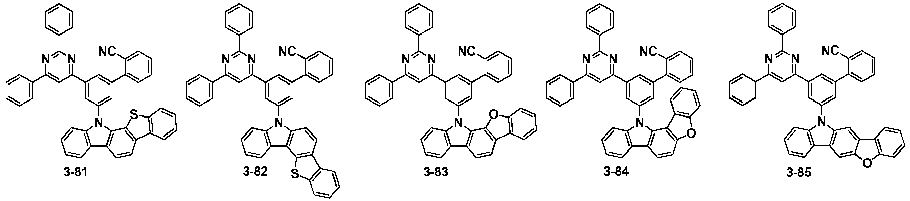

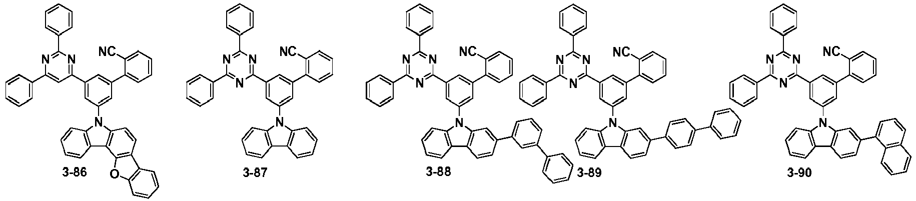

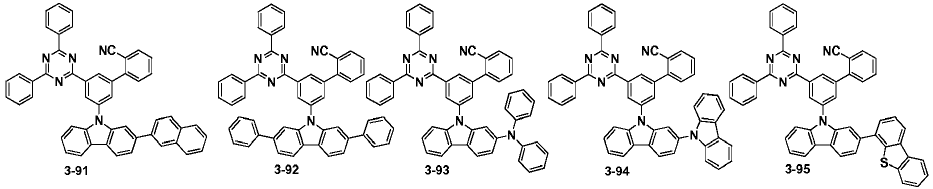

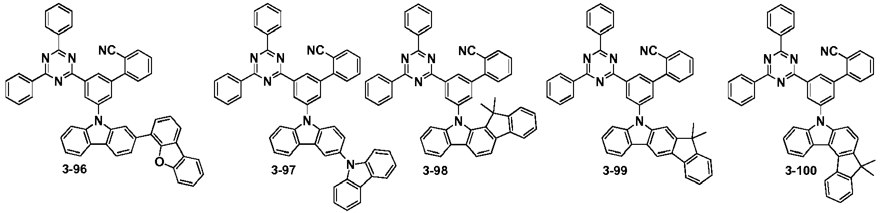

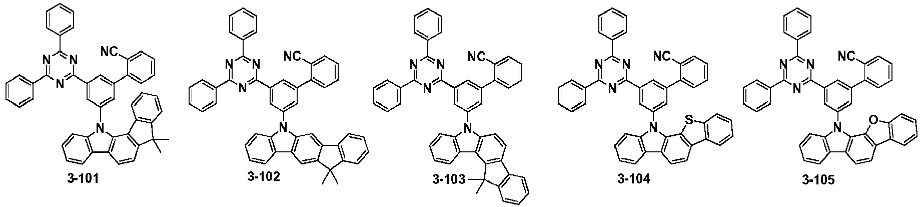

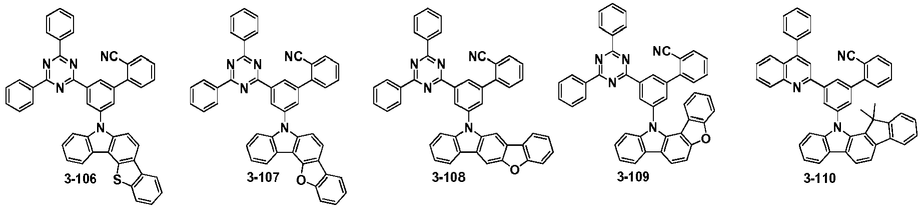

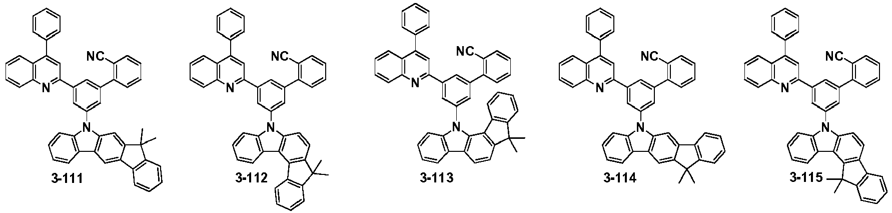

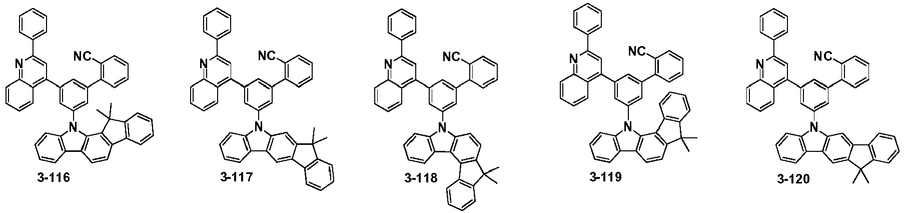

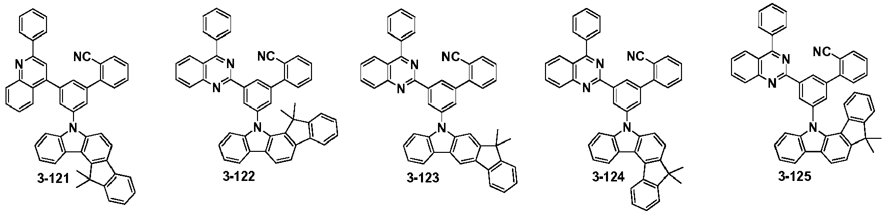

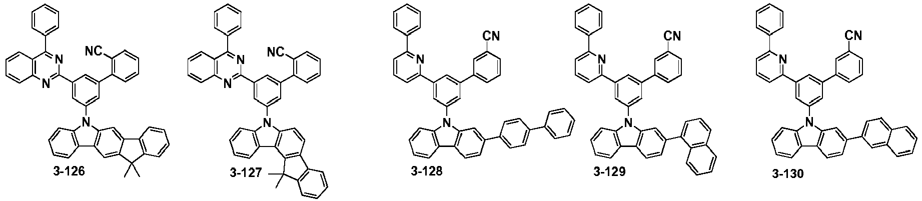

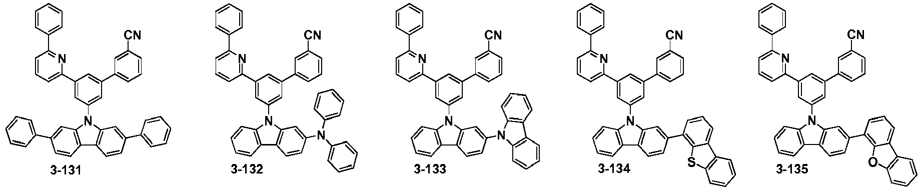

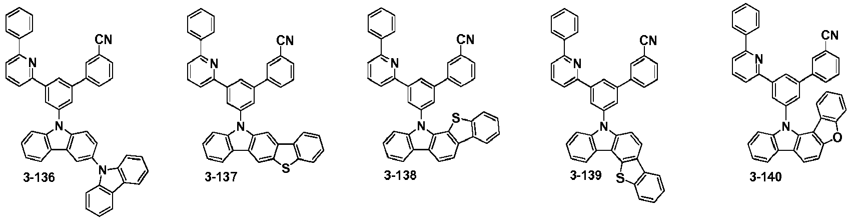

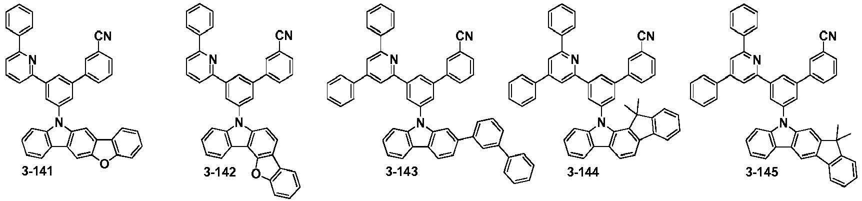

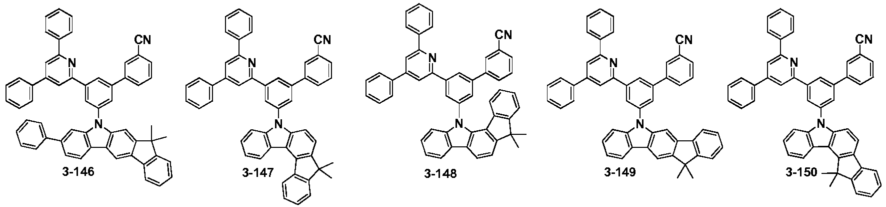

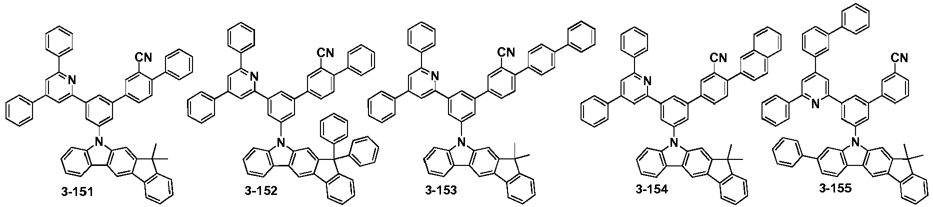

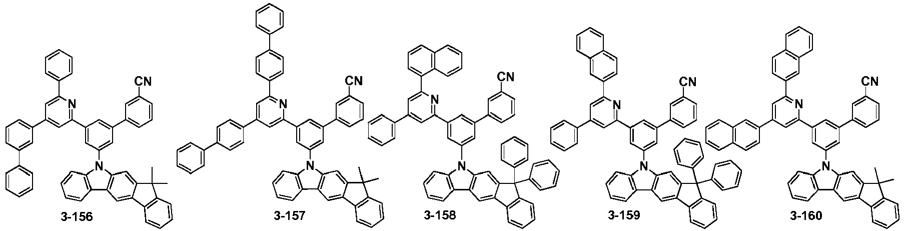

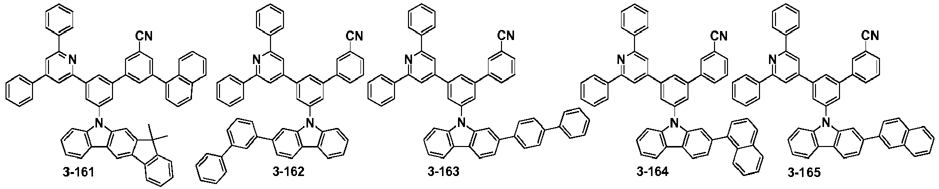

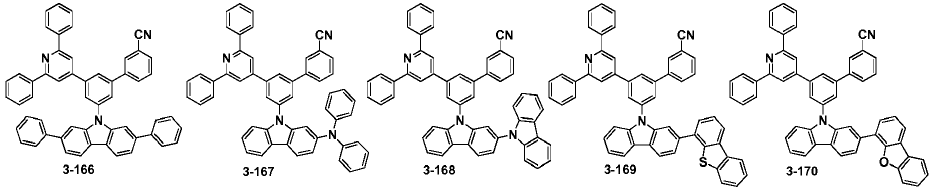

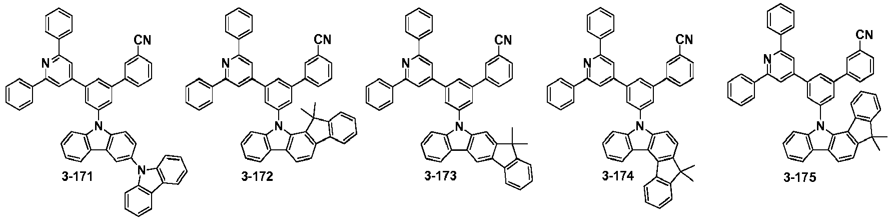

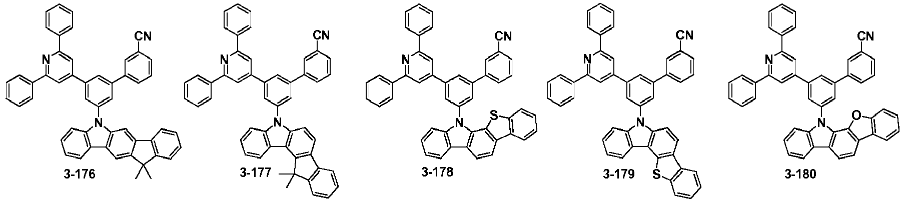

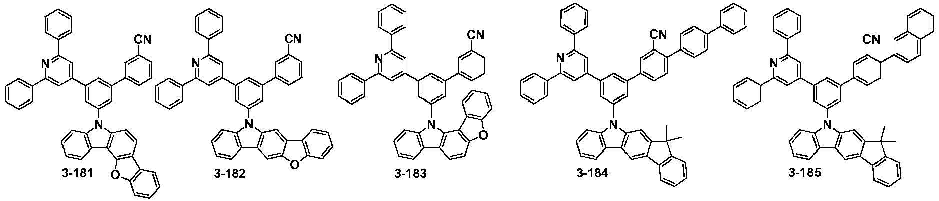

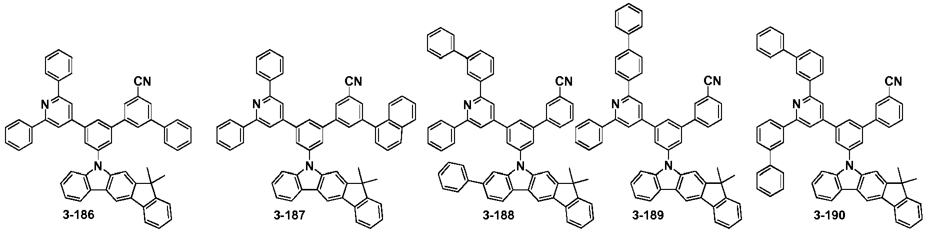

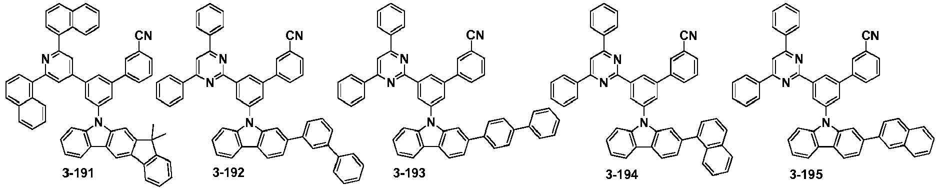

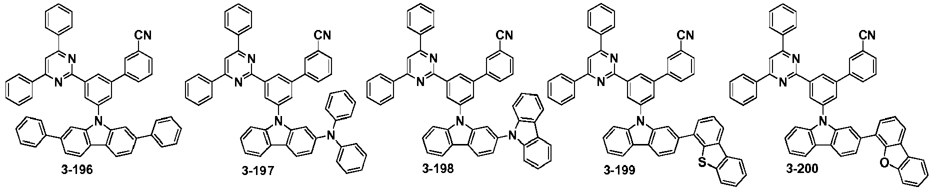

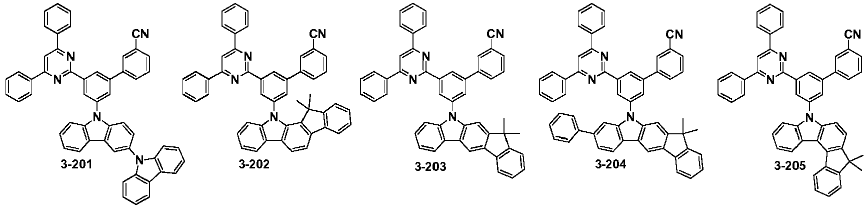

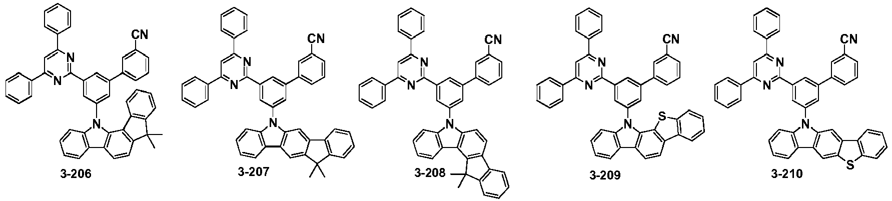

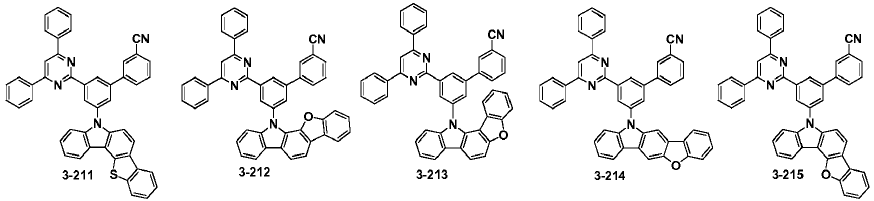

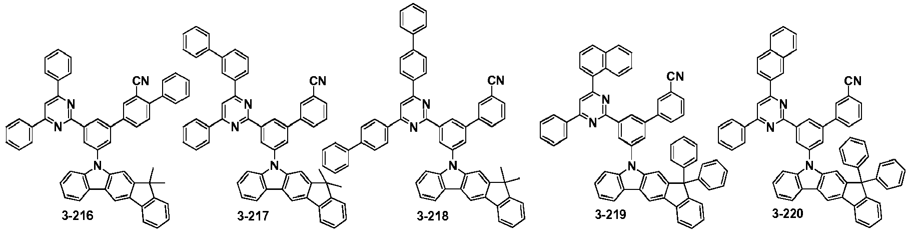

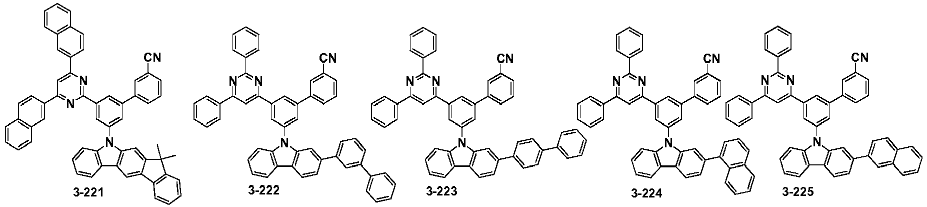

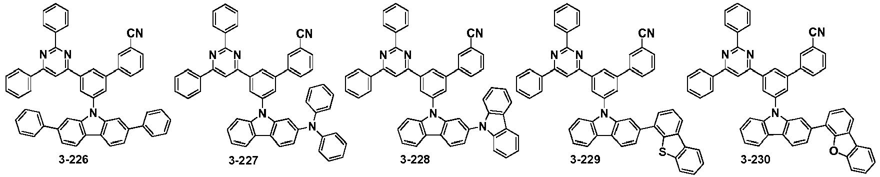

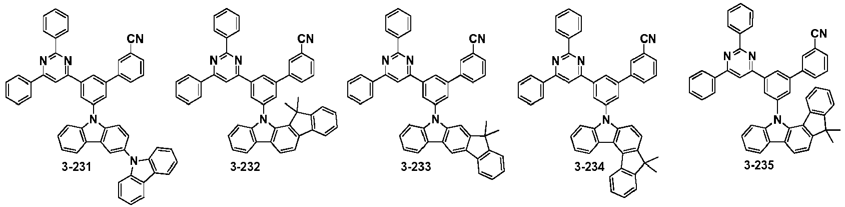

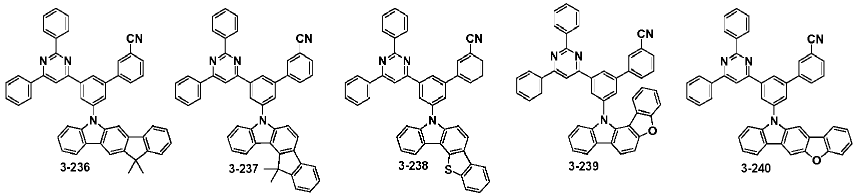

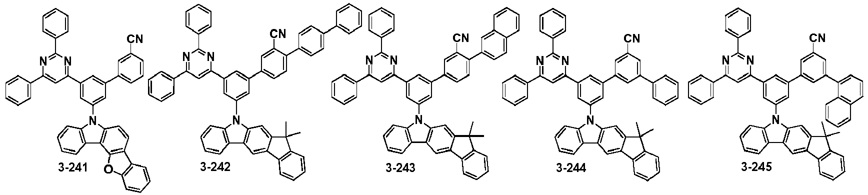

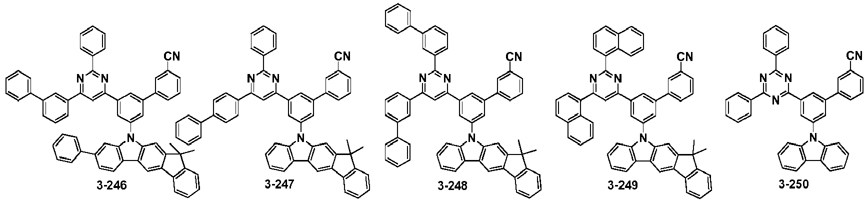

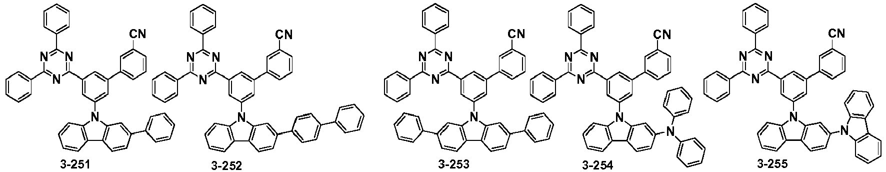

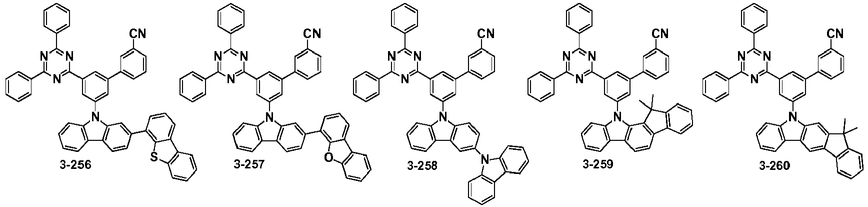

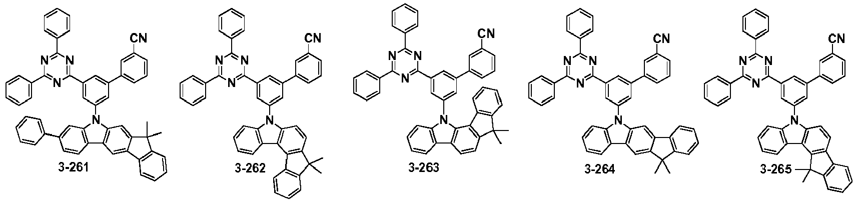

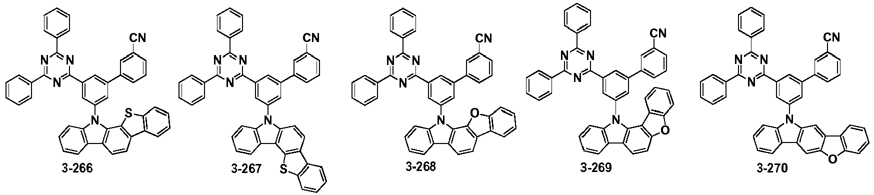

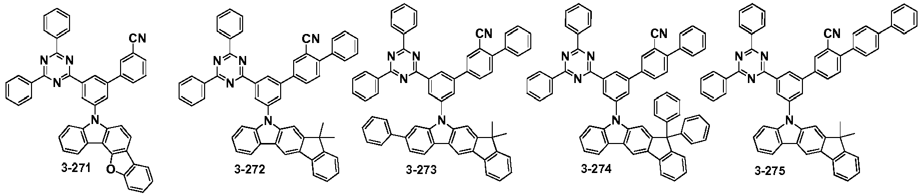

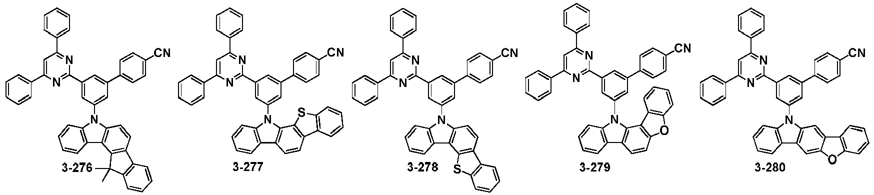

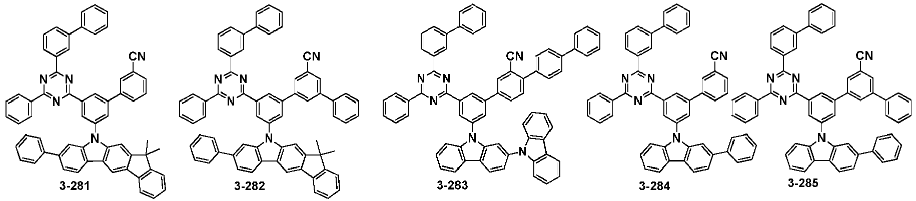

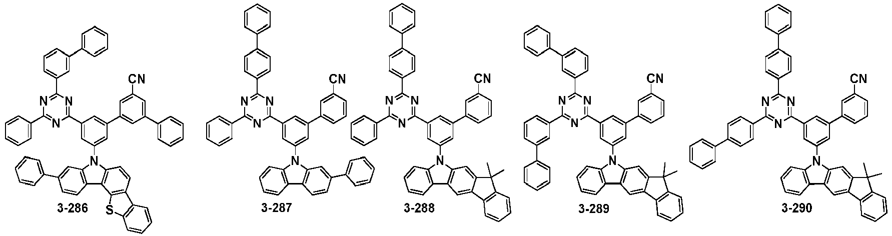

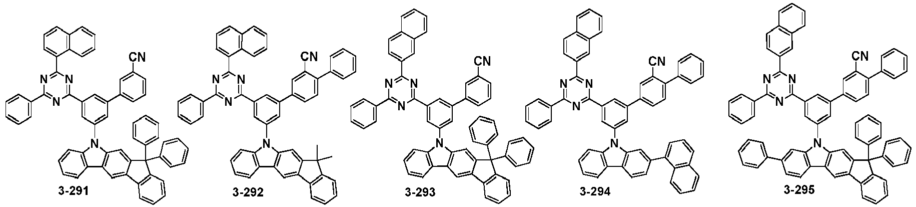

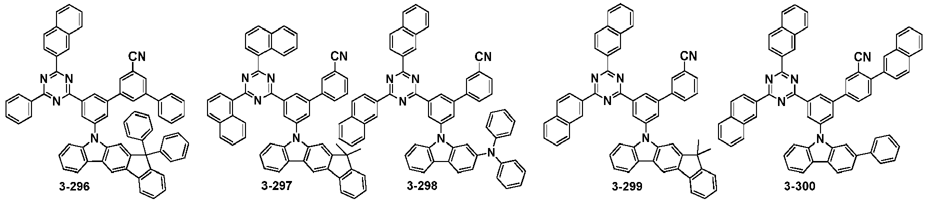

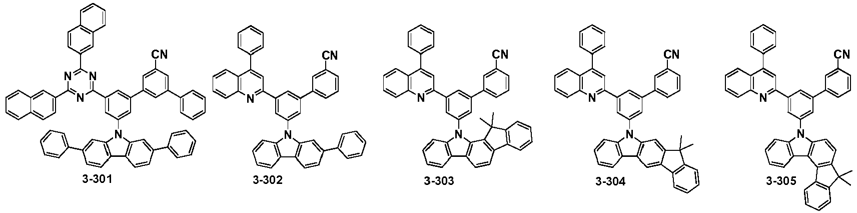

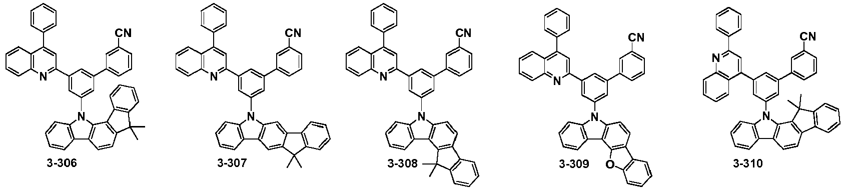

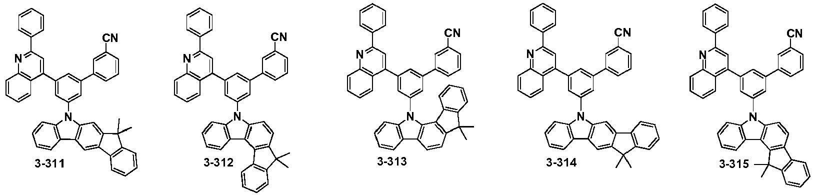

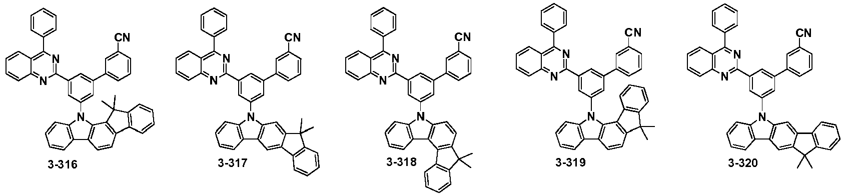

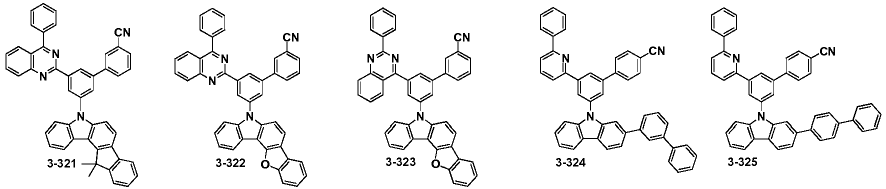

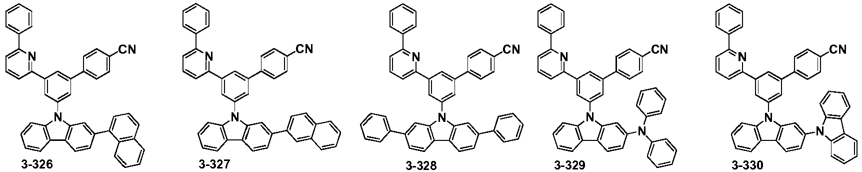

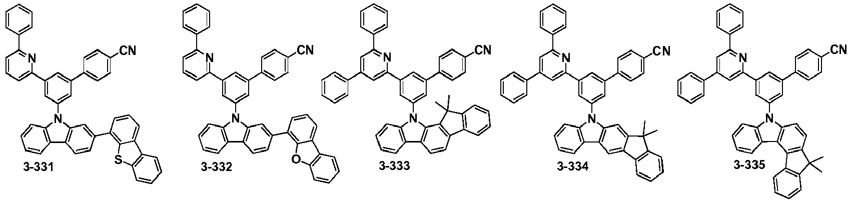

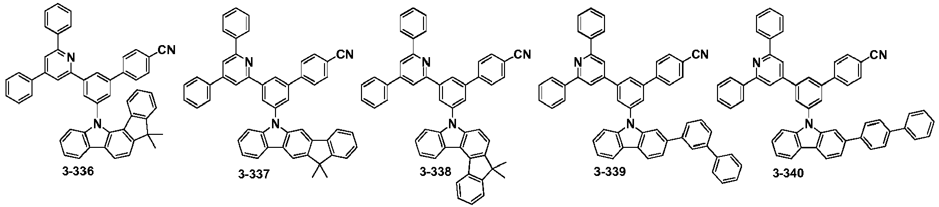

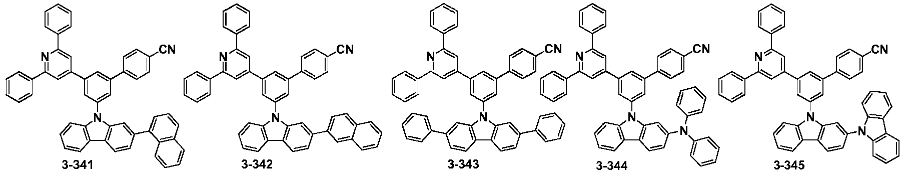

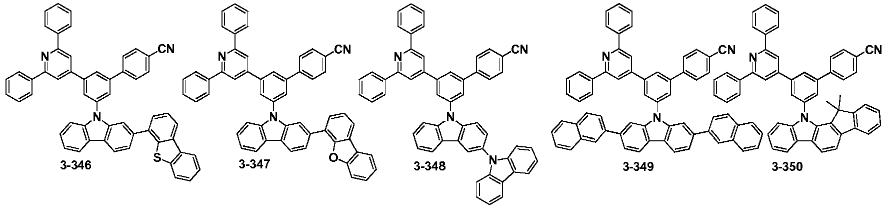

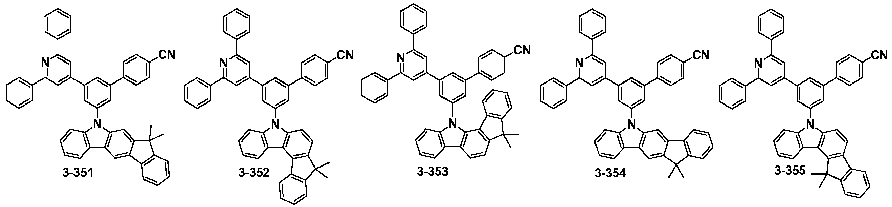

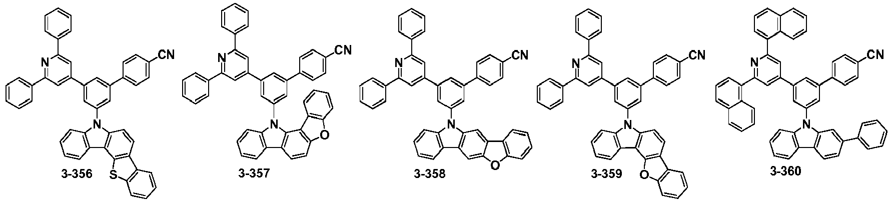

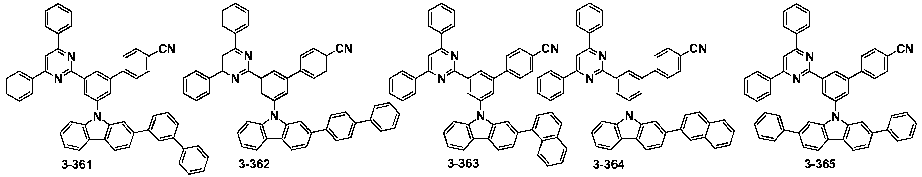

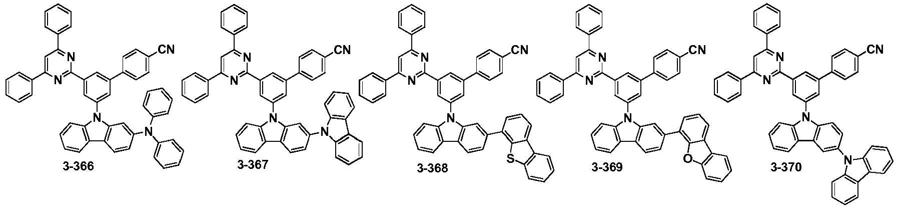

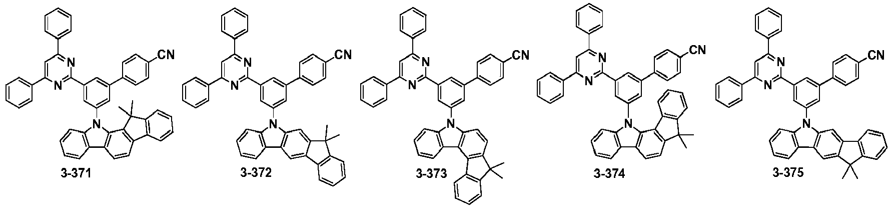

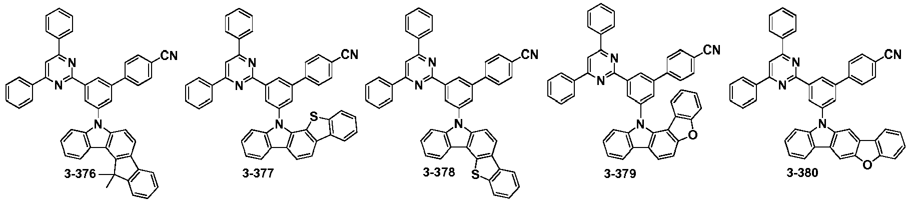

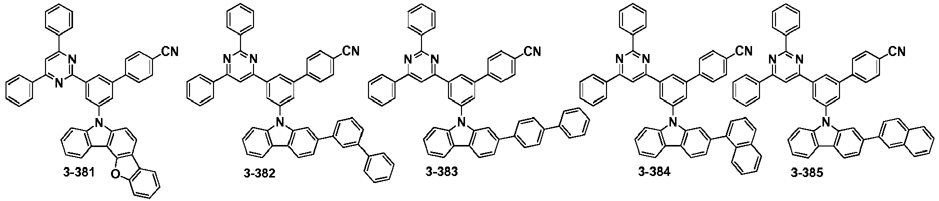

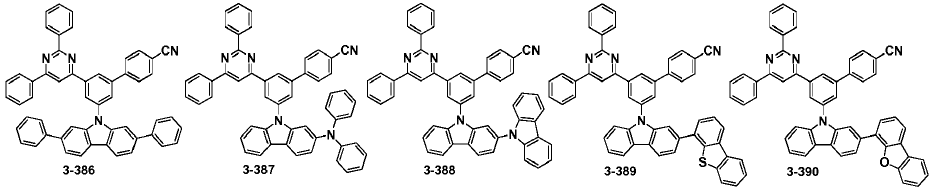

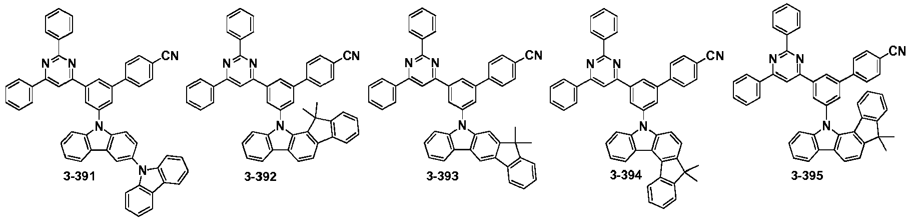

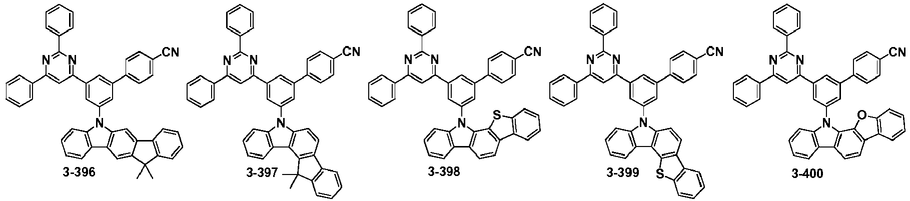

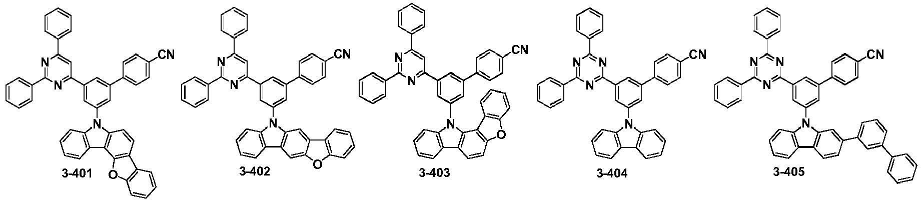

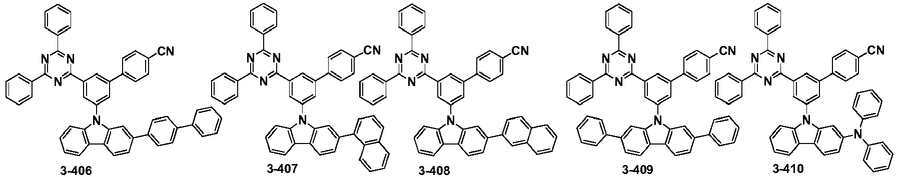

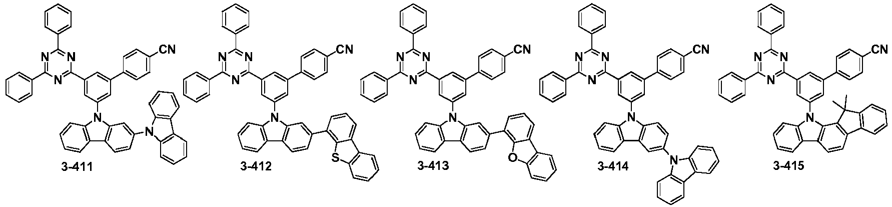

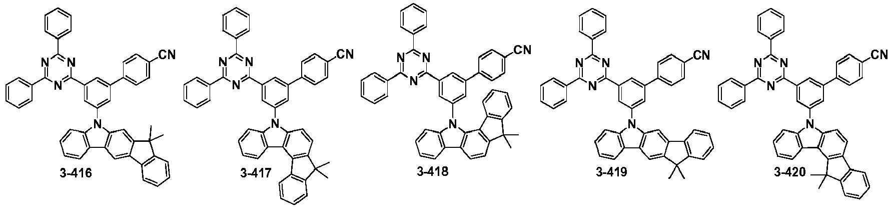

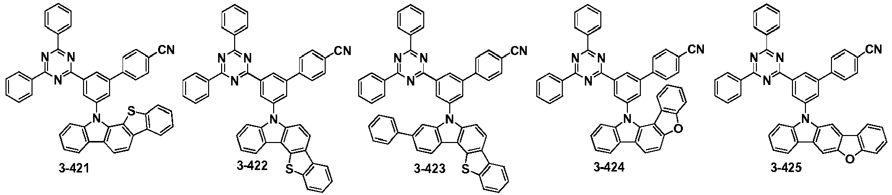

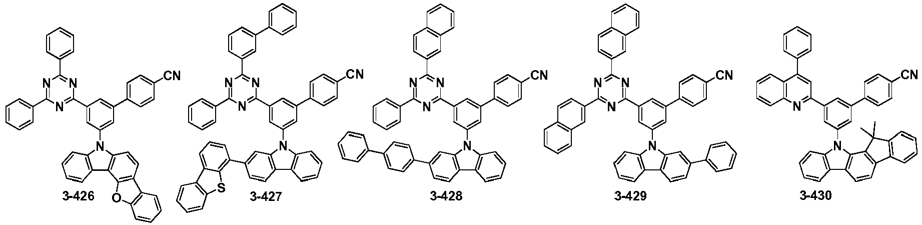

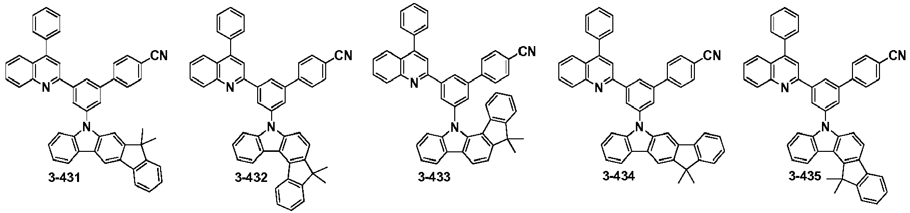

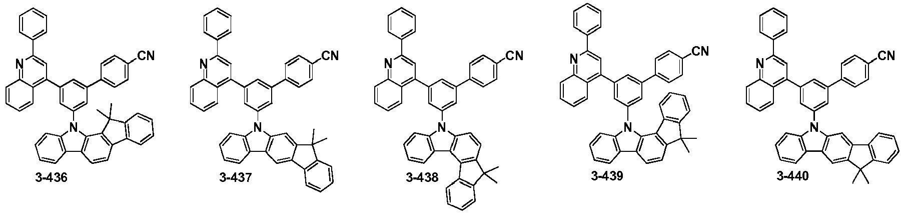

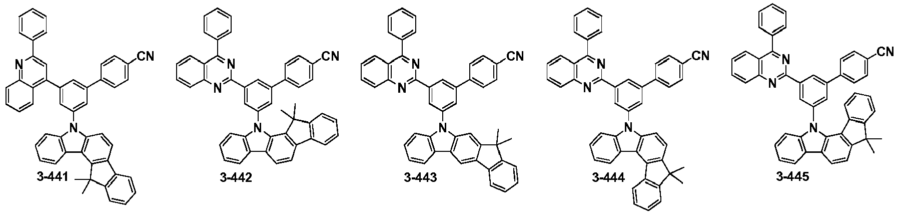

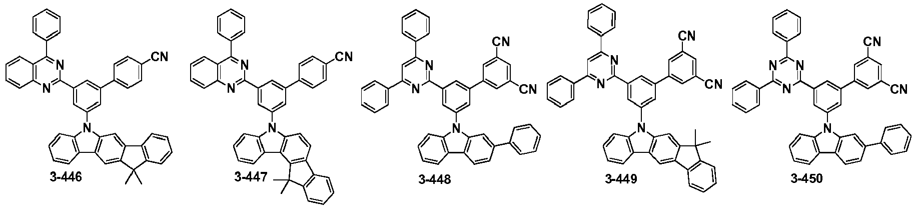

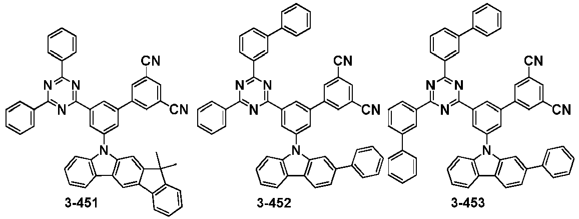

12. The organic light emitting device of claim 9, wherein the organic light emitting device comprises as a host material for a light emitting layer: the hetero-cyclic compound; and the compound represented by Chemical Formula 2 or 3.

13. The organic light emitting device of claim 9, wherein the organic light emitting device comprises as a host material for a light emitting layer: the hetero-cyclic compound; and the compound represented by Chemical Formula 2 or 3 at a weight ratio of 1:10 to 10:1.
